# EUROPEAN PATENT SPECIFICATION

(11) **EP 4 291 164 B1**
(45) Date of publication and mention of the grant of the patent: **08.07.2026**
(21) Application number: 22753385.8
(22) Date of filing: 11.02.2022
(51) Int. Cl.: A61K 9/107, A61K 38/46, C12N 5/10, C12N 9/22, C12N 15/09, C12N 15/11, C12Q 1/6806

(54) **ENGINEERED EXTRACELLULAR VESICLES AND THEIR USES**
MANIPULIERTE EXTRAZELLULÄRE VESIKEL UND DEREN VERWENDUNGEN
VÉSICULES EXTRACELLULAIRES MODIFIÉES ET LEURS UTILISATIONS

(30) Priority: 12.02.2021 US 202163148872 P
(43) Date of publication of application: 20.12.2023
(73) Proprietor: Wake Forest University Health Sciences, Winston-Salem, NC 27157 (US)
(72) Inventor: LU, Baisong, Winston-Salem, North Carolina 27157 (US); ATALA, Anthony, Winston-Salem, North Carolina 27157 (US)
(74) Representative: Forresters IP LLP
(86) International application number: PCT/US2022/016053
(87) International publication number: WO 2022/174004

(56) References cited:
- EP-A1- 4 059 948
- WO-A1-2015/002956
- WO-A1-2019/213257
- WO-A1-2019/213257
- WO-A1-2020/099682
- US-A1- 2020 308 571
- US-A1- 2020 390 700
- YE YANGYANG ET AL: "An engineered exosome for delivering sgRNA:Cas9 ribonucleoprotein complex and genome editing in recipient cells", vol. 8, no. 10, 19 May 2020 (2020-05-19), GB, pages 2966 - 2976, XP093208126, ISSN: 2047-4830, Retrieved from the Internet <URL:https://pubs.rsc.org/en/content/articlepdf/2020/bm/d0bm00427h> DOI: 10.1039/D0BM00427H
- PIN LYU ET AL: "Delivering Cas9/sgRNA ribonucleoprotein (RNP) by lentiviral capsid-based bionanoparticles for efficient 'hit-and-run' genome editing", NUCLEIC ACIDS RESEARCH, vol. 1, 12 July 2019 (2019-07-12), GB, pages 1 - 13, XP055606543, ISSN: 0305-1048, DOI: 10.1093/nar/gkz605
- MICHELLE E. HUNG ET AL: "A platform for actively loading cargo RNA to elucidate limiting steps in EV-mediated delivery", JOURNAL OF EXTRACELLULAR VESICLES, vol. 5, no. 1, 1 January 2016 (2016-01-01), pages 31027, XP055711976, DOI: 10.3402/jev.v5.31027
- KOSTYUSHEV DMITRY ET AL: "Gene Editing by Extracellular Vesicles", INTERNATIONAL JOURNAL OF MOLECULAR SCIENCES, vol. 21, no. 19, 5 October 2020 (2020-10-05), pages 7362, XP055935379, Retrieved from the Internet <URL:https://www.ncbi.nlm.nih.gov/pmc/articles/PMC7582630/pdf/ijms-21-07362.pdf> DOI: 10.3390/ijms21197362
- YAO XINGANG, LYU PIN, YOO KYUNG, YADAV MANISH KUMAR, SINGH RAVI, ATALA ANTHONY, LU BAISONG: "Engineered extracellular vesicles as versatile ribonucleoprotein delivery vehicles for efficient and safe CRISPR genome editing", JOURNAL OF EXTRACELLULAR VESICLES, TAYLOR & FRANCIS, UK, vol. 10, no. 5, 16 March 2021 (2021-03-16), UK , XP055897973, ISSN: 2001-3078, DOI: 10.1002/jev2.12076

## Description

### FIELD

This disclosure describes compositions and methods of using same for eukaryotic gene editing.

### REFERENCE TO A SEQUENCE LISTING SUBMITTED AS A TEXT FILE VIA EFS-WEB

The official copy of the sequence listing is submitted electronically via EFS-Web as an ASCII formatted sequence listing with a file named 1293190_seqlist.txt, created on February 7, 2022, and having a size of 121 KB and is filed concurrently with the specification. The sequence listing contained in this ASCII formatted document is part of the specification.

### BACKGROUND

CRISPR-based genome editing effectors is important to reduce off-target effects and immune responses. Recently extracellular vesicles (EVs) have been explored for Cas9 ribonucleoprotein (RNP) delivery. However, the efficiency of these EVs as a RNP delivery vehicle was limited. Thus, compositions and methods for efficient packing of functional RNPs into EVs are necessary.

Ye et al., Biomaterials Science, 2020, vol. 8, no. 10, pages 2966 - 2976, discloses exosomes that were engineered by fusing GFP and GFP nanobody with exosomal membrane protein CD63 and Cas9 protein, respectively, to capture and load Cas9 proteins into exosomes. Kostyushev et al., International Journal of Molecular Sciences, vol. 21, no. 19, page 7362, reviews gene editing using extracellular vesicles. WO2020/099682 discloses extracellular vesicles for treating urea cycle disorders which utilize a construct of CD63-PUF to load mRNA into extracellular vesicles.

### SUMMARY

The invention is defined by the appended claims. Provided herein are plasmid systems and extracellular vesicles for the delivery of nucleic acid sequences (e.g., mRNA sequence encoding a heterologous polypeptide) and heterologous polypeptides to a cell. Ex vivo methods for modifying cells using the plasmid systems and extracellular vesicles described herein are also provided.

Provided herein is a plasmid system comprising: (a) a first mammalian expression plasmid comprising a eukaryotic promoter operably linked to a nucleic acid sequence, wherein the nucleic acid sequence comprises: (i) a nucleic acid sequence encoding a CRISPR-associated endonuclease; and a guide RNA (gRNA) coding sequence, wherein the gRNA coding sequence comprises at least one Com aptamer coding sequence; or (ii) a nucleic acid encoding a heterologous polypeptide and at least one Com aptamer coding sequence; and (b) a second mammalian expression plasmid comprising a eukaryotic promoter operably linked to a nucleic acid sequence encoding a fusion protein comprising CD63 and at least one Com aptamer binding protein, wherein the Com aptamer binding protein (ABP) binds to the at least one Com aptamer coding sequence of the first mammalian expression plasmid. In some embodiments, the plasmid system further comprises an envelope plasmid comprising a nucleic acid sequence encoding vesicular stomatis virus G (VSV G) protein.

In some embodiments, the CRISPR-associated endonuclease is a Cas9 protein, a Cpfl protein or a derivative of either. In some embodiments, the CRISPR-associated endonuclease is a catalytically impaired CRISPR-associated endonuclease. In some embodiments, the catalytically impaired CRISPR-associated endonuclease coding sequence encodes a Cas9 D10A protein.

In some embodiments, the nucleic acid sequence of the first mammalian expression plasmid encodes a nucleic acid sequence encoding an adenosine base pair editor (ABE), wherein the ABE is a fusion protein comprising an adenosine deaminase and the catalytically impaired CRISPR-associated endonuclease. In some embodiments, the adenine base editor is ABE 7.10 or ABE8.

In some embodiments, the fusion protein comprises a first Com ABP fused to the N-terminus of CD63 and a second Com ABP fused to the C-terminus of CD63.

In some embodiments, the sgRNA coding sequence comprises at least one com aptamer sequence inserted into the tetraloop or the ST2 loop of the gRNA coding sequence.

Also provided is an extracellular vesicle comprising: (a) a ribonucleotide protein (RNP) complex comprising: (i) a CRISPR-associated endonuclease; and a gRNA comprising at least one Com aptamer coding sequence; or (ii) a mRNA encoding a heterologous polypeptide and at least one Com aptamer coding sequence; and (b) a fusion protein comprising CD63 and at least one Com aptamer binding protein (ABP), wherein the Com ABP binds to the at least one Com aptamer coding sequence. In some embodiments, the extracellular vesicle further comprises a VSV-G protein.

In some embodiments, the CRISPR-associated endonuclease is a Cas9 protein, a Cpfl protein or a derivative of either. In some embodiments, the CRISPR-associated endonuclease is a catalytically impaired CRISPR-associated endonuclease. In some embodiments, the catalytically impaired CRISPR-associated endonuclease coding sequence encodes a Cas9 D10A protein. In some embodiments, the RNP comprises an adenine base pair editor (ABE), wherein the ABE is a fusion protein comprising an adenosine deaminase and the catalytically impaired CRISPR-associated endonuclease. In some embodiments, the adenine base editor is ABE 7.10 or ABE8.

In some embodiments, the fusion protein comprises a first Com ABP fused to the N-terminus of CD63 and a second Com ABP fused to the C-terminus of CD63.

In some embodiments, the sgRNA coding sequence comprises at least one com aptamer sequence inserted into the tetraloop or the ST2 loop of the gRNA coding sequence. In some embodiments, the extracellular vesicle is an exosome or a microvesicle.

Further provided is a method of producing an extracellular vesicle, the method comprising: (a) transfecting a plurality of eukaryotic cells with the first mammalian expression plasmid and the second mammalian expression plasmid of any of the plasmid systems described herein; and (b) culturing the transfected eukaryotic cells for sufficient time for extracellular vesicles to be produced. In some embodiments, the method further comprises transfecting the plurality of eukaryotic cells with the envelope plasmid of any of the plasmid systems described herein.

In some embodiments, the extracellular vesicle comprises: (a) a RNP comprising: (i) a CRISPR-associated endonuclease; and (ii) a gRNA comprising at least one Com aptamer coding sequence; and (b) a fusion protein comprising CD63 and at least one Com aptamer binding protein, wherein the Com aptamer binding protein (ABP) binds to the at least one Com aptamer coding sequence. In some embodiments, the extracellular vesicle comprises: (a) (i) a RNP comprising: a CRISPR-associated endonuclease; and a gRNA comprising at least one Com aptamer coding sequence, or (ii) an mRNA encoding the heterologous polypeptide and the at least one Com aptamer sequence; (b) a fusion protein comprising CD63 and at least one Com aptamer binding protein, wherein the Com aptamer binding protein (ABP) binds to the Com aptamer coding sequence; and(c) a VSV-G protein.

In some embodiments, the plurality of eukaryotic cells are mammalian cells. In some embodiments, the method further comprises isolating the extracellular vesicles from the cultured transfected eukaryotic cells.

Also provided is an extracellular vesicle made by any of the methods provided herein.

Further provided is an ex vivo method of modifying a genomic target sequence in a cell, the method comprising transducing a plurality of eukaryotic cells with a plurality of extracellular vesicles, wherein the plurality of extracellular vesicles comprises an extracellular vesicles described herein, wherein the RNP binds to the genomic target sequence in genomic DNA of the cell, thereby modifying the genomic target sequence. In some embodiments, the plurality of eukaryotic cells are mammalian cells. In some embodiments, the plurality of eukaryotic cells are cells present in a subject. In some embodiments, the subject is a human subject. In some embodiments, the subject is injected with the plurality of extracellular vesicles.

Also provided is a cell containing any of the plasmid systems described herein. A cell modified using any of the methods for modifying a cell described herein is also provided.

Further provided is a plurality of extracellular vesicles comprising an extracellular vesicles described herein, for use in a method for treating a disease in a subject comprising: a) providing cells obtained from the subject; b) modifying the cells of the subject using any of the methods for modifying a cell described herein; and c) administering the modified cells to the subject. In some embodiments, the disease is cancer. In some embodiments, the disease is sickle cell anemia. In some embodiments, the cells are T cells.

### DESCRIPTION OF THE FIGURES

The present application includes the following figures. The figures are intended to illustrate certain embodiments and/or features of the compositions and methods, and to supplement any description(s) of the compositions and methods. The figures do not limit the scope of the compositions and methods, unless the written description expressly indicates that such is the case.

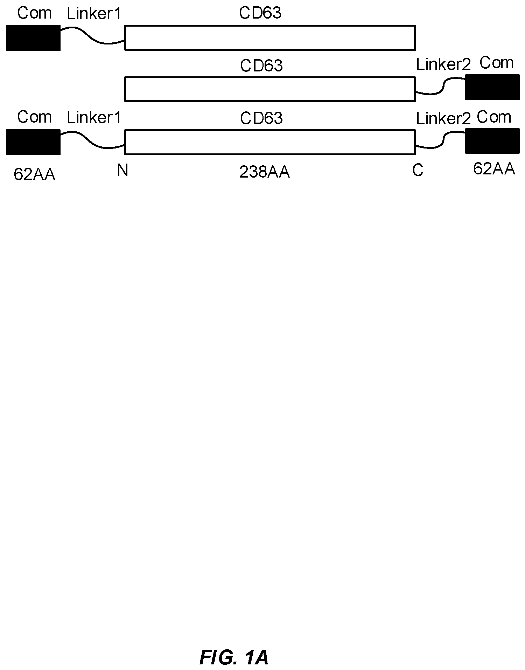

FIG. 1A illustrates a fusion protein comprising Com and CD63 according to certain aspects of this disclosure. Com was fused to the N-terminus, C-terminus or both termini of CD63 with linker peptide in between. The sequences for linker 1 and linker 2 were "GGHNSGGGGGQSPGPAA" (SEQ ID NO: 115) and "SGGGGSMASNFTQFVLVDNGGTGDV" (SEQ ID NO: 116) respectively.

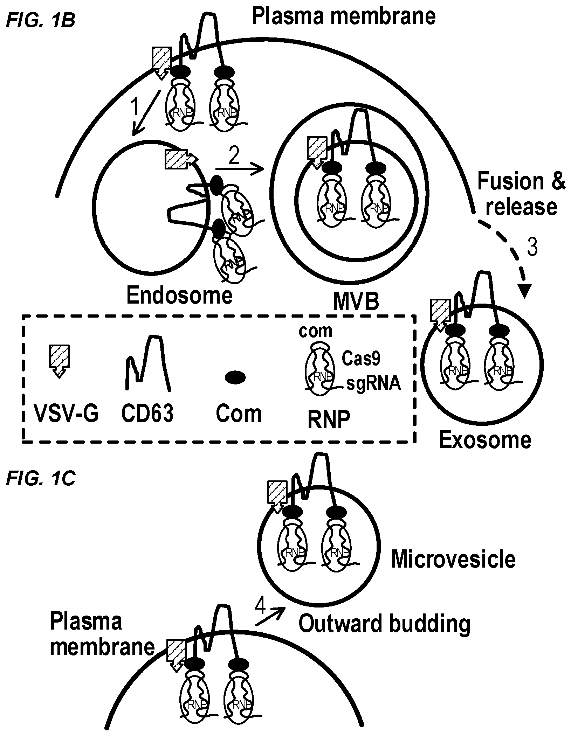

FIG. 1B is a diagram showing the recruitment of Cas9 or ABE RNPs into exosomes according to certain aspects of this disclosure. RNPs associate with Com-CD63-Com on the plasma membrane through com/Com interaction and then enter the endosome system via endocytosis (step 1). The early endosomes become multivesicular bodies (MVB) following the inward budding of the outer endosomal membrane (step 2). The intraluminal vesicles are released into the medium as exosomes when the membranes of MVBs fuse with the cell membrane (step 3). Here Com is drawn as a monomer but may function as a homodimer.
FIG. 1C shows the recruitment of Cas9 RNPs into microvesicles (step 4) according to certain aspects of this disclosure.

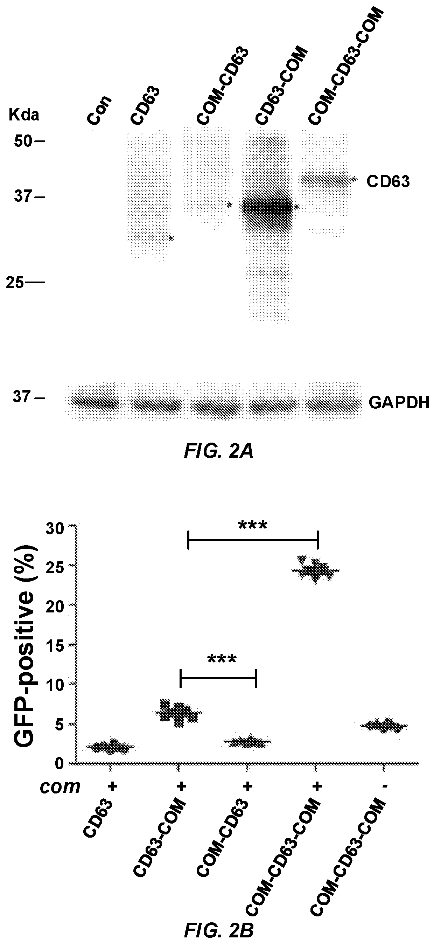

FIG. 2A is a Western blot showing expression of Com and CD63 fusion proteins according to certain aspects of this disclosure. Plasmid DNA for CD63, CD63-Com, Com-CD63 and Com-CD63-Com expression were transfected into HEK293T cells and the expression of CD63 or CD63-fusion proteins were detected by anti-CD63 antibody. GAPDH was detected for loading control.
FIG. 2B shows flow cytometry detection of gene editing activities according to certain aspects of this disclosure. 2.5x10⁴ *HBB-IL2RG* GFP reporter cells were treated with RNP-enriched EVs secreted by 0.6 million cells in 48 hours. The RNPs were IL2RG-targeting SaCas9 RNPs (n=6). ***, p<0.0001 between the indicated conditions (Tukey posttests following ANOVA).

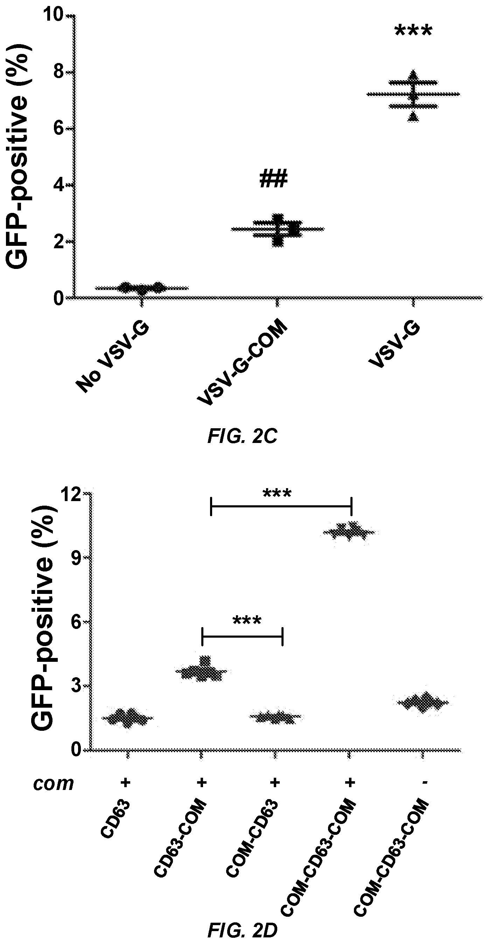

FIG. 2C shows that vesicular stomatitis virus- G (VSV-G) improves gene editing activity of the EV-delivered RNPs according to certain aspects of this disclosure. *IL2RG-*targeting SaCas9 RNPs were packaged into EVs with or without VSV-G protein. The RNP-enriched EVs were added to *HBB-IL2RG* GFP reporter cells to examine GFP-positive cells by flow cytometry (n=3). ##, p<0.01 compared with the group without VSV-G; ***, p<0.0001 when compared with all other conditions (Tukey posttests following ANOVA).
FIG. 2D shows detection of gene editing activities of EV-delivered SpCas9 RNPs by flow cytometry according to certain aspects of this disclosure. *HBB-IL2RG* GFP reporter cells (2.5x10⁴) were treated with RNP-enriched EVs secreted by 0.6 million cells in 48 hours. The RNPs were *IL2RG-*targeting SpCas9 RNPs. ***, p<0.0001 between the indicated conditions (n=3, Tukey posttests following ANOVA).

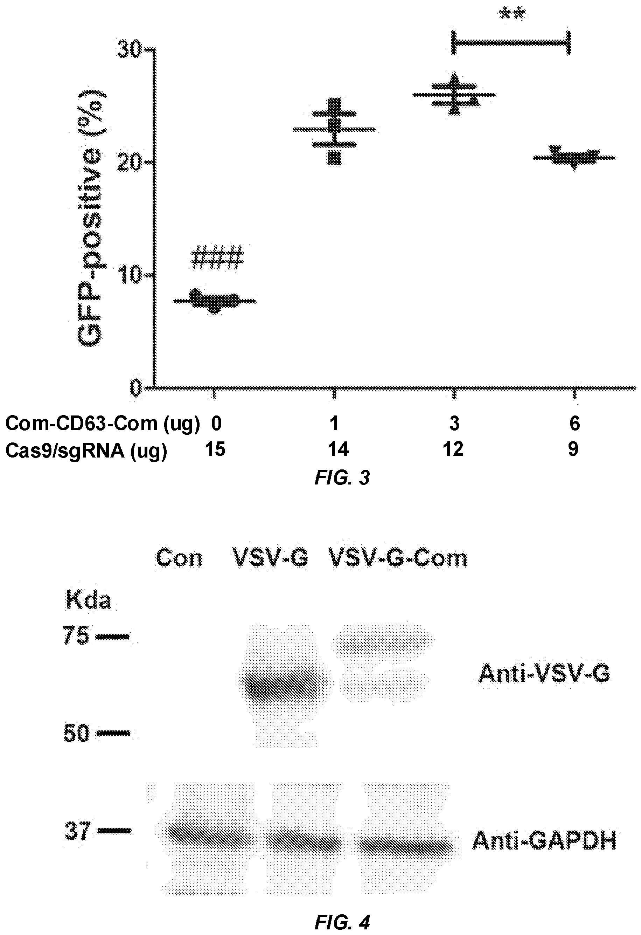

FIG. 3 shows optimization of DNA ratios for most efficient production of RNP loaded EVs according to certain aspects of this disclosure. The indicated amount of pCom-CD63-Com and pX601-Tetra-com-*IL2RG* DNA was used in transfection for making the RNP-loaded EVs. The RNP-loaded EVs produced by 0.5 million cells in 48 hours were concentrated and added to 2.5x10⁴ *HBB-IL2RG* GFP reporter cells. GFP-positive cells were detected by flow cytometry. *** indicates p<0.0001 between the indicated conditions (n=3, Tukey posttests following ANOVA).
FIG. 4 is a Western blot showing detection of VSV-G and VSV-G-Com fusion protein according to certain aspects of this disclosure. Control cells were mock transfected.

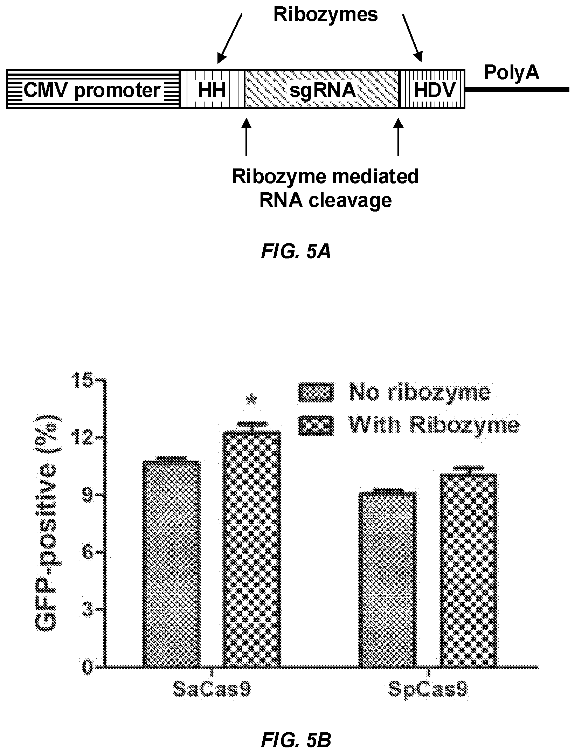

FIG. 5A is a schematic of sgRNA driven by RNA polymerase II promoter and flanked by the Hammerhead (HH) ribozyme and hepatitis delta virus (HDV) ribozyme. The sgRNAs were *IL2RG-*targeting sgRNAs for SaCas9 and SpCas9.
FIG. 5B is a comparision of gene editing activities of EV delivered SaCas9 or SpCas9 RNPs (targeting *IL2RG* and *DMD* exon 53 respectively), with or without extra sgRNA, according to certain aspects of this disclosure. RNP-loaded EVs were prepared with and without the respective plasmid DNA shown in FIG. 5A. Gene editing activities were assayed in *HBB-IL2RG* GFP reporter cells by flow cytometry.

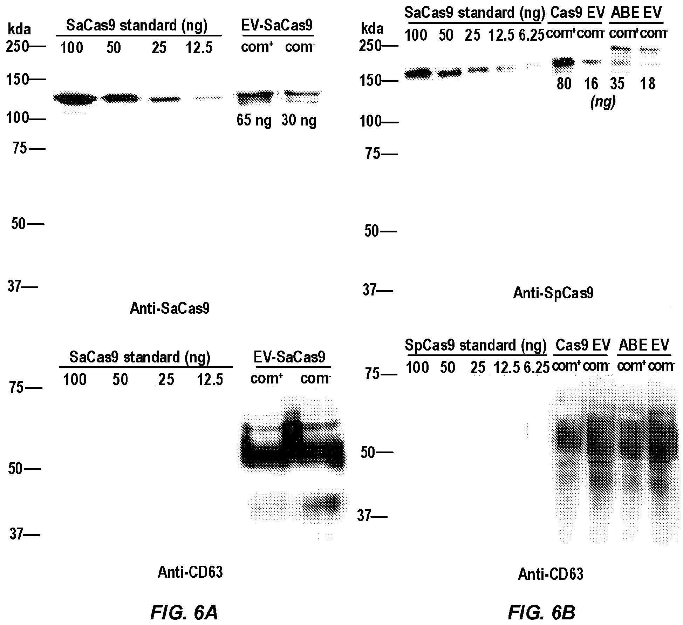

FIG. 6A shows enrichment of SaCas9 RNPs in EVs according to certain aspects of this disclosure. Com-CD63-Com, SaCas9 and *IL2RG* sgRNA with and without com modification were co- expressed in 5x10⁶ HEK293T cells, EVs were collected for 48 hours. One-fifth of the EVs were analyzed by Western blot. Numbers under protein bands were estimated protein mass (ng) based on protein standards.
FIG. 6B shows enrichment of SpCas9 RNPs and ABE into EVs according to certain aspects of this disclosure. *IL2RG* and Site 5 were targeted for SpCas9 and ABE respectively. Experimental conditions were similar as in FIG. 6A.

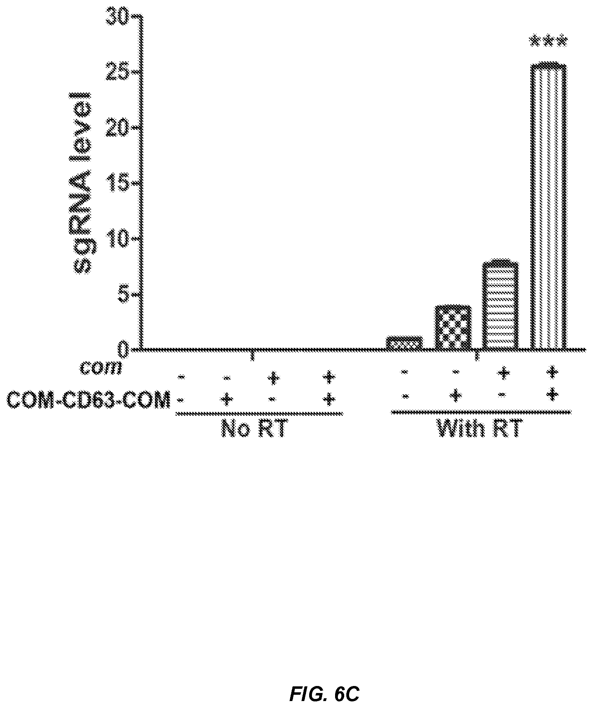

FIG. 6C shows Com and com dependent enrichment of sgRNA into EVs according to certain aspects of this disclosure. SaCas9 and *IL2RG-*targeting sgRNA were packaged in EVs (n=3). sgRNA was unmodified (com-) or with a com replacing the Tetra loop (com+). In EVs without Com-CD63-Com, CD63 was overexpressed instead. RNAs were extracted from EVs and the sgRNA was detected by RT-qPCR using primers Scid-g2F and sgRNA-R3. ***, p<0.0001 when compared with all other conditions (Tukey posttests following ANOVA).

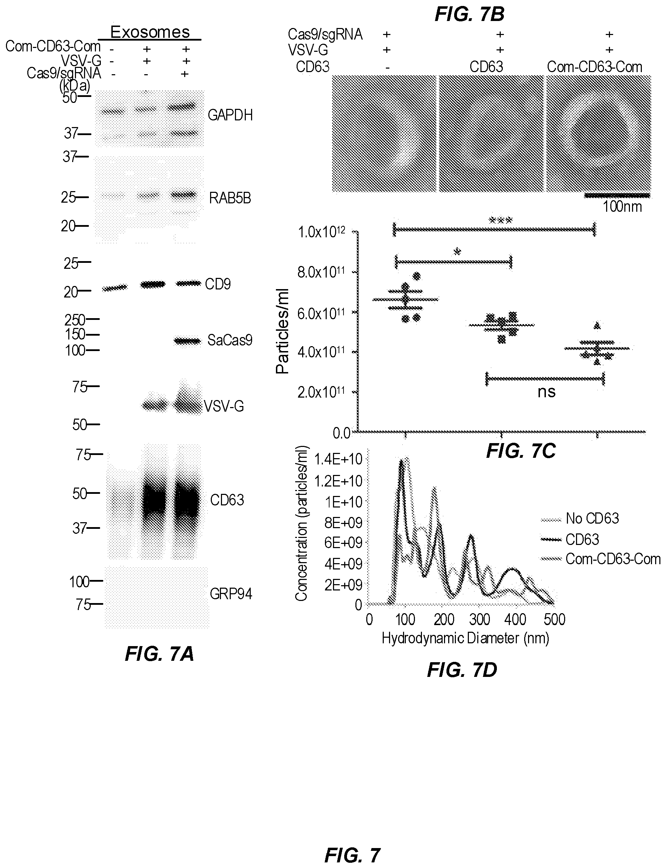

FIG. 7A is a Western blot analysis of proteins in EVs according to certain aspects of this disclosure. EVs were collected from cells with or without *IL2RG*-targeting SaCas9 RNPs. 1/20^{th} of the EVs secreted by 1 million cells, in 48 hours, and were loaded.
FIG. 7B shows transmission electric microscopy analysis of EVs according to certain aspects of this disclosure.
FIG. 7C shows nanoparticle tracking analysis of EV particle concentrations according to certain aspects of this disclosure.
FIG. 7D shows nanoparticle tracking analysis of EV size distribution according to certain aspects of this disclosure. EVs secreted by 5 million cells in 48 hours were re-suspended in 500 µl PBS for analysis in FIGS. 7B, 7C and 7D.

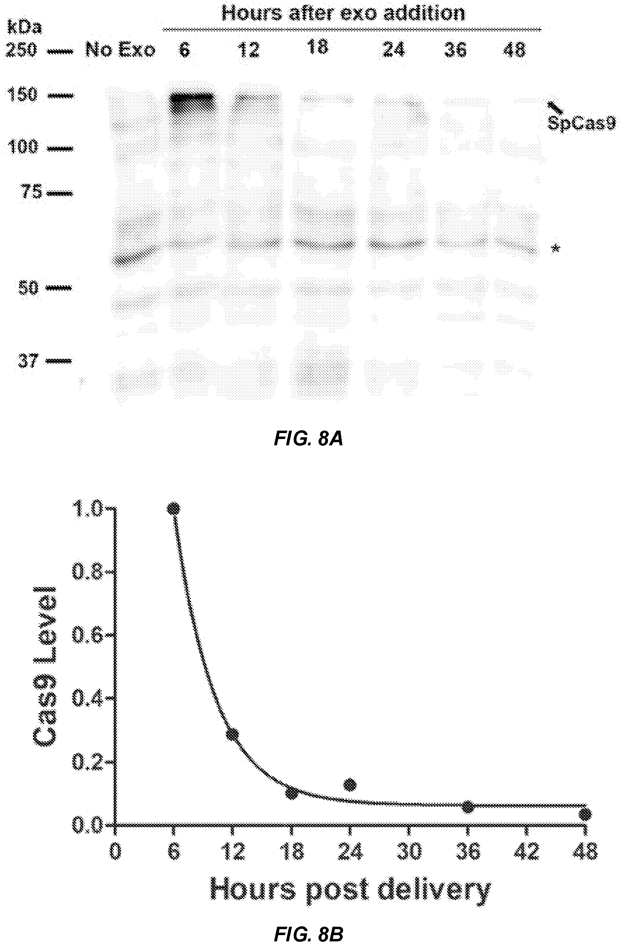

FIG. 8A is a Western blot of degradation of EV delivered SpCas9 RNPs according to certain aspects of this disclosure. The arrow indicates the position of SpCas9 protein. The asterisk indicates a nonspecific band as the indication of similar loading. EVs secreted by 0.2 million cells in 48 hours were added to 2.5x10⁴ HEK293T cells.
FIG. 8B shows densitometry analysis (IMAGEJ) of Cas9 protein levels, at various times, according to certain aspects of this disclosure. Half-life was estimated using one phase decay (GraphPad Prism 5).

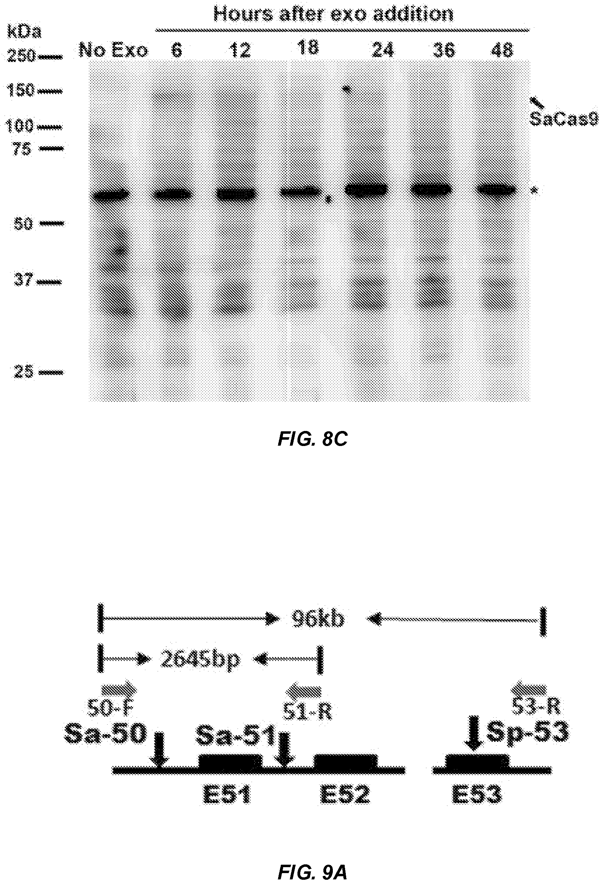

FIG. 8C is a Western blot of degradation of EV delivered SaCas9 RNPs according to certain aspects of this disclosure. The arrow indicates the position of SaCas9 protein. The asterisk indicates a nonspecific band as the indication of similar loading. EVs secreted by 0.2 million cells in 48 hours were added to 2.5x10⁴ HEK293T cells.
FIG. 9A is a diagram showing an exemplary strategy to detect co-targeting of two loci according to certain aspects of this disclosure. The three sgRNAs, Sa-50, Sa-51 and Sp-53, target DMD intron 50, intron 51 and exon 53 respectively. The primers, 50-F, 51-R and 53-R used for PCR detection of deletions are also shown. The solid boxes indicate hDMD exons 51 to 53. Distances between primers before sequence removal are listed.

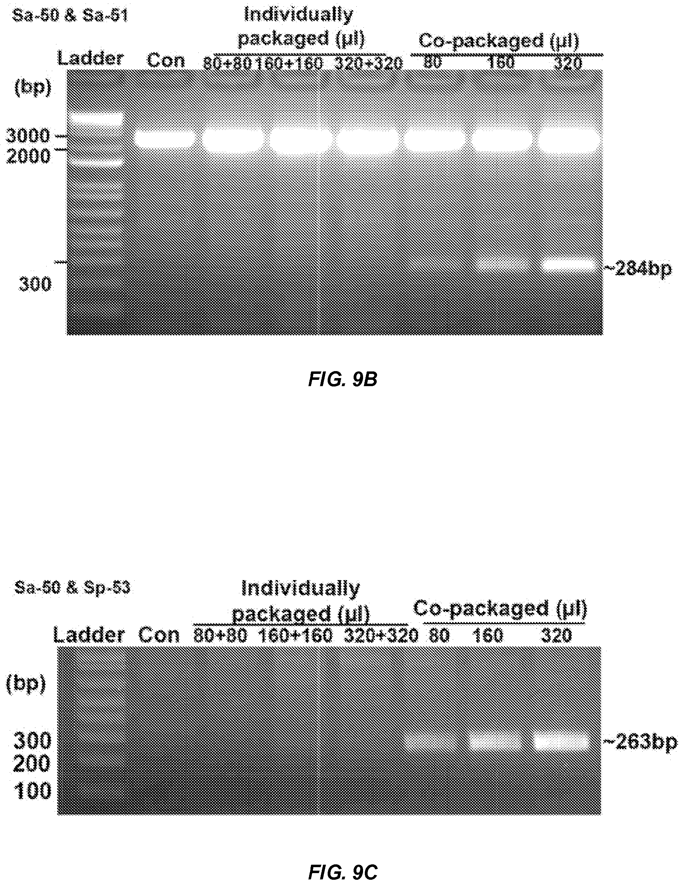

FIG. 9B shows that co-packaged SaCas9 RNPs targeting different loci were more efficient in multiplex genome editing, according to certain aspects of this disclosure.
FIG. 9C shows that SaCas9 RNPs and SpCas9 RNPs could be co-packaged in EVs for efficient multiplex genome editing, according to certain aspects of this disclosure.

### Definitions

As used in this specification and the appended claims, the singular forms "a," "an," and "the" include plural reference unless the context clearly dictates otherwise.

The use herein of the terms "including," "comprising," or "having," and variations thereof, is meant to encompass the elements listed thereafter and equivalents thereof as well as additional elements. Embodiments recited as "including," "comprising," or "having" certain elements are also contemplated as "consisting essentially of and "consisting of those certain elements. As used herein, "and/or" refers to and encompasses any and all possible combinations of one or more of the associated listed items, as well as the lack of combinations where interpreted in the alternative ("or").

As used herein, the transitional phrase "consisting essentially of" (and grammatical variants) is to be interpreted as encompassing the recited materials or steps "and those that do not materially affect the basic and novel characteristic(s)" of the claimed invention. See In re Herz, 537 F.2d 549, 551-52, 190 U.S.P.Q. 461, 463 (CCPA 1976) (emphasis in the original); see also MPEP §2111.03. Thus, the term "consisting essentially of" as used herein should not be interpreted as equivalent to "comprising."

The term "nucleic acid" or "nucleotide" refers to deoxyribonucleic acids (DNA) or ribonucleic acids (RNA) (e.g., mRNA) and polymers thereof in either single- or double-stranded form. It is understood that when an RNA is described, its corresponding DNA is also described, wherein uridine is represented as thymidine. Similarly, when a DNA is described, its corresponding RNA is also described wherein thymidine is represented by uridine. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). The polynucleotides of the invention also encompass all forms of sequences including, but not limited to, single-stranded forms, double-stranded forms, hairpins, stem-and-loop structures, and the like.

The term "gene" can refer to the segment of DNA involved in producing or encoding a polypeptide chain. It may include regions preceding and following the coding region (leader and trailer) as well as intervening sequences (introns) between individual coding segments (exons). Alternatively, the term "gene" can refer to the segment of DNA involved in producing or encoding a non-translated RNA, such as an rRNA, tRNA, guide RNA, or micro RNA.

As used herein the phrase "heterologous" refers to what is not normally found in nature. The term "heterologous polypeptide" refers to a polypeptide not normally found in a given cell in nature. As such, a heterologous polypeptide may be: (a) foreign to its host cell (i.e., is exogenous to the cell); or (b) naturally found in the host cell (i.e., endogenous) but present at an unnatural quantity in the cell (i.e., greater or lesser quantity than naturally found in the host cell). The term "heterologous promoter" refers to a promoter sequence not normally found in a given cell in nature or not normally found operably linked to polynucleotide expressing a given protein.

"Treating" refers to any indicia of success in the treatment or amelioration or prevention of the disease, condition, or disorder, including any objective or subjective parameter such as abatement; remission; diminishing of symptoms or making the disease condition more tolerable to the patient; slowing in the rate of degeneration or decline; or making the final point of degeneration less debilitating. The treatment or amelioration of symptoms can be based on objective or subjective parameters; including the results of an examination by a physician. Accordingly, the term "treating" includes the administration of the compounds or agents of the present disclosure to prevent or delay, to alleviate, or to arrest or inhibit development of the symptoms or conditions associated with a disease, condition or disorder as described herein. The term "therapeutic effect" refers to the reduction, elimination, or prevention of the disease, symptoms of the disease, or side effects of the disease in the subject. "Treating" or "treatment" using the methods of the present disclosure includes preventing the onset of symptoms in a subject that can be at increased risk of a disease or disorder associated with a disease, condition or disorder as described herein, but does not yet experience or exhibit symptoms, inhibiting the symptoms of a disease or disorder (slowing or arresting its development), providing relief from the symptoms or side effects of a disease (including palliative treatment), and relieving the symptoms of a disease (causing regression). Treatment can be prophylactic (to prevent or delay the onset of the disease, or to prevent the manifestation of clinical or subclinical symptoms thereof) or therapeutic suppression or alleviation of symptoms after the manifestation of the disease or condition. The term "treatment," as used herein, includes preventative (e.g., prophylactic), curative, or palliative treatment.

A "promoter" is defined as one or more a nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription.

"Polypeptide," "peptide," and "protein" are used interchangeably herein to refer to a polymer of amino acid residues. All three terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass full-length proteins, truncated proteins, and fragments thereof, and amino acid chains, wherein the amino acid residues are linked by covalent peptide bonds. As used throughout, the term "fusion polypeptide" or "fusion protein" is a polypeptide comprising two or more proteins or fragments thereof. In some embodiments, a linker comprising about 3 to 10 amino acids can be positioned between any two proteins or fragments thereof to help facilitate proper folding of the proteins upon expression.

The term "identity" or "substantial identity", as used in the context of a polynucleotide or polypeptide sequence described herein, refers to a sequence that has at least 60% sequence identity to a reference sequence. Alternatively, percent identity can be any integer from 60% to 100%. Exemplary embodiments include at least: 60%, 65%, 70%, 75%, 80%, 85%, 90%, 91%, 92%, 93%, 94%, 95%, 96%, 97%, 98%, or 99%, as compared to a reference sequence using the programs described herein; preferably BLAST using standard parameters, as described below. It is understood that sequences having at 60%, 65%, 70%, 75%, 80%, 85%, 90%, 91%, 92%, 93%, 94%, 95%, 96%, 97%, 98%, or 99% identity to any nucleotide or polypeptide sequence set forth herein, for example, any one of SEQ ID NOs: 1-49, can be used in the compositions and methods provided herein. It is understood that a nucleic acid sequence can comprise, consist of, or consist essentially of any nucleic acid sequence described herein. Similarly, a polypeptide can comprise, consist of, or consist essentially of, any polypeptide sequence described herein. For sequence comparison, typically one sequence acts as a reference sequence to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, about 20 to 50, about 20 to 100, about 50 to about 200 or about 100 to about 150, in which a sequence may be compared to a reference sequence of the same number of contiguous positions after the two sequences are optimally aligned. Methods of alignment of sequences for comparison are well-known in the art. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (U.S.A.) 85: 2444 (1988), by computerized implementations of these algorithms (e.g., BLAST), or by manual alignment and visual inspection.

Algorithms that are suitable for determining percent sequence identity and sequence similarity are the BLAST and BLAST 2.0 algorithms, which are described in Altschul et al. (1990) J. Mol. Biol. 215: 403-410 and Altschul et al. (1977) Nucleic Acids Res. 25: 3389-3402, respectively. Software for performing BLAST analyses is publicly available through the National Center for Biotechnology Information (NCBI) web site. The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al, supra). These initial neighborhood word hits acts as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands.

The BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.01, more preferably less than about 10-5, and most preferably less than about 10-20.

As used throughout, by subject is meant an individual. For example, the subject is a mammal, such as a primate, and, more specifically, a human. Non-human primates are subjects as well. The term subject includes domesticated animals, such as cats, dogs, etc., livestock (for example, cattle, horses, pigs, sheep, goats, etc.) and laboratory animals (for example, ferret, chinchilla, mouse, rabbit, rat, gerbil, guinea pig, etc.). Thus, veterinary uses and medical uses and formulations are contemplated herein. The term does not denote a particular age or sex. Thus, adult and newborn subjects, whether male or female, are intended to be covered. As used herein, patient or subject may be used interchangeably and can refer to a subject afflicted with a disease or disorder.

An "expression cassette" is a nucleic acid construct, generated recombinantly or synthetically, with a series of specified nucleic acid elements that permit transcription of a particular polynucleotide sequence in a host cell. An expression cassette may be part of a plasmid, viral genome, or nucleic acid fragment. Typically, an expression cassette includes a polynucleotide to be transcribed, operably linked to a promoter, followed by a transcription termination signal sequence. An expression cassette may or may not include specific regulatory sequences, such as 5' or 3' untranslated regions from human globin genes.

A "reporter gene" encodes proteins that are readily detectable due to their biochemical characteristics, such as enzymatic activity or chemifluorescent features. These reporter proteins can be used as selectable markers. One specific example of such a reporter is green fluorescent protein. Fluorescence generated from this protein can be detected with various commercially-available fluorescent detection systems. Other reporters can be detected by staining. The reporter can also be an enzyme that generates a detectable signal when contacted with an appropriate substrate. The reporter can be an enzyme that catalyzes the formation of a detectable product. Suitable enzymes include, but are not limited to, proteases, nucleases, lipases, phosphatases and hydrolases. The reporter can encode an enzyme whose substrates are substantially impermeable to eukaryotic plasma membranes, thus making it possible to tightly control signal formation. Specific examples of suitable reporter genes that encode enzymes include, but are not limited to, CAT (chloramphenicol acetyl transferase; Alton and Vapnek (1979) Nature 282: 864-869); luciferase (lux); β-galactosidase; LacZ; β.-glucuronidase; and alkaline phosphatase (Toh, et al. (1980) Eur. J. Biochem. 182: 231-238; and Hall et al. (1983) J. Mol. Appl. Gen. 2: 101. Other suitable reporters include those that encode for a particular epitope that can be detected with a labeled antibody that specifically recognizes the epitope.

The "CRISPR/Cas" system refers to a widespread class of bacterial systems for defense against foreign nucleic acid. CRISPR/Cas systems are found in a wide range of eubacterial and archaeal organisms. CRISPR/Cas systems include type I, II, and III sub-types. The CRISPR/Cas system classification as described in by Makarova, et al. (Nat Rev Microbiol. 2015 Nov; 13(11):722-36) defines five types and 16 subtypes based on shared characteristics and evolutionary similarity. These are grouped into two large classes based on the structure of the effector complex that cleaves genomic DNA. The Type II CRISPR/Cas system was the first used for genome engineering, with Type V following in 2015. Wild-type type II CRISPR/Cas systems utilize an RNA-mediated nuclease Cas protein or homolog (referred to herein as a "CRISPR-associated endonuclease") in complex with guide RNA to recognize and cleave foreign nucleic acid. Cas9 proteins also use an activating RNA (also referred to as a transactivating or tracr RNA). Guide RNAs having the activity of either a guide RNA or both a guide RNA and an activating RNA, depending on the type of CRISPR-associated endonuclease used therewith, are also known in the art. In some cases, such dual activity guide RNAs are referred to as a single guide RNA (sgRNA). Synthetic guide RNAs that do not contain an activating RNA sequence may also be referred to as sgRNAs. In this disclosure, the terms sgRNA and gRNA are used interchangeably to refer to an RNA molecule that complexes with a CRISPR-associated endonuclease and localizes the ribonucleoprotein complex to a target DNA sequence.

In the compositions, methods, and compositions for use in the methods provided herein, the CRISPR-associated endonuclease can be a catalytically impaired nuclease. As used throughout, "catalytically impaired" refers to decreased CRISPR-associated endonuclease enzymatic activity for cleaving one or both strands of DNA. Examples of catalytically impaired CRISPR-associated endonuclease include but are not limited to catalytically impaired Cas9, catalytically impaired Cpfl and catalytically impaired C2c2. In some instances, the catalytically impaired CRISPR-associated endonuclease is a catalytically impaired Cas9, for example Cas9 D10A, which cleaves or nicks only one strand of DNA. In some instances, the CRISPR-associated endonuclease may be a catalytically impaired CRISPR-associated endonuclease, wherein the endonuclease cannot cleave both strands of a double-stranded DNA molecule, i.e., cannot make a double-stranded break. Modifications include, but are not limited to, altering one or more amino acids to inactivate the nuclease activity or the nuclease domain. For example, and not to be limiting, D10A and/or H840A mutations can be made in Cas9 from Streptococcus pyogenes to reduce or inactivate Cas9 nuclease activity. Other modifications include removing all or a portion of the nuclease domain of Cas9, such that the sequences exhibiting nuclease activity are absent from Cas9. Accordingly, a catalytically impaired Cas9 may include polypeptide sequences modified to reduce nuclease activity or removal of a polypeptide sequence or sequences to reduce nuclease activity. The catalytically impaired Cas9 retains the ability to bind to DNA even though the nuclease activity has been inactivated. Accordingly, a catalytically impaired Cas9 includes the polypeptide sequence or sequences required for DNA binding but includes modified nuclease sequences or lacks nuclease sequences responsible for nuclease activity. It is understood that similar modifications can be made to reduce nuclease activity in other site-directed nucleases, for example in Cpf1 or C2c2. In some examples, the Cas9 protein is a full-length Cas9 sequence from S. pyogenes lacking the polypeptide sequence of the RuvC nuclease domain and/or the HNH nuclease domain and retaining the DNA binding function. In other examples, the Cas9 protein sequences have at least 30%, 40%, 50%, 60%, 70%, 80%, 90%, 95%, 98% or 99% identity to Cas9 polypeptide sequences lacking the RuvC nuclease domain and/or the HNH nuclease domain and retains DNA binding function.

As used herein, "activity" in the context of sgRNA activity, or RNP activity, i.e., RNP activity of a complex comprising: (1) a gRNA and (2) a CRISPR-associated endonuclease, refers to the ability of a sgRNA to bind to a target genetic element. Typically, activity also refers to the ability of an RNP (i.e., and sgRNA complexed with a CRISPR-associated endonuclease) to edit the genome of a cell. In some examples, activity refers to the ability of an ABE RNP (i.e., an sgRNA complexed with an ABE) to edit base pairs, i.e., perform an A to G change in one strand of DNA.

As used herein, the phrase "editing" in the context of editing of a genome of a cell refers to inducing a structural change in the sequence of the genome at a target genomic region, for example, cleaving a genomic sequence and inserting a donor sequence into the genome of a cell, at the cleavage site, via homology directed repair (HDR), or cleaving a sequence and allowing repair via non-homologus end joining (NHEJ). In some examples editing is performed by an ABE. For example, the editing can take the form of an A to G change in one strand of DNA (or a T to C change on the opposite strand of DNA) at a target genomic region. The nucleotide sequence can encode a polypeptide or a fragment thereof. See, for example, Gaudelli et al., "Programmable base editing of A-T to G-C in genomic DNA without DNA cleavage," Nature 551: 464-471 (2017).

As used herein, "an adenine base editor" or "ABE" refers to a fusion protein comprising an adenosine deaminase and a catalytically impaired CRISPR-associated endonuclease. In some instances, the adenosine deaminase is a tadA enzyme that deaminates adenine on a single-strand of DNA to form inosine. See, Gaudelli et al, (2017). In some instances, the ABE is a fusion protein comprising a catalytically impaired CRISPR-associated endonuclease and one or more copies, for example, two, three, four copies, etc. of an adenosine deaminase. In some instances the ABE comprises the fusion protein is encoded by a nucleic acid sequence comprising SEQ ID NO: 27. In some instances, the ABE comprises SEQ ID NO: 28.

As used herein, the term "ribonucleoprotein complex" "RNPs", and the like refers to a complex between: (1) a CRISPR-associated endonuclease, and a crRNA (e.g., guide RNA or single guide RNA), (2) a CRISPR-associated endonuclease and a trans-activating crRNA (tracrRNA), (3) a CRISPR-associated endonuclease and a guide RNA, or (4) a combination thereof (e.g., a complex containing the CRISPR-associated endonuclease and the catalytically impaired Cas9 protein, a tracrRNA, and a crRNA guide). In some embodiments, the CRISPR-associated endonuclease is catalytically impaired. In some embodiments, the catalytically impaired CRISPR-associate endonuclease is fused to an adenosine deaminase.

As used herein, a "cell" can be any eukaryotic cell, for example, human T cell or a cell capable of differentiating into a T cell, for example, a T cell that expresses a TCR receptor molecule. These include hematopoietic stem cells and cells derived from hematopoietic stem cells. Populations of cells, for example, populations of cells comprising viral particles or genetically modified cells made by any of the genomic editing methods provided herein, are also provided.

As used herein, the phrase "hematopoietic stem cell" refers to a type of stem cell that can give rise to a blood cell. Hematopoietic stem cells can give rise to cells of the myeloid or lymphoid lineages, or a combination thereof. Hematopoietic stem cells are predominantly found in the bone marrow, although they can be isolated from peripheral blood, or a fraction thereof. Various cell surface markers can be used to identify, sort, or purify hematopoietic stem cells. In some cases, hematopoietic stem cells are identified as c-kit+ and lin-. In some cases, human hematopoietic stem cells are identified as CD34+, CD59+, Thy1/CD90+, CD38lo/-, C-kit/CD117+, lin-. In some cases, human hematopoietic stem cells are identified as CD34-, CD59+, Thy1/CD90+, CD38lo/-, C-kit/CD117+, lin-. In some cases, human hematopoietic stem cells are identified as CD133+, CD59+, Thy1/CD90+, CD38lo/-, C-kit/CD117+, lin-. In some cases, mouse hematopoietic stem cells are identified as CD34lo/-, SCA-1+, Thy1+/lo, CD38+, C-kit +, lin-. In some cases, the hematopoietic stem cells are CD150+CD48-CD244-.

As used herein, the phrase "hematopoietic cell" refers to a cell derived from a hematopoietic stem cell. The hematopoietic cell may be obtained or provided by isolation from an organism, system, organ, or tissue (e.g., blood, or a fraction thereof). Alternatively, an hematopoietic stem cell can be isolated and the hematopoietic cell obtained or provided by differentiating the stem cell. Hematopoietic cells include cells with limited potential to differentiate into further cell types. Such hematopoietic cells include, but are not limited to, multipotent progenitor cells, lineage-restricted progenitor cells, common myeloid progenitor cells, granulocyte-macrophage progenitor cells, or megakaryocyte-erythroid progenitor cells. Hematopoietic cells include cells of the lymphoid and myeloid lineages, such as lymphocytes, erythrocytes, granulocytes, monocytes, and thrombocytes. In some embodiments, the hematopoietic cell is an immune cell, such as a T cell, B cell, macrophage, a natural killer (NK) cell or dendritic cell. In some embodiments the cell is an innate immune cell.

As used herein, the phrase "T cell" refers to a lymphoid cell that expresses a T cell receptor molecule. T cells include human alpha beta (αβ) T cells and human gamma delta (γδ) T cells. T cells include, but are not limited to, naive T cells, stimulated T cells, primary T cells (e.g., uncultured), cultured T cells, immortalized T cells, helper T cells, cytotoxic T cells, memory T cells, regulatory T cells, natural killer T cells, combinations thereof, or subpopulations thereof. T cells can be CD4+, CD8+, or CD4+ and CD8+. T cells can also be CD4-, CD8-, or CD4- and CD8-. T cells can be helper cells, for example helper cells of type TH1, TH2, TH3, TH9, TH17, or TFH. T cells can be cytotoxic T cells. Regulatory T cells can be FOXP3+ or FOXP3-. T cells can be alpha/beta T cells or gamma/delta T cells. In some cases, the T cell is a CD4+CD25hiCD127lo regulatory T cell. In some cases, the T cell is a regulatory T cell selected from the group consisting of type 1 regulatory (Tr1), TH3, CD8+CD28-, Treg17, and Qa-1 restricted T cells, or a combination or sub-population thereof. In some cases, the T cell is a FOXP3+ T cell. In some cases, the T cell is a CD4+CD25loCD127hi effector T cell. In some cases, the T cell is a CD4+CD25loCD127hiCD45RAhiCD45RO- naive T cell. A T cell can be a recombinant T cell that has been genetically manipulated.

As used herein, the phrase "primary" in the context of a primary cell is a cell that has not been transformed or immortalized. Such primary cells can be cultured, sub-cultured, or passaged a limited number of times (e.g., cultured 0, 1, 2, 3, 4, 5, 6, 7, 8, 9, 10, 11, 12, 13, 14, 15, 16, 17, 18, 19, or 20 times). In some cases, the primary cells are adapted to in vitro culture conditions. In some cases, the primary cells are isolated from an organism, system, organ, or tissue, optionally sorted, and utilized directly without culturing or sub-culturing. In some cases, the primary cells are stimulated, activated, or differentiated. For example, primary T cells can be activated by contact with (e.g., culturing in the presence of) CD3, CD28 agonists, IL-2, IFN-γ, or a combination thereof.

As used herein the term "extracellular vesicle (EV)" refers to membrane-bound vesicles that are naturally released from eukaryotic cells. As such EVs are cell-derived vesicles, i.e., a lipid bilayer delimited particles, comprising a membrane that encloses an internal space (lumen). Generally EVs range in diameter from 20 nm to 1000 nm. EVs include, but are not limited to, exosomes and microvesicles. EVs are released by cells and found in most biological fluids including urine, plasma, cerebrospinal fluid, saliva etc. as well, as in tissue culture conditioned media

### DETAILED DESCRIPTION

The invention is defined in the appended claims. The following description recites various aspects and embodiments of the present compositions, methods, and compositions for use in methods. No particular embodiment is intended to define the scope of the compositions and methods. Rather, the embodiments merely provide non-limiting examples of various compositions and methods that are at least included within the scope of the disclosed compositions and methods. The description is to be read from the perspective of one of ordinary skill in the art; therefore, information well known to the skilled artisan is not necessarily included.

Provided herein are compositions, systems, methods of manufacture, and *ex vivo* methods for efficient delivery of nucleic acid sequences (e.g., mRNA sequences) and RNPs to eukaryotic cells using EVs. Using the compositions and methods described herein, nucleic acid sequences (e.g., mRNA sequences) and RNPs can be efficiently packaged in EVs and delivered to eukaryotic cells. For example, components, systems, methods of manufacture, and *ex vivo* methods for efficient delivery to cells of RNPs comprising (1) a CRISPR-associated endonuclease and (2) an sgRNA, via EVs, are provided. The EVs described herein have a limited half-life, thus reducing the risk of RNA and DNA off-target mediated mutagenesis. Delivery of RNPs into eukaryotic cells allows for efficient delivery, for example, in cells that are difficult to transfect, such as primary cells while reducing off-target effects.

### Plasmid Systems

Provided herein are plasmid systems that are used to deliver CRISPR component coding sequences, i.e., an sgRNA and a CRISPR-associated endonuclease, into mammalian cells being used to generate the EVs of this disclosure. For example provided herein is a a plasmid system comprising: (a) a first mammalian expression plasmid comprising a eukaryotic promoter operably linked to a nucleic acid sequence, wherein the nucleic acid sequence comprises: (i) a nucleic acid sequence encoding a CRISPR-associated endonuclease; and a guide RNA (gRNA) coding sequence, wherein the gRNA coding sequence comprises at least one Com aptamer coding sequence, or (ii) a nucleic acid sequence encoding a heterologous polypeptide and at least one Com aptamer coding sequence; and (b) a second mammalian expression plasmid comprising a eukaryotic promoter operably linked to a nucleic acid sequence encoding a fusion protein comprising CD63 and at least one Com aptamer binding protein, wherein the Com aptamer binding protein (ABP) binds to the at least one Com aptamer coding sequence of the first mammalian expression plasmid. In some embodiments, the plasmid system further comprises an envelope plasmid comprising a nucleic acid sequence encoding vesicular stomatis virus G (VSV G) protein.

The first mammalian expression plasmid of the systems provided herein comprises CRISPR component coding sequences, e.g., the coding sequence for a CRISPR-associated endonuclease and a gRNA. The gRNA coding sequence comprises at least one Com aptamer coding sequence. In some instances, the at least one Com aptamer coding sequence may be positioned at the 5' end or the 3' end of the gRNA. In some instances, the at least one Com aptamer coding sequence may be inserted at an internal position within the gRNA such as, for example, at one or more of the loops formed in the folded gRNA. For example, where the gRNA is for the Cas9 protein, the at least one Com aptamer coding sequence may be positioned at the tetra loop, the stem loop 2 (ST2), or the 3' end of the gRNA. In some instances, a spacer of 1-30 nucleotides may be positioned between the gRNA the at least one aptamer coding sequence, or flanking the at least one Com aptamer coding sequence. In some embodiments, the sgRNA coding sequence comprises at least one com aptamer sequence inserted into the tetraloop or the ST2 loop of the gRNA coding sequence.

In the systems provided herein, the first mammalian expression plasmid comprises at least one Con aptamer coding sequence that encodes a Com aptamer sequence that is bound specifically by a Com aptamer-binding protein (ABP) encoded by the second mammalian expression plasmid of the system. In the context of this disclosure, an aptamer sequence is an RNA sequence that forms a tertiary loop structure that is specifically bound by an ABP. ABPs are RNA-binding proteins or RNA-binding protein domains. Suitable aptamer coding sequences include polynucleotide sequences that encode known bacteriophage aptamer sequences. Further aptamer coding sequences include those encoding the aptamer sequences provided above in Table 1. In some instances, the aptamers are bound by a dimer of ABP. These aptamer sequences are RNA sequences known to be bound specifically by bacteriophage proteins. In embodiments of the invention, the at least one Com aptamer coding sequence encodes an aptamer sequence bound specifically by a Com ABP. Further aptamer coding sequence encodes an aptamer sequence bound specifically by an ABP selected from the group consisting of MS2 coat protein, PP7 coat protein, or lambda N RNA-binding domain. In embodiments of the invention, the at least one aptamer coding sequence is a com aptamer and the ABP is a Com protein.

**Table 1. Aptamer-Binding Proteins and Corresponding Aptamer Sequences**

| | Aptamer-Binding Proteins | | | |
|---|---|---|---|---|
| | MS2 coat protein | PP7 coat protein | lambda N peptide (amino acids 1-22) | Com (Control of mom) protein |
| Nucleic Acid Sequence | SEQ ID NO: 1 | SEQ ID NO :3 | SEQ ID NO:5 | SEQ ID NO:7 |
| Amino Acid Sequence | SEQ ID NO: 2 | SEQ ID NO: 4 | SEQ ID NO:6 | SEQ ID NO:8 |
| Aptamer (RNA) | SEQ ID NO:9 | SEQ ID NO:11 | SEQ ID NO:13 (Box-B aptamer) | SEQ ID NO:15 |
| Aptamer (DNA) | SEQ ID NO:10 | SEQ ID NO:12 | SEQ ID NO:14 | SEQ ID NO:16 |

In some instances, the first mammalian expression vector comprises a sgRNA that comprises one aptamer coding sequence downstream thereof. In other instances, the gRNA may comprise 1, 2, 3, 4, 5, 6, 7, 8, 9, 10, 11, or 12 aptamer coding sequences. For example, in some instances, the gRNA may comprise two aptamer coding sequences in tandem. In some embodiments the two or more tandem aptamer coding sequences are the same. In some embodiments, the two or more tandem aptamer coding sequences are different.

As used throughout, a sgRNA is a single guide RNA sequence that interacts with a CRISPR-associated endonuclease (a CRISPR site-directed nuclease) and specifically binds to or hybridizes to a target nucleic acid within the genome of a cell (genomic target sequence), such that the sgRNA and the CRISPR-associated endonuclease co-localize to the target nucleic acid in the genome of the cell. Each sgRNA includes a DNA targeting sequence or protospacer sequence of about 10 to 50 nucleotides in length that specifically binds to or hybridizes to a target DNA sequence in the genome. For example, the DNA targeting sequence may be about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length. For example, the DNA targeting sequence may be about 15-30 nucleotides, about 15-25 nucleotides, about 10-25 nucleotides, or about 18-23 nucleotides. In one example, the DNA targeting sequence is about 20 nucleotides. In some embodiments, the sgRNA comprises a crRNA sequence and a transactivating crRNA (tracrRNA) sequence. In some embodiments, the sgRNA does not comprise a tracrRNA sequence.

Generally, the DNA targeting sequence is designed to complement (e.g., perfectly complement) or substantially complement (e.g., having 1-4 mismatches) to the target DNA sequence. In some cases, the DNA targeting sequence can incorporate wobble or degenerate bases to bind multiple genetic elements. In some cases, the 19 nucleotides at the 3' or 5' end of the binding region are perfectly complementary to the target genetic element or elements. In some cases, the binding region can be altered to increase stability. For example, non-natural nucleotides, can be incorporated to increase RNA resistance to degradation. In some cases, the binding region can be altered or designed to avoid or reduce secondary structure formation in the binding region. In some cases, the binding region can be designed to optimize G-C content. In some cases, G-C content is preferably between about 40% and about 60% (e.g., 40%, 45%, 50%, 55%, 60%). In some cases, the binding region, can be selected to begin with a sequence that facilitates efficient transcription of the sgRNA. For example, the binding region can begin at the 5' end with a G nucleotide. In some cases, the binding region can contain modified nucleotides such as, without limitation, methylated or phosphorylated nucleotides.

As used herein, the term "complementary" or "complementarity" refers to base pairing between nucleotides or nucleic acids, for example, and not to be limiting, base pairing between a sgRNA and a target sequence. Complementary nucleotides are, generally, A and T (or A and U), and G and C. The guide RNAs described herein can comprise sequences, for example, DNA targeting sequence that are perfectly complementary or substantially complementary (e.g., having 1-4 mismatches) to a genomic sequence.

The sgRNA includes a sgRNA constant region that interacts with or binds to the CRISPR-associated endonuclease. In the constructs provided herein, the constant region of an sgRNA can be from about 75 to 250 nucleotides in length. In some examples, the constant region is a modified constant region comprising one, two, three, four, five, six, seven, eight, nine, ten or more nucleotide substitutions in the stem, the stem loop, a hairpin, a region in between hairpins, and/or the nexus of a constant region. In some instances, a modified constant region that has at least 80%, 85%, 90%, or 95% activity, as compared to the activity of the natural or wild-type sgRNA constant region from which the modified constant region is derived, may be used in the constructs described herein. In particular, modifications should not be made at nucleotides that interact directly with a CRISPR-associated endonuclease or at nucleotides that are important for the secondary structure of the constant region.

The CRISPR-associated endonuclease encoded on the first mammalian expression plasmid of any of the systems described herein are RNA-guided site-directed nucleases. For example, and not to be limiting, the CRISPR-associated endonuclease can be a Cas9 polypeptide (Type II) or a Cpfl polypeptide (Type V). See, for example, Abudayyeh et al., Science 2016 August 5; 353(6299):aaf5573; Fonfara et al. Nature 532: 517-521 (2016), and Zetsche et al., Cell 163(3): p. 759-771, 22 October 2015. As used throughout, the term "Cas9 polypeptide" means a Cas9 protein, or a fragment or derivative thereof, identified in any bacterial species that encodes a Type II CRISPR/Cas system. See, for example, Makarova et al. Nature Reviews, Microbiology, 9: 467-477 (2011), including supplemental information. CRISPR-associated endonucleases, such as Cas9 and Cas9 homologs, are found in a wide variety of eubacteria, including, but not limited to bacteria of the following taxonomic groups: Actinobacteria, Aquificae, Bacteroidetes-Chlorobi, Chlamydiae-Verrucomicrobia, Chlroflexi, Cyanobacteria, Firmicutes, Proteobacteria, Spirochaetes, and Thermotogae. An exemplary Cas9 protein is the Streptococcus pyogenes Cas9 protein (SpCas9). Another exempary Cas9 protein is the Staphylococcus aureus Cas9 protein (SaCas9). Additional Cas9 proteins and homologs thereof are described in, e.g., Chylinksi, et al., RNA Biol. 2013 May 1; 10(5): 726-737 ; Nat. Rev. Microbiol. 2011 June; 9(6): 467-477; Hou, et al., Proc Natl Acad Sci USA. 2013 Sep 24;110(39):15644-9; Sampson et al., Nature. 2013 May 9;497(7448):254-7; and Jinek, et al., Science. 2012 Aug 17;337(6096):816-21. The Cas9 nuclease domains can be optimized for efficient activity or enhanced stability in the host cell. Other CRISPR-associated endonucleases include Cpfl (See, e.g., Zetsche et al., Cell, Volume 163, Issue 3, p759-771, 22 October 2015) and homologs thereof.

Full-length Cas9 is an endonuclease comprising a recognition domain and two nuclease domains (HNH and RuvC, respectively) that creates double-stranded breaks in DNA sequences. In the amino acid sequence of Cas9, HNH is linearly continuous, whereas RuvC is separated into three regions, one left of the recognition domain, and the other two right of the recognition domain flanking the HNH domain. Cas9 is targeted to a genomic site in a cell by interacting with a guide RNA that hybridizes to a 20-nucleotide DNA sequence that immediately precedes an NGG motif recognized by Cas9. This results in a double-strand break in the genomic DNA of the cell. In some examples, a Cas9 nuclease that requires an NGG protospacer adjacent motif (PAM) immediately 3' of the region targeted by the guide RNA can be utilized. As another example, Cas9 proteins with orthogonal PAM motif requirements can be utilized to target sequences that do not have an adjacent NGG PAM sequence. Exemplary Cas9 proteins with orthogonal PAM sequence specificities include, but are not limited to those described in Esvelt et al., Nature Methods 10: 1116-1121 (2013). Various Cas9 nucleases can be utilized in the methods described herein. For example, a Cas9 nuclease that requires an NGG protospacer adjacent motif (PAM) immediately 3' of the region targeted by the guide RNA, such as SpCas9, can be utilized. Such Cas9 nucleases can be targeted to any region of a genome that contains an NGG sequence. In another example, a Cas9 nuclease that requires an NNGRRT (SEQ ID NO:106) or NNGRR(N) (SEQ ID NO: 107) PAM immediately 3' of the region targeted by the guide RNA, such as SaCas9, can be utilized. As another example, Cas9 proteins with orthogonal PAM motif requirements can be utilized to target sequences that do not have an adjacent NGG PAM sequence. Exemplary Cas9 proteins with orthogonal PAM sequence specificities include, but are not limited to those described in Nature Methods 10, 1116-1121 (2013), and those described in Zetsche et al., Cell, Volume 163, Issue 3, p759-771, 22 October 2015.

Any of the CRISPR-associated endonucleases described herein can be catalytically impaired. In some cases, the catalytically impaired CRISPR-associated endonuclease is a Cas9 nickase, for example, Cas9 D10A . In some instances, Cas9 10A is encoded by SEQ ID NO: 21. In some instances, the Cas9 10A comprises SEQ ID NO: 22. is Normally, when a Cas9 nickase is bound to target nucleic acid as part of a complex with a guide RNA, a single strand break or nick is introduced into the target nucleic acid. A pair of Cas9 nickases, each bound to a structurally different guide RNA, can be targeted to two proximal sites of a target genomic region. Exemplary Cas9 nickases include Cas9 nucleases having a D10A or H840A mutation.

In some embodiments, the nucleic acid sequence of the first mammalian expression plasmid encodes a nucleic acid sequence encoding an adenosine base pair editor (ABE), wherein the ABE is a fusion protein comprising an adenosine deaminase and the catalytically impaired CRISPR-associated endonuclease. In some embodiments, the adenine base editor is ABE 7.10 or ABE8.

The plasmid systems described herein comprise a second mammalian expression plasmid comprising a eukaryotic promoter operably linked to a nucleic acid sequence encoding a fusion protein comprising CD63 and at least one Com aptamer binding protein, wherein the Com aptamer binding protein (ABP) binds to the at least one Com aptamer coding sequence of the first mammalian expression plasmid.

In some embodiments, the fusion protein comprises an ABP fused to the N-terminus of CD63 and/or an ABP fused to the C-terminus of CD63, wherein at least one is a Com ABP. In some embodiments, the fusion protein comprises a first Com ABP fused to the N-terminus of CD63 and a second Com ABP fused to the C-terminus of CD63, wherein the first and second ABP are the same. In some embodiments, the fusion protein comprises a first ABP fused to the N-terminus of CD63 and a second ABP fused to the C-terminus of CD63, wherein the first and second ABP are different, wherein at least one is a Com ABP. In some embodiments, the first and second ABP is Com which binds to the com aptamer in the first mammalian expression plasmid.

As used herein, CD63, CD63 antigen, or a fragment thereof, is a member of the transmembrane 4 superfamily of proteins, also known as the tetraspanin family. CD63 is a cell-surface protein, characterized by the presence of four hydrophobic domains, that is found on the cell surface of extracellular vesicles. An exemplary amino acid sequence for CD63 is set forth as SEQ ID NO: 43. SEQ ID NO 43 is encoded by SEQ ID NO: 42. It is understood that any of the isoforms of CD63 or a fragment thereof can be used in the compositions and methods described herein. Exemplary CD63 isoform protein sequences are set forth under GenBank Accession Nos. NP_001244318.1, NP_001771.1, NP_001254627.1, NP_001244319.1, NP_001244320.1, NP_001244329.1, NP_001244330.1, NP_001244321.1, and XP_024305051.1.

The mammalian expression plasmids comprise a eukaryotic promoter operably linked to the nucleic acid sequence encoding the CRISPR-endonuclease, the sgRNA coding sequence or the CD63-Com ABP fusion protein. The systems described herein also can include an envelope plasmid having an envelope coding sequence that encodes a viral envelope glycoprotein. For example, the Env nucleotide sequence may encode VSV-G. The envelope coding sequence is operably linked to a eukaryotic promoter. In some instances, the eukaryotic promoter is a RNA polymerase II promoter.

As used herein, "vesicular stomatitis virus G" or "VSV-G" is a viral envelope protein that can be used to facilitate trafficking or escape of any of the EVs described herein from the endosome system in recipient cells. An exemplary VSV-G amino acid sequence is set forth as SEQ ID NO: 45. SEQ ID NO: 45 is encoded by SEQ ID NO: 44. Another exemplary VSV-G protein sequence is set forth under GenBank Accession Nos. CAC47944.1.

In some instances, a RNA polymerase II promoter is operably linked to the CRISPR-associated endonuclease coding sequence and a RNA polymerase III promoter is operably linked to the gRNA coding sequence.

The RNA polymerase II promoter sequence is selected from a mammalian species. The RNA polymerase III promoter sequences is selected from a mammalian species. For example, these promoter sequences can be selected from a human, cow, sheep, buffalo, pig, or mouse, to name a few. In some examples, the RNA polymerase II promoter sequence is a CMV, FE1α, or SV40 sequence. In some examples, the RNA polymerase III promoter sequence is a U6 or an H1 sequence. In some examples, the RNA polymerase II sequence is a modified RNA polymerase II sequence. For example, the RNA polymerase II sequences having at least 80%, 85%, 90%, 95%, or 99% identity to a wild-type RNA polymerase II promoter sequence from any mammalian species can be used in the constructs provided herein. In some examples, the RNA polymerase III sequence is a modified RNA polymerase III sequence. For example, the RNA polymerase III sequences having at least 80%, 85%, 90%, 95%, or 99% identity to a wild-type RNA polymerase III promoter sequence from any mammalian species can be used in the constructs provided herein. Those of skill in the art readily understand how to determine the identity of two polypeptides or nucleic acids. For example, the identity can be calculated after aligning the two sequences so that the identity is at its highest level, as described above. In some instances, the eukaryotic promoter is an inducible or regulatable promoter.

Coding sequences transcribed from a RNA pol II promoter include a poly(A) signal and a transcription terminator sequence downstream of the coding sequence. Commonly used mammalian terminators (SV40, hGH, BGH, and rbGlob) include the sequence motif AAUAAA (SEQ ID NO: 108) which promotes both polyadenylation and termination. Coding sequences transcribed from a RNA pol III promoter include a simple run of T residues downstream of the coding sequence as a terminator sequence. The role of the terminator, a sequence-based element, is to define the end of a transcriptional unit (such as a gene) and initiate the process of releasing the newly synthesized RNA from the transcription machinery. Terminators are found downstream of the gene to be transcribed, and typically occur directly after any 3' regulatory elements, such as the polyadenylation or poly(A) signal.

In some instances, the mammalian expression plasmid may also include at least one polynucleotide sequence encoding a RNA-stabilizing sequence positioned downstream of the CRISPR component coding sequence or the Com aptamer coding sequence if positioned downstream of the CRISPR component coding sequence. The polynucleotide sequence encoding the RNA-stabilizing sequence is transcribed downstream of the CRISPR/Cas system component coding sequence and stabilizes the longevity of the transcribed RNA sequence. In one example, the polynucleotide sequence encoding the RNA-stabilizing sequence is positioned downstream of the catalytically impaired CRISPR-associated endonuclease coding sequence. In another example, the polynucleotide sequence encoding the RNA-stabilizing sequence is positioned downstream of the gRNA coding sequence. An exemplary RNA-stabilizing sequence is the sequence of the 3' UTR of human beta globin gene as set forth in SEQ ID NO:17 (DNA) and SEQ ID NO:18 (RNA). In some embodiments, the RNA-stabilizing sequence comprises two or more copies of SEQ ID NO: 17. Other RNA-stabilizing sequences are described in Hayashi, T. et al., Developmental Dynamics 239(7):2034-2040

(2010) and Newbury, S. et al., Cell 48(2):297-310 (1987). In some instances, a spacer of 1-30 nucleotides may be positioned between the CRISPR component coding sequence and the at least one polynucleotide sequence encoding RNA-stabilizing sequence.

In some instances, any of the mammalian expression plasmids described herein may comprise one or more expression cassettes. In some instances the first mammalian expression plasmid comprises a first expression cassette that encodes the CRISPR-associated endonuclease and a second expression cassette that encodes the gRNA comprising at least one Com aptamer. In some instances, the mammalian expression plasmid may also comprise a reporter gene.

Also disclosed herein are kits the include the components of the systems described in this disclosure. The kits may include one or more of the plasmids described herein.

### Extracellular Vesicles

In another aspect, provided are EVs, for example, EVs made by any of the systems and methods described herein. In some embodiments, the EVs are exosomes and/or microvesicles. Extracellular vesicles made by any of the methods described herein are also provided. A plurality of EVs is also provided. The EVs contain (a) a ribonucleotide protein (RNP) complex comprising: (i) a CRISPR-associated endonuclease and a gRNA comprising at least one Com aptamer coding sequence, or (ii) a mRNA encoding a heterologous polypeptide and at least one Com aptamer coding sequence; and (b) a fusion protein comprising CD63 and at least one Com aptamer binding protein (ABP), wherein the ABP binds to the at least one Com aptamer coding sequence. In some embodiments, the extracellular vesicle further comprises a VSV-G protein.

Any of the first mammalian expression plasmids described herein wherein at least one Com aptamer is attached or inserted into the gRNA sequence, can be used to generate EVs containing RNPs. These EVs are useful to transduce eukaryotic cells of interest.

In some embodiments, one or more first mammalian expression plasmids, wherein each of the expression plasmids targets a different site in the genome of cell are used to generate EVs that contain one or more RNPs that target one or more sites in the genome of the cell. For example, and not to be limiting, a first mammalian expression plasmid comprising a sgRNA that targets site A in the genome of the cell and a first mammalian expression plasmid comprising a sgRNA that targets site B in the genome of a cell can be transduced into cells with the second mammalian expression plasmid encoding a CD63-ABP fusion protein, to generate EVs comprising RNPs that target site A and RNPs that target site B. EVs that target three or more, four or more, five or more, or six or more sites in the genome can be generated using similar methods.

The EVs comprise a fusion protein comprising one or more Com ABPs. The EVs contain CD63 or a fragment thereof, fused with at least one Com ABP. In some embodiments, the fusion protein comprises an ABP fused to the N-terminus of CD63 and/or an ABP fused to the C-terminus of CD63, wherein at least one ABP is Com. In some embodiments, the fusion protein comprises a first Com ABP fused to the N-terminus of CD63 and a second Com ABP fused to the C-terminus of CD63, wherein the first and second ABP are the same. In some embodiments, the fusion protein comprises a first ABP fused to the N-terminus of CD63 and a second ABP fused to the C-terminus of CD63, wherein the first and second ABP are different, wherein at least one of the ABPs is Com. In some embodiments, the first and second ABP is Com, which binds to the com aptamer that is attached or inserted in the gRNA. In some embodiments, the fusion protein comprises two or more ABPs in tandem, fused to the N-terminus of CD63 and/or the C-terminus of CD63, wherein at least one of the ABPs is Com.

An ABP is a polypeptide sequence that binds to an RNA aptamer sequence. In embodiments of the invention at least one ABP is Com. As set forth above, several ABPs are suitable for use in this disclosure. In particular, suitable ABPs include bacteriophage RNA-binding proteins that bind specifically to known RNA aptamer sequences, which are RNA sequences that form stem-loop structures. Exemplary non-viral aptamer binding protein include MS2 coat protein, PP7 coat protein, lambda N peptide, and Com protein. The lambda N peptide may be amino acids 1-22 of the lambda N protein, which are the RNA-binding domain of the protein. Information about these ABP and the aptamer sequences to which they bind is provided above in Table 1.

As set forth above, gRNA generally comprises a DNA targeting sequence and a constant region that interacts with the CRISPR-associated endonuclease. In some instances, the gRNA may comprise a transactivating crRNA (tracrRNA) sequence. For example, the gRNA may comprise a tracrRNA where it is to be used in conjunction with a Cas9 protein or derivative. In other instances, the gRNA does not comprise a tracrRNA sequence. For example, the gRNA may not comprise a tracrRNA sequence where it is to be used in conjunction with a Cpf1 protein or derivative.

In some instances, the gRNA comprises at least one Com aptamer sequence. In some instances, the at least one Com aptamer sequence may be positioned at the 5' end or the 3' end of the gRNA. In some instances, the at least one Com aptamer sequence may be inserted at an internal position within the gRNA such as, for example, at one or more of the loops formed in the folded gRNA. For example, where the gRNA is for a Cas9 protein, the at least one aptamer sequence may be positioned at the tetra loop, the stem loop 2 (ST2), or the 3' end of the gRNA. In some instances, a spacer of 1-30 ribonucleotides may be positioned between the gRNA and the at least one Com aptamer sequence, or flanking the at least one Com aptamer sequence.

### Methods of Making EVs

Described herein are methods of using any of the plasmids and systems provided in this disclosure to produced EVs. For example, provided herein is method of producing an extracellular vesicle, the method comprising: (a) transfecting a plurality of eukaryotic cells with the first mammalian expression plasmid (i.e., a plasmid encoding a CRISPR-associated endonuclease and a sgRNA) and the second mammalian expression plasmid (i.e., a plasmid encoding a CD63-Com ABP fusion protein) of any of the plasmid systems described herein; and (b) culturing the transfected eukaryotic cells for sufficient time for extracellular vesicles to be produced. In some embodiments, the method further comprises transfecting the plurality of eukaryotic cells with the envelope plasmid (i.e., a plasmid encoding VSV-G) of any of the plasmid systems described herein.

In some embodiments, the extracellular vesicle comprises: (a) a RNP comprising: (i) a CRISPR-associated endonuclease; and (ii) a gRNA comprising at least one Com aptamer coding sequence; and (b) a fusion protein comprising CD63 and at least one Com aptamer binding protein, wherein the Com aptamer binding protein (ABP) binds to the at least one Com aptamer coding sequence. In some embodiments, the extracellular vesicle comprises: (a) a RNP comprising: (i) a CRISPR-associated endonuclease and a gRNA comprising at least one Com aptamer coding sequence, or (ii) an mRNA encoding the heterologous polypeptide and the at least one Com aptamer sequence; (b) a fusion protein comprising CD63 and at least one Com aptamer binding protein, wherein the Com aptamer binding protein (ABP) binds to the Comn aptamer coding sequence; and(c) a VSV-G protein.

In some embodiments, the plurality of eukaryotic cells are mammalian cells. In some embodiments, the method further comprises isolating the extracellular vesicles from the cultured transfected eukaryotic cells. Methods for isolating EVs are known in the art. See, for example, Konoshenko et al. "Isolation of Extracellular Vesicles: General Methodologies and Latest Trends," Hindawi BioMed Research International Vol. 2018, Article ID 8545347; and Furi et al. "Extracellular vesicle isolation: present and future," Ann Transl. Med. 5(12): 263 (2017). In some embodiments, the eukaryotic cells are selected from the group consisting of HEK293T cells, C2C12 cells, primary myoblasts, neural stem cells, pluripotent stem cells, and mesenchymal stem cells.

### Gene Editing Methods

Described herein are ex vivo methods of using the plasmids and systems provided in this disclosure, or the plasmids and systems provided in this disclosure for use methods, in CRISPR/Cas systems for editing DNA targets, for example, a gene, in the genome of a eukaryotic cell.

In the methods provided herein, eukaryotic cells comprising a target genomic sequence of interest to be modified are transduced with EVs that contain a fusion protein comprising CD63, or a fragment thereof, fused to at least one Com aptamer-binding protein (ABP) and an RNP comprising (1) a gRNA and (2) a CRISPR-associated endonuclease. In some embodiments, the EVs contain (1) a fusion protein comprising CD63, or a fragment thereof, fused to at least one Com aptamer-binding protein (ABP); (2) an RNP comprising (a) a gRNA and (b) a CRISPR-associated endonuclease; and (3) an envelope protein or a fragment thereof (for example VSV-G or a fragment thereof). In some embodiments, VSV-G facilitates entry of EVs into transduced cells. In some embodiments, the CRISPR-associated endonuclease is catalytically impaired. In some embodiments, the catalytically impaired CRISPR-associated endonuclease is fused to an adenosine deaminase as part of an ABE. Examples of CRISPR-associated endonucleases, including catalytically impaired CRISPR-associate endonucleases are described above.

In some embodiments, the gene editing methods provided herein result in increased editing efficiency by the CRISPR-associated endonuclease or ABE. For example, when using any of the EVs comprising an RNP described herein, in the methods provided herein, editing efficiency can be increased by at least 10%, 20%, 30%, 40%, 50%, 60%, 70%, 75%, 80%, 85%, 90%, 95%, 96%, 97%, 98%, 99%, or greater, as compared to editing efficiency when RNPs are delivered using non-EV delivery. In some instances, the increase in gene editing efficiency is a two-fold, a four-fold, a ten-fold, a twenty-fold, a fifty-fold increase or greater. In some embodiments, the increase is relative to lentiviral delivery of RNPs.

In some embodiments, using the methods provided herein, the transduction efficiency of delivering RNPs into cells, using the EVs described herein, is increased as compared to non-EV delivery. In some instances transduction efficiency is increased by at least 10%, 20%, 30%, 40%, 50%, 60%, 70%, 75%, 80%, 85%, 90%, 95%, 96%, 97%, 98%, 99% or greater, as compared to transduction efficiency when RNPs are delivered to cells using non-EV delivery. In some instances, the increase in transduction efficiency is a two-fold, a four-fold, a ten-fold, a twenty-fold, a fifty-fold increase or greater. In some embodiments, the increase is relative to lentiviral delivery of RNPs.

In some embodiments, the gene editing methods provided herein result in reduced guide independent RNA off-target gene editing events, for example, those associated CRISPR-associated endonuclesase or ABEs. For example, when using an ABE in the methods provided herein, guide-independent RNA off-target activity can be reduced by at least 5%, 10%, 20%, 30%, 40%, 50%, 60%, 70%, 75%, 80%, 85%, 90%, 95%, 96%, 97%, 98%, 99%, or greater, as compared to RNA off-target activity when RNPs are delivered using non-EV delivery. In some instances, guide independent DNA off-target gene editing events are also reduced. For example, in the methods provided herein, guide-dependent DNA off-target activity can be reduced by at least 5%, 10%, 20%, 30%, 40%, 50%, 60%, 70%, 90%, 95%, 99% or greater when RNPs are delivered using non-EV delivery.

In some instances, the transduced eukaryotic cells are mammalian cells. In some instances, the eukaryotic cells may be *in vitro* cultured cells. In some instances, the eukaryotic cells may *ex vivo* cells obtained from a subject. In other instances, the plasmids and systems provided in this disclosure are for use in methods described herein and the eukaryotic cells are present in a subject. As used throughout, by subject is meant an individual. For example, the subject is a mammal, such as a primate, and, more specifically, a human. Non-human primates are subjects as well. The term subject includes domesticated animals, such as cats, dogs, etc., livestock (for example, cattle, horses, pigs, sheep, goats, etc.) and laboratory animals (for example, ferret, chinchilla, mouse, rabbit, rat, gerbil, guinea pig, etc.). Thus, veterinary uses and medical uses and formulations are contemplated herein. The term does not denote a particular age or sex. Thus, adult and newborn subjects, whether male or female, are intended to be covered. As used herein, patient or subject may be used interchangeably and can refer to a subject afflicted with a disease or disorder. The EVs provided by this disclosure may be injected into a subject according to known, routine methods. In some instances, the viral particles of the system are injected intravenously (IV), intraperitoneally (IP), intramuscularly, or into a specific organ. The EVs may also be implanted, for example, into a tumor.

In some instances, the provided methods are for modifying a target loci of interest, the method comprising transducing a plurality of eukaryotic cells with a plurality of EVs, wherein the plurality of EVs comprise a fusion protein comprising CD63 or a fragment thereof, fused to at least one Com ABP; and a ribonucleotide protein (RNP) complex comprising (1) a gRNA and (2) a CRISPR-associated endonuclease, wherein the RNP binds to the genomic target sequence in genomic DNA of the cell and the CRISPR-associated endonuclease alters the genomic DNA of the cell. As described above, EVs comprising two or more RNPs wherein each RNP targets a different locus can be used to modify two or more loci of interest in a eukaryotic cells. As described above, the RNPs are packaged into the EVs via the interaction of an aptamer sequence attached to or inserted into a gRNA sequence that forms a complex with the catalytically impaired CRISPR-associated endonuclease.

The methods described can be used with any CRISPR-associated endonuclease that requires a constant region of an sgRNA for function. These include, but are not limited to RNA-guided site-directed nucleases. Examples include nucleases present in any bacterial species that encodes a Type II or V CRISPR/Cas system. Suitable CRISPR-associated endonucleases are described throughout this disclosure. For example, and not to be limiting, the site-directed nuclease can be a catalytically impaired Cas9 polypeptide, a catalytically impaired Cpfl polypeptide, a catalytically impaired Cas9 nickase or derivatives thereof.

Generally, the sgRNA is targeted to specific regions at or near a gene. In some instances, the sgRNA can be targeted to a region where single base changes are necessary, for example, to correct a single base mutation in the human beta-globin gene that causes sickle cell anemia. The sgRNA allows the RNPs described herein to a specific site in the genomic sequence of a cell. Once the RNP binds to the specific site in the genomic sequence, the CRISPR-associated endonuclease cleaves one or more strands of the DNA at the specific site. When an ABE is used, the ABE catalyzes adenosine (A) to inosine formation in one strand, while the catalytically impaired endonuclease, for example, Cas9 D10A nicks the opposite strand, i.e., the non-edited strand. Since inosine is read as guanosine by polymerase enzymes, DNA repair and replication mechanism replace the original A-T base pair with a G-C base pair at the target site. See, Gaudelli et al. (2017).

In some instances, the modifications to the system components as described in this disclosure do not impair how the system components function following transduction into eukaryotic cells. Rather, the components may function similarly or better than unmodified components upon transduction into eukaryotic cells. For example, the CD63-Com ABP fusion proteins in the EVs may not interfere with the EV transduction of eukaryotic cells. Similarly, if the RNPs packaged in the EVs comprise at least one Com aptamer sequence, the at least one Com aptamer sequence may not interfere with the EV transduction of eukaryotic cells. In some instances, the EV containing the CD63-Com ABP fusion protein may result in greater gene editing upon transduction into eukaryotic cells relative to EVs that do not comprise CD63-Com ABP fusion protein.

The eukaryotic cells can be *in vitro* or *ex vivo.* In some embodiments, the cell is a primary cell (isolated from a subject). As used herein, a primary cell is a cell that has not been transformed or immortalized. Such primary cells can be cultured, sub-cultured, or passaged a limited number of times (e.g., cultured 0, 1, 2, 3, 4, 5, 6, 7, 8, 9, 10, 11, 12, 13, 14, 15, 16, 17, 18, 19, or 20 times). In some cases, the primary cells are adapted to in vitro culture conditions. In some cases, the primary cells are isolated from an organism, system, organ, or tissue, optionally sorted, and utilized directly without culturing or sub-culturing. In some cases, the primary cells are stimulated, activated, or differentiated. In some embodiments, the cells are cultured under conditions effective for expanding the population of modified cells. In some embodiments, cells modified by any of the methods provided herein are purified. In some cases, cells are removed from a subject, modified using any of the methods described herein and re-administered to the patient.

In some instances, once the cells have been transduced with the EVs described above, the cells are cultured for a sufficient amount of time to allow for gene editing to occur, such that a pool of cells expressing a detectable phenotype can be selected from the plurality of transduced cells. The phenotype can be, for example, cell growth, survival, or proliferation. In some examples, the phenotype is cell growth, survival, or proliferation in the presence of an agent, such as a cytotoxic agent, an oncogene, a tumor suppressor, a transcription factor, a kinase (e.g., a receptor tyrosine kinase), a gene (e.g., an exogenous gene) under the control of a promoter (e.g., a heterologous promoter), a checkpoint gene or cell cycle regulator, a growth factor, a hormone, a DNA damaging agent, a drug, or a chemotherapeutic. The phenotype can also be protein expression, RNA expression, protein activity, or cell motility, migration, or invasiveness. In some examples, the selecting the cells on the basis of the phenotype comprises fluorescence activated cell sorting, affinity purification of cells, or selection based on cell motility.

In some examples, the selecting the cells can also comprise analysis of the genomic DNA of the cells such as by amplification, sequencing, SNP analysis, etc. Sequencing methods include, but are not limited to, shotgun sequencing, bridge PCR, Sanger sequencing (including microfluidic Sanger sequencing), pyrosequencing, massively parallel signature sequencing, nanopore DNA sequencing, single molecule real-time sequencing (SMRT) (Pacific Biosciences, Menlo Park, CA), ion semiconductor sequencing, ligation sequencing, sequencing by synthesis (Illumina, San Diego, Ca), Polony sequencing, 454 sequencing, solid phase sequencing, DNA nanoball sequencing, heliscope single molecule sequencing, mass spectroscopy sequencing, pyrosequencing, Supported Oligo Ligation Detection (SOLiD) sequencing, DNA microarray sequencing, RNAP sequencing, tunneling currents DNA sequencing, and any other DNA sequencing method identified in the future. One or more of the sequencing methods described herein can be used in high throughput sequencing methods. As used herein, the term "high throughput sequencing" refers to all methods related to sequencing nucleic acids where more than one nucleic acid sequence is sequenced at a given time.

### Compositions for Use in Methods of Treatment

Any of the compositions described herein can be used in methods to treat a disease (e.g., cancer, a blood disorder (for example, sickle cell anemia or beta thalassemia), an infectious disease, an autoimmune disease, transplantation rejection, graft vs. host disease or other inflammatory disorder) in a subject.

In some methods, the cancer to be treated is selected from a cancer of B-cell origin, breast cancer, gastric cancer, neuroblastoma, osteosarcoma, lung cancer, colon cancer, chronic myeloid cancer, leukemia (e.g., acute myeloid leukemia, chronic lymphocytic leukemia (CLL) or acute lymphocytic leukemia (ALL)), prostate cancer, colon cancer, renal cell carcinoma, liver cancer, kidney cancer, ovarian cancer, stomach cancer, testicular cancer, rhabdomyosarcoma, and Hodgkin's lymphoma. In some embodiments, the cancer of B-cell origin is selected from the group consisting of B-lineage acute lymphoblastic leukemia, B-cell chronic lymphocytic leukemia, and B-cell non-Hodgkin's lymphoma.

In some methods, the cells of the subject are modified *in vivo,* for example, by delivering any of the EVs described herein to the subject. See, for example, Murphy et al. Experimental and Molecular Medicine 51: 1-12 (2019)). In some embodiments, the EVs can be targeted to a cell or tissue by modification of the EVs to include a binding moiety that binds to a target, for example, a tumor antigen on a tumor cell. In some embodiments, a desired ligand can be linked to the EVs via association with polyethylene glycol (PEG), such that the PEGylated EVs coated with the desired ligand can be specifically targeted to a cell-surface target. See, for example, Rocco et al. Stem Cells International, vol. 2016, Article ID 5029619, 12 pages, 2016. In some embodiments, an effective amount of any of the EVs described herein are administered to the subject. As used herein, an "effective amount" is an amount sufficient to effect beneficial or desired results. An effective amount can be administered in one or more administrations, applications or dosages. In some embodiments, the EVs are delivered in a pharmaceutical composition. Typically, such pharmaceutical compositions are formulated for in use *in vivo,* ex vivo or *in vitro* using pharmaceutically acceptable excipients.

The dosage of EVs administered to a subject will depend the disease or the symptoms to be treated or alleviated, the administration route, as well as various other parameters of relevance known to a skilled person. The amount of EVs to be administered to the subject can be determined by quantitating an EV protein, for example, CD63, with a bicinchoninic acid (BCA) method. The amount of protein to be delivered to the subject can be determined by Western blot detection of the CRISPR-associated endonuclease, for example, Cas9, in EVs, respectively. It is understood that populations of EVs are also provided herein. The EV concentration in any of the compositions described herein may be expressed in many different ways, for instance amount of EV protein per unit (often volume) or per dose, number of EVs or particles per unit (often volume, per subject, per kg of body weight, etc.). For example, and not to be limiting, a composition comprising from about 10⁶ to about 10²⁵ EVs can be administered to a subject in one or more doses. For example, a composition comprising 10⁶ 10⁷, 10⁸, 10⁹, 10¹⁰, 10¹¹, 10¹², 10¹³ 10¹⁴, 10¹⁵, 10¹⁶, 10¹⁷, 10¹⁸, 10¹⁹, 10²⁰, 10²¹, 10²², 10²³, 10²⁴, 10²⁵ or any other amount of EVs, in between these amounts, can be administered to the subject in one or more administrations.

In some methods, the method of treating a disease in a subject comprises: a) providing cells obtained from the subject; b) modifying the cells using any of the methods provided herein; and c) administering the modified cells to the subject. In some methods, the modified cells are expanded and/or differentiated prior to administering the modified cells to the subject. In some embodiments, modification occurs by contacting eukaryotic cells with any of the EVs described herein, wherein the RNP (i.e., a complex comprising a CRISPR-associated endonuclease and a gRNA) delivered by the EV into the cell binds to a site in the genome targeted by the sgRNA, and modifies the genome the cell. In some embodiments, the cells are modified to, for example, correct a mutation, insert a functional copy of a gene, insert a nucleic acid sequence encoding a tumor antigen, edit a base, or otherwise alter the genomic sequence of the cells to treat the disease.

Optionally, the disease is selected from the group consisting of cancer, a blood disorder (for example, sickle cell anemia or beta thalassemia), an infectious disease, an autoimmune disease, transplantation rejection, graft vs. host disease or other inflammatory disorder in a subject. In some methods for treating cancer, the cells obtained form the subject are modified to express a tumor specific antigen. As used throughout, the phrase "tumor-specific antigen" means an antigen that is unique to cancer cells or is expressed more abundantly in cancer cells than in in non-cancerous cells. Optionally, the cells obtained from the subject are T cells. Optionally, the modified cells are expanded prior to administration to the subject.

It is to be understood that the figures and descriptions of the disclosure have been simplified to illustrate elements that are relevant for a clear understanding of the disclosure. It should be appreciated that the figures are presented for illustrative purposes and not as construction drawings. Omitted details and modifications or alternative embodiments are within the purview of persons of ordinary skill in the art.

It can be appreciated that, in certain aspects of the disclosure, a single component may be replaced by multiple components, and multiple components may be replaced by a single component, to provide an element or structure or to perform a given function or functions. Except where such substitution would not be operative to practice certain embodiments of the disclosure, such substitution is considered within the scope of the disclosure.

The examples presented herein are intended to illustrate potential and specific implementations of the disclosure. It can be appreciated that the examples are intended primarily for purposes of illustration of the disclosure for those skilled in the art. There may be variations to these diagrams or the operations described herein. For instance, in certain cases, method steps or operations may be performed or executed in differing order, or operations may be added, deleted or modified.

Where a range of values is provided, it is understood that each intervening value, to the smallest fraction of the unit of the lower limit, unless the context clearly dictates otherwise, between the upper and lower limits of that range is also specifically disclosed. Any narrower range between any stated values or unstated intervening values in a stated range and any other stated or intervening value in that stated range is encompassed. The upper and lower limits of those smaller ranges may independently be included or excluded in the range, and each range where either, neither, or both limits are included in the smaller ranges is also encompassed within the technology, subject to any specifically excluded limit in the stated range. Where the stated range includes one or both of the limits, ranges excluding either or both of those included limits are also included.

Different arrangements of the components depicted in the drawings or described above, as well as components and steps not shown or described are possible. Similarly, some features and sub-combinations are useful and may be employed without reference to other features and sub-combinations. Embodiments of the disclosure have been described for illustrative and not restrictive purposes, and alternative embodiments will become apparent to readers of this patent. Accordingly, the present disclosure is not limited to the embodiments described above or depicted in the drawings, and various embodiments and modifications can be made without departing from the scope of the claims below.

### EXAMPLES

### Example 1. Materials and Methods

**Plasmids.** CD63-pEGFP-C2 (Addgene #62964) and pMD2.G (Addgene #12259) were purchased from Addgene. Plasmids generated for the studies described herein are provided in Table 2. Gene synthesis was done by GenScript Inc. (Piscataway NJ). All constructs generated were confirmed by Sanger sequencing. Sequence information for primers, oligoes, and synthesized DNA fragments is in Table 3. Target sequences for sgRNAs are listed in Table 4.All constructs generated were confirmed by Sanger sequencing. Sequence information for primers and oligonucleotides are listed in Tables 3 and 4. It is understood that the sequences for the components of the plasmids listed in Table 2 can be separated by nucleic acid linkers, for example, linkers of about 2 to 100 bases. Optionally, any of the constructs described herein can include one or more introns, for example, between the promoter sequence and a nucleic acid encoding a polypeptide sequence (e.g., CD63, ABE etc.), to facilitate expression of one or more polypeptides sequences in the construct.

**Table 2. Plasmids**

| No. | Name | Purpose | Generation strategy |
|---|---|---|---|
| 1 | pCom-CD63 | Mammalian expression plasmid expressing a fusion protein Com-CD63 (Com at the N-terminus). | Use COM-CD63-F1 (cgtcagatccgctagCCACCATGAAATCAATTCGCtgtaaaaactg) and COM-CD63-R1 (CGGGCCTGGGCTCTGTCCACCTCCACCTCCGGAGTTGT GACCGCCATAACGCACGGTTTC) to amplify the COM coding region 251 bp from pspAX2-D64V-NC-com-new 2. The fragment was inserted between the NheI-KpnI sites ofplasmid CD63-pEGFP C2 (Addgene # 62964) by infusion reaction. |
| | | This plasmid comprises a CMV enhancer and promoter (SEQ ID NO: 36 ), a 5' UTR (SEQ ID NO: 37 ), an ABP-encoding (Com) sequence (SEQ ID NO: 7), a sequence encoding linker (SEQ ID NO: 38), and a sequence encoding CD63 (SEQ ID NO: 42). | cgttacataacttacggtaaatggcccgcctggctgaccgcccaacgacccccgcccattg acgtcaataatgacgtatgttcccatagtaacgccaatagggactttccattgacgtcaatgg gtggagtatttacggtaaactgcccacttggcagtacatcaagtgtatcatatgccaagtacg ccccctattgacgtcaatgacggtaaatggcccgcctggcattatgcccagtacatgacctt atgggactttcctacttggcagtacatctacgtattagtcatcgctattaccatggtgatgcgg ttttggcagtacatcaatgggcgtggatagcggtttgactcacggggatttccaagtctccac cccattgacgtcaatgggagtttgttttggcaccaaaatcaacgggactttccaaaatgtcgt aacaactccgccccattgacgcaaatgggcggtaggcgtgtacggtgggaggtctatata agcagagctggtttagtgaaccgtcagatccgctagCCACCATGAAATCAA TTCGCtgtaaaaactgcaacaaactgttatttaaggcggattcctttgatcacattgaaat caggtgtccgcgttgcaaacgtcacatcataatgctCaaCgcTtgTgaAcaCccTac ggagaaacattgtgggaaaagagaaaaaatcacgcattctgacGAAACCGTGC GTTATGGCGGTCACAACTCCGGAGGTGGAGGTGGACA GAGCCCAGGCCCGgcagccatggcggtggaaggaggaatgaaatgtgtgaa gttcttgctctacgtcctcctgctggccttttgcgcctgtgcagtgggactgattgccgtgggt |
| | | | gtcggggcacagcttgtcctgagtcagaccataatccagggggctacccctggctctctgt tgccagtggtcatcatcgcagtgggtgtcttcctcttcctggtggcttttgtgggctgctgcgg ggcctgcaaggagaactattgtcttatgatcacgtttgccatctttctgtctcttatcatgttggt ggaggtggccgcagccattgctggctatgtgtttagagataaggtgatgtcagagtttaata acaacttccggcagcagatggagaattacccgaaaaataaccacactgcttcgatcctgga caggatgcaggcagattttaagtgctgtggggctgctaactacacagattgggagaaaatc ccttccatgtcgaagaaccgagtccccgactcctgctgcattaatgttactgtgggctgtgg gattaatttcaacgagaaggcgatccataaggagggctgtgtggagaagattgggggctg gctgaggaaaaatgtgctggtggtagctgcagcagcccttggaattgcttttgtcgaggtttt gggaattgtctttgcctgctgcctcgtgaagagtatcagaagtggctacgaggtgatgtag (SEQ ID NO: 46) |
| | | The protein expressed by the plasmid comprises Com (SEQ ID NO: 8), a linker (SEQ ID NO: 39) and CD63 (SEQ ID NO: 44). | MKSIRCKNCNKLLFKADSFDHIEIRCPRCKRHIIMLNACE HPTEKHCGKREKITHSDETVRYGGHNSGGGGGQSPGPAA MAVEGGMKCVKFLLYVLLLAFCACAVGLIAVGVGAQLV LSQTIIQGATPGSLLPVVIIAVGVFLFLVAFVGCCGACKEN YCLMITFAIFLSLIMLVEVAAAIAGYVFRDKVMSEFNNNF RQQMENYPKNNHTASILDRMQADFKCCGAANYTDWEKI PSMSKNRVPDSCCINVTVGCGINFNEKAIHKEGCVEKIGG WLRKNVLVVAAAALGIAFVEVLGIVFACCLVKSIRSGYE VM (SEQ ID NO: 47) |
| 2 | pCD63-Com | Mammalian expression plasmid expressing a fusion protein CD63-Com (Com at the C-terminus). | Use CD63-COM-F1 (ttgggggctggctgaggaa) (SEQ ID NO: 109) and CD63-COM-MR (ccatggatccacctccaccggacatcacctcgtagccacttc) (SEQ ID NO: 110) to amplify a 153 bp fragment from pCD63-EGFP-C2 (Addgene ID 62964). Use CD63-COM-MF (gaagtggctacgaggtgatgtccggtggaggtggatccatgg) (SEQ ID NO: 111) and CD63-COM-R (Tagatccggtggatcagatctctaataacgcacggtttcgtcag) (SEQ ID NO: 112) to amplify the 305 bp fragment encoding COM. Then use the two PCR fragment as the template and use CD63-COM-F1 and CD63-COM-R to amplify the 416 bp fragment, which is then inserted into BbvC1-BamH1 sites of plasmid CD63-pEGFP C2 (Addgene # 62964) by Infusion reaction. |
| | | This plasmid comprises a CMV enhancer and promoter (SEQ ID NO:36), a 5' UTR (SEQ ID NO:37), a sequence encoding CD63 (SEQ ID NO:42), a sequence encoding a linker (SEQ ID NO: 40), and an ABP-encoding (Com) sequence (SEQ ID NO: 7) ). | |
| | | | Gacattgattattgactagttattaatagtaatcaattacggggtcattagttcatagccca tatatggagttccgcgttacataacttacggtaaatggcccgcctggctgaccgcccaac gacccccgcccattgacgtcaataatgacgtatgttcccatagtaacgccaatagggac tttccattgacgtcaatgggtggagtatttacggtaaactgcccacttggcagtacatcaa gtgtatcatatgccaagtacgccccctattgacgtcaatgacggtaaatggcccgcctgg cattatgcccagtacatgaccttatgggactttcctacttggcagtacatctacgtattagtc atcgctattaccatggtgatgcggttttggcagtacatcaatgggcgtggatagcggtttg actcacggggatttccaagtctccaccccattgacgtcaatgggagtttgttttggcacca aaatcaacgggactttccaaaatgtcgtaacaactccgccccattgacgcaaatgggc ggtaggcgtgtacggtgggaggtctatataagcagagctggtttagtgaaccgtcagatc cgctagtcgagctcaagcttcgaattctgcagtcgacggtaccagagcccaggcccggca gccatggcggtggaaggaggaatgaaatgtgtgaagttcttgctctacgtcctcctgc tggccttttgcgcctgtgcagtgggactgattgccgtgggtgtcggggcacagcttgtc ctgagtcagaccataatccagggggctacccctggctctctgttgccagtggtcatcat cgcagtgggtgtcttcctcttcctggtggcttttgtgggctgctgcggggcctgcaagg agaactattgtcttatgatcacgtttgccatctttctgtctcttatcatgttggtggaggt ggccgcagccattgctggctatgtgtttagagataaggtgatgtcagagtttaataac aacttccggcagcagatggagaattacccgaaaaataaccacactgcttcgatcctg gacaggatgcaggcagattttaagtgctgtggggctgctaactacacagattgggag aaaatcccttccatgtcgaagaaccgagtccccgactcctgctgcattaatgttactgt gggctgtgggattaatttcaacgagaaggcgatccataaggagggctgtgtggaga agattgggggctggctgaggaaaaatgtgctggtggtagctgcagcagcccttggaa |
| | | | ttgcttttgtcgaggttttgggaattgtctttgcctgctgcctcgtgaagagtatcagaa gtggctacgaggtgatgtccggtggaggtggatccatggcttctaactttactcagttcgtt ctcgtcgacaatggcggaactggcGACGTGATGAAATCAATTCGCtgt aaaaactgcaacaaactgttatttaaggcggattcctttgatcacattgaaatcaggtgtccg cgttgcaaacgtcacatcataatgctCaaCgcTtgTgaAcaCccTacggagaaacat tgtgggaaaagagaaaaaatcacgcattctgacgaaaccgtgcgttattag (SEQ ID NO: 48) |
| | | The protein expressed by the plasmid comprises C63 (SEQ ID NO: 43), a linker sequence (SEQ ID NO: 41)and Com (SEQ ID NO: 8). | MAVEGGMKCVKFLLYVLLLAFCACAVGLIAVGVGAQLV LSQTIIQGATPGSLLPVVIIAVGVFLFLVAFVGCCGACKEN YCLMITFAIFLSLIMLVEVAAAIAGYVFRDKVMSEFNNNF RQQMENYPKNNHTASILDRMQADFKCCGAANYTDWEKI PSMSKNRVPDSCCINVTVGCGINFNEKAIHKEGCVEKIGG WLRKNVLVVAAAALGIAFVEVLGIVFACCLVKSIRSGYE VMSGGGGSMASNFTQFVLVDNGGTGDVMKSIRCKNCN KLLFKADSFDHIEIRCPRCKRHIIMLNACEHPTEKHCGKR EKITHSDETVRY (SEQ ID NO: 49) |
| 3 | pCom-CD63-Com | Mammalian expression plasmid expressing a fusion protein Com-CD63-Com (Com at both ends of CD63). | Use COM-CD63-F1 (cgtcagatccgctagCCACCATGAAATCAATTCGCtgtaaaaactg) (SEQ ID NO: 113) and COM-CD63-R1 (CGGGCCTGGGCTCTGTCCACCTCCACCTCCGGAGTTGT GACCGCCATAACGCACGGTTTC) (SEQ ID NO: 114) to amplify the COM coding region 251 bp from pspAX2-D64V-NC-com-new 2. The fragment was inserted between the NheI-KpnI sites of plasmid CD63-pEGFP C2 (Addgene # 62964) by infusion reaction. |
| | | This plasmid comprises a CMV enhancer and promoter (SEQ ID NO:36), a5'UTR (SEQ ID NO: 37 ), a nucleic acid encoding a linker sequence (SEQ ID NO: 37 ), an ABP-encoding (Com) sequence (SEQ ID NO: 36 ). a sequence encoding CD63 (SEQ ID NO: 42), a nucleic acid encoding a linker (SEQ ID NO: 40) and an ABP-encoding (Com) sequence (SEQ ID NO: 7). | cgttacataacttacggtaaatggcccgcctggctgaccgcccaacgacccccgcccattg acgtcaataatgacgtatgttcccatagtaacgccaatagggactttccattgacgtcaatgg gtggagtatttacggtaaactgcccacttggcagtacatcaagtgtatcatatgccaagtacg ccccctattgacgtcaatgacggtaaatggcccgcctggcattatgcccagtacatgacctt atgggactttcctacttggcagtacatctacgtattagtcatcgctattaccatggtgatgcgg ttttggcagtacatcaatgggcgtggatagcggtttgactcacggggatttccaagtctccac cccattgacgtcaatgggagtttgttttggcaccaaaatcaacgggactttccaaaatgtcgt aacaactccgccccattgacgcaaatgggcggtaggcgtgtacggtgggaggtctatata agcagagctggtttagtgaaccgtcagatccgctagCCACCATGAAATCAA TTCGCtgtaaaaactgcaacaaactgttatttaaggcggattcctttgatcacattgaaat caggtgtccgcgttgcaaacgtcacatcataatgctCaaCgcTtgTgaAcaCccTac ggagaaacattgtgggaaaagagaaaaaatcacgcattctgacGAAACCGTGC GTTATGGCGGTCACAACTCCGGAGGTGGAGGTGGACA GAGCCCAGGCCCGgcagccatggcggtggaaggaggaatgaaatgtgtgaa gttcttgctctacgtcctcctgctggccttttgcgcctgtgcagtgggactgattgccgtgggt gtcggggcacagcttgtcctgagtcagaccataatccagggggctacccctggctctctgt tgccagtggtcatcatcgcagtgggtgtcttcctcttcctggtggcttttgtgggctgctgcgg ggcctgcaaggagaactattgtcttatgatcacgtttgccatctttctgtctcttatcatgttggt ggaggtggccgcagccattgctggctatgtgtttagagataaggtgatgtcagagtttaata acaacttccggcagcagatggagaattacccgaaaaataaccacactgcttcgatcctgga caggatgcaggcagattttaagtgctgtggggctgctaactacacagattgggagaaaatc ccttccatgtcgaagaaccgagtccccgactcctgctgcattaatgttactgtgggctgtgg gattaatttcaacgagaaggcgatccataaggagggctgtgtggagaagattgggggctg gctgaggaaaaatgtgctggtggtagctgcagcagcccttggaattgcttttgtcgaggtttt gggaattgtctttgcctgctgcctcgtgaagagtatcagaagtggctacgaggtgatgtccg gtggaggtggatccatggcttctaactttactcagttcgttctcgtcgacaatggcggaactg gcGACGTGATGAAATCAATTCGCtgtaaaaactgcaacaaactgttattt aaggcggattcctttgatcacattgaaatcaggtgtccgcgttgcaaacgtcacatcataatg ctCaaCgcTtgTgaAcaCccTacggagaaacattgtgggaaaagagaaaaaatcac gcattctgacgaaaccgtgcgttattag (SEQ ID NO: 50) |
| | | The protein expressed by the plasmid comprises Com (SEQ ID NO: 8), a linker sequence (SEQ ID NO: 39 ) | MKSIRCKNCNKLLFKADSFDHIEIRCPRCKRHIIMLNACE HPTEKHCGKREKITHSDETVRYGGHNSGGGGGQSPGPAA MAVEGGMKCVKFLLYVLLLAFCACAVGLIAVGVGAQLV LSQTIIQGATPGSLLPVVIIAVGVFLFLVAFVGCCGACKEN YCLMITFAIFLSLIMLVEVAAAIAGYVFRDKVMSEFNNNF RQQMENYPKNNHTASILDRMQADFKCCGAANYTDWEKI PSMSKNRVPDSCCINVTVGCGINFNEKAIHKEGCVEKIGG WLRKNVLVVAAAALGIAFVEVLGIVFACCLVKSIRSGYE VMSGGGGSMASNFTQFVLVDNGGTGDVMKSIRCKNCN KLLFKADSFDHIEIRCPRCKRHIIMLNACEHPTEKHCGKR EKITHSDETVRY (SEQ ID NO: 51) |
| | | CD63 (SEQ ID NO: 43), a linker sequence (SEQ ID NO: 41)and Com (SEQ ID NO: 8). | |

**Table 3. Primers**

| Primer name | SEQ | Use |
|---|---|---|
| Scid-g2F | CACCGACACAGACAGACTAC ACCCA (SEQ ID NO: 52) | For PCR detection of *IL2RG* targeting *sgRNA.* |
| sgRNA-R3 | GATAAACACGGCATTTTGCCT TG (SEQ ID NO: 53) | |
| Reporter-F | Tccatttcaggtgtcgtgag (SEQ ID NO: 54) | To amplify the DNA in the GFP reporter cassette. |
| Reporter-R2 | TCCAGCTCGACCAGGATG (SEQ ID NO: 55) | |
| IL2RG-F2 | GAAGCTATGACAGAGGAAAC G (SEQ ID NO: 56) | Used with IL2RG-3301R to amplify the IL2RG DNA for NGS. |
| HBB-R1 | AGCCAGGGCTGGGCATAAAA G (SEQ ID NO: 57) | Used to amplify the HBB region for NGS with HBB-R3 |
| HBB-R3 | TGGGAAAATAGACCAATAGG CAGAG (SEQ ID NO: 58) | Used to amplify the HBB region for NGS with HBB-R or HBB-R |
| IL2RG-3301R | GGCAGCTGCAGGAATAAGAG (SEQ ID NO: 59) | To amplify the endogenous IL2RG target sequence |
| DMD50-F | GCTGCTCTTTCTGGCATTG (SEQ ID NO: 60) | To detect DNA deletion between sgRNA Sa-50 and DMD-53 (96kb) by PCR. |
| DMD53-R | TCCAGCCATTGTGTTGAATC (SEQ ID NO: 61) | |
| DMD53-F | TCCTGTTGTTCATCATCCTAGC (SEQ ID NO: 62) | To amplify the DMD 53 exon target region for NGS |
| DMD53-R | TCCAGCCATTGTGTTGAATC (SEQ ID NO: 63) | |
| hCLCN5-F | | To amplify the CLCN5 target region for NGS |
| hCLCN5-R | TGTCTTACCTCTCGGTGCCT (SEQ ID NO: 65) | To amplify the CLCN5 target region for NGS |
| DMD50-F | GCTGCTCTTTCTGGCATTG (SEQ ID NO: 66) | To detect *DMD* exon 50 deletion between sgRNA Sa-50 and Sa-51 by PCR. |
| DNM51-R2 | CAGTTACAGTTATTACCGCAG CA (SEQ ID NO: 67) | |
| ABE-g5-onF | GTCTGAGGTCACACAGTGGG (SEQ ID NO: 68) | For qPCR to detect base editing at ABE site 5 with ABE-g5-onF |
| g5-ABE-R | AGCCCTGACTCATCATTACCC (SEQ ID NO: 69) | |
| GAPDH-onF | AGGAGTAAGACCCCTGGACC (SEQ ID NO: 70) | To amplify the GAPDH target region for NGS |
| GAPDH-onR | TCTCACCTTGACACAAGCCC (SEQ ID NO: 71) | |
| P53-onF | CTGGCATTCTGGGAGCTTCA (SEQ ID NO: 72 | To amplify the TP53 target region for NGS |
| P53-onR | GAGACCTGTGGGAAGCGAAA (SEQ ID NO: 73) | |
| g5-onF | GTCTGAGGTCACACAGTGGG (SEQ ID NO: 74) | To amplify the intergenic region of chromosome 20 for NGS |
| g5-onR | CTGAGAGCAGGGACCACATC (SEQ ID NO: 75) | |

**Table 4. Target sequences and oligonucleotide sequences for cloning guide into sgRNA-expressing plasmid.**

| Target gene | Target sequence with PAM | sgRNA name | Forward Oligo for cloning | Reverse Oligo for cloning | Vector | Restriction enzyme |
|---|---|---|---|---|---|---|
| **For SpCas9** | | | | | | |
| *IL2RG* | GCGCTTGCTCT TCATTCCCTGG G (SEQ ID NO: 76) | *IL2RG* | ACCGGCGCTT GCTCTTCATTC CCT (SEQ ID NO: 86) | AAACAGGAAT GAAGAGCAAGC GC (SEQ ID NO: 96) | pspCas9 -3'UTR-ST2-com-vector | BbsI |
| *HBB(Sic* kle mutant) | GTAACGGCAG ACTTCTCCTCAGG GG (SEQ ID NO: 77) | *HBB-sp-gl* | ACCGGTAACG GCAGACTTCTC CAC (SEQ ID NO: 87) | AAACGTGGAGA AGTCTGCCGTT AC (SEQ ID NO: 97) | pspCas9 -3'UTR-Tetra-com-vector, | BbsI |
| *DMD* Exon 53 | ACTGTTGCCTC CGGTTCTGAAG G (SEQ ID NO: 78) | *DMD53* | ACCGactgttgcctc cggttctga (SEQ ID NO: 88) | AAACtcagaaccgga ggcaacagt (SEQ ID NO: 98) | pspCas9 -3'UTR-ST2-com-vector | BbsI |
| CLCN5 Exon 2 | GAGGACAAGT CGTACAATGGT GG (SEQ ID NO: 79) | *hCLCN5-sp-g2* | ACCGGAGGAC AAGTCGTACA ATGG (SEQ ID NO: 89) | AAACCCATTGT ACGACTTGTCC TC (SEQ ID NO: 99) | pspCas9 -3'UTR-Tetra-com-vector | BbsI |
| Intergenic site 5 | GATGAGATAA TGATGAGTCAG GG (SEQ ID NO: 80) | G5 | ACCG GATGAGATAA TGATGAGTCA (SEQ ID NO: 90) | aaacTGACTCATC ATTATCTCATC (SEQ ID NO: 100) | pspCas9 -3'UTR-ST2-com-vector | BbsI |
| TP53 | CCATTGTTCAA TATCGTCCGGG G (SEQ ID NO: 81) | P53-g1 | ACCGCCATTGT TCAATATCGTC CG (SEQ ID NO: 91) | AAACCGGACGA TATTGAACAAT GG (SEQ ID NO: 101) | pspCas9 -3'UTR-ST2-com-vector | BbsI |
| GAPDH | AGCCCCAGCA AGAGCACAAG AGG (SEQ ID NO: 82) | GAPDH-g1 | ACCGAGCCCC AGCAAGAGCA CAAG (SEQ ID NO: 92) | AAACCTTGTGC TCTTGCTGGGG CT (SEQ ID NO: 102) | pspCas9 -3'UTR-ST2-com-vector | BbsI |

| **For SaCas9** | | | | | | |
|---|---|---|---|---|---|---|
| *DMD* Intron 50 | TATGTGGCTTT ACCAAGGTCCC AGAGT (SEQ ID NO: 83) | *Sa-50* | CACCgTATGTG GCTTTACCAAG GTCC (SEQ ID NO: 93) | AAACGGACCTT GGTAAAGCCAC ATAc (SEQ ID NO: 103) | pX601-Tetra-com-vector | BsaI |
| *DMD* Intron 51 | GTGTTATTACT TGCTACTGCA GAGAGT (SEQ ID NO: 84) | *Sa-51* | CACCGTGTTAT TACTTGCTACT GCA (SEQ ID NO: 94) | AAACTGCAGTA GCAAGTAATAA CAC (SEQ ID NO: 104) | pX601-Tetra-com-vector | BsaI |
| *IL2RG* | ACACAGACAG ACTACACCCAG GGAAT (SEQ ID NO: 85) | *IL2RG* | CACCGACACA GACAGACTAC ACCCA (SEQ ID NO: 95) | AAACTGGGTGT AGTCTGTCTGT GTC (SEQ ID NO: 105) | pX601-Tetra-com-vector | BsaI |

**GFP reporter assays for gene editing activities.** HEK293T derived HBB-IL2RG EGFP reporter cells with target sequences for human beta hemoglobin (HBB) sickle cell mutant and human IL2RG (Javidi-Parsijani et al. PLoS One 12, e0177444 (2017)), and DMD reporter cells with target sequence from human DMD exon 53 (Lyu et al., PLoS One 15, e0239468 (2020)) were used to detect gene editing activities of Cas9 RNPs targeting HBB, IL2RG, and DMD exon 53, respectively. The GFP-reporter cells expressed no EGFP due to the disruption of the EGFP reading frame by the insertion of the respective target sequences right after the start codon of EGFP coding sequence. INDELs formed after gene editing may restore the EGFP reading frame, resulting in EGFP expression. GFP-positive cells were analyzed by fluorescence microscopy or flow cytometry (BD Biosciences, Accuri C6 (San Jose, CA)).

**Production of Cas9 or ABE RNP-enriched extracellular vesicles (EVs).** RNP-enriched EVs were produced by co-transfection of three plasmids into HEK293T cells: plasmid DNA expressing a fusion protein between ABP Com and CD63, pDM2.G expressing VSV-G, and the target plasmid expressing the gene editing effector (SaCas9, SpCas9 or ABE) and the respective gene-specific sgRNA (See Table 2 for various target plasmids). Briefly, 5 million actively proliferating HEK293T cells grown in 10-cm dishes were incubated with 5 ml Opti-MEM^{®} medium. 3 µg of Com-CD63-Com fusion protein expressing plasmid, 3 µg pMD2.G, and 12 µg target plasmid DNA were mixed in 0.5 ml Opti-MEM^{®} medium. Fifty-four µl of Fugene HD (Promega (Madison, WI)) or 54 µg of polyethylenimine (Synchembio, Cat. No. SH-35421, Chicago, IL) were mixed in 0.5 ml Opti-MEM^{®} medium. The DNA mixture and the transfection reagent mixture were then mixed and incubated at room temperature for 15 mins, before they were added to the cells in Opti-MEM^{®} medium. 24 hr after transfection, the medium was changed into 10 ml Opti-MEM^{®} medium and the RNP-enriched EVs were collected 72 hr after transfection. For transfection of cells grown in other tissue culture vessels, the amounts of DNA and transfection reagent were scaled based on tissue culture surface area.

**EV concentration.** Ultracentrifugation was used to concentrate EVs from tissue culture medium following procedures described in Lu et al. (PloS One 12, e e0185992 (2017)). Briefly, the cell culture medium was centrifuged at 1000 × g for 30 minutes at 4°C to remove cell debris. The supernatant was centrifuged at 120,000 × g for 70 minutes at 4°C. The pellet was washed once with PBS and centrifuged again under the same conditions. The resulting pellet containing the EVs was resuspended in PBS. Typically, EVs from 10 ml supernatants were resuspended in 500 µl (20X concentration) for in vitro experiments. These EVs can be stored at -80°C or be used immediately.

**Nanoparticle tracking analysis of EVs.** Hydrodynamic diameters and concentrations of EVs were measured using the Nanosight NS500 instrument (Malvern Instruments, UK) using the instrument's software (version NTA3.2). The instrument was primed using phosphate buffered saline (PBS), pH 7.4 and the temperature was maintained at 25 °C. Accurate particle tracking was verified using 50 nm and 100 nm polystyrene nanoparticle standards (Malvern Instruments) prior to examining samples. Concentrated samples containing EVs were serial diluted 1000 fold in PBS. The linear range for quantification of EV concentration in each sample fell between 10-40,000 fold dilutions. Therefore, all samples were diluted 1,000-fold in PBS. Five independent measurements (60 sec each) were obtained for each sample in triplicate. Data are reported as the mean (multiplied by dilution factor for concentration determination) of these measurements ± standard error of the mean.

**EV mediated RNP delivery.** RNP enriched EVs concentrated from supernatant of 0.6~20 million cells were added to 2.5x10⁴ cells grown in Opti-MEM (Cat. No. 11058021, ThermoFisher, Waltham, MA) in 24-well plates. It is important that the medium had low serum, since the presence of FBS in the medium inhibits EV mediated RNP delivery. After incubation for 12-24 hours, the medium was changed to DMEM medium with 10% FBS. Thirty-six hours after EV treatment, gene editing was analyzed by flow cytometry, fluorescent microscopy or next generation sequencing (NGS).

**Examining degradation of EV-delivered Cas9.** HEK293T cells (2.5x10⁴) were grown in RMPI1640 medium with 0.5% FBS in 24-well plates to limit proliferation. The cells were treated with RNP-loaded EVs at different times, but were collected at the same time. EVs secreted by 0.2 million cells in 48 hours (~8x10⁹ vesicles) were added to each well. Just before EV treatment, the medium was changed to Opti-MEM^{®} medium. The cells were collected at 6 hr, 12 hr, 18 hr, 24 hr, 36 hr, and 48 hr after EV addition, washed twice with PBS buffer, and lysed in Laemmli buffer for Western blot analysis.

**Western blotting analysis of EVs.** EVs secreted by 5x10⁶ cells in 48 hours were concentrated and resuspended in 500 µl PBS. Sixty µl of EV solution was mixed with equal volume of 2x Laemmli buffer, and boiled at 95 °C for 5 mins. Ten µl of each sample was loaded in each lane of a 10% SDS-PAGE gel for Western blot analysis. The antibodies used included mouse monoclonal anti-SaCas9 antibody (Millipore Sigma, Cat. MAB131872, clone 6F7, 1:1000 (St. Louis, MO)), GAPDH antibody (CST, 1:1000), RAB5B antibody (Abcam, Cat. ab230020, 1:1000 (Cambridge, UK)), CD9 antibody (SBI, Cat. EXOAB-CD9A-1, 1:1000 (Palo Alto, CA)), VSV-G antibody (Sigma, Cat. V4888, 1:1000), CD63 antibody (Abcam, Cat. ab68418, 1:1000), GRP94 antibody (CST, Cat. 20292T, 1:1000 (Danvers, MA)), and anti-Rabbit IgG (H+L) (Cat No. 31460, 1:5000) secondary antibodies. Chemiluminescent reagents (Pierce ECL Western blotting substrate, Cat.32106 (Waltham, MA)) were used to visualize the protein signals under the LAS-3000 system (Fujifilm (Valhalla, NY)). Densitometry (ImageJ software (Version 1.49), National Institutes of Health, imagej.nih.gov/ij/index.html) was used to quantitate protein amount based on Western blotting images.

**RT-qPCR and qPCR analyses.** A RNeasy^{®} Plus Mini Kit (QIAGEN Cat No. 74136 (Hilden, Germany)) was used to isolate RNA from collected EVs. The QuantiTect^{®} Reverse Transcription Kit (QIAGEN) was used to reverse-transcribe the RNA to cDNA. For HBB sgRNA1 and HBB sgRNA1Tetra-com detection, sgRNA-F1 and sgRNA-R3 were used as primers in SYBR^{™} Green based RT-qPCR. ABE-g5-onF and g5-ABE-R were used as qPCR primers to detect base editing at site 5. PCR was run on an ABI 7500 instrument. Primer information is included in Table 3.

**Transmission electron microscopy.** Transmission electron microscopy was performed at the Cellular Imaging Shared Resource of Wake Forest Baptist Health Center (Winston-Salem, NC). Collected EVs were (about 6.0 x 10¹¹ vesicles/ml) stained with uranyl acetate. The particles were absorbed on plain carbon grids, dried and observed under a FEI Tecnai G2 30 electron microscope (FEI, Hillsboro, OR). The diameters of the particles were measured with NIH ImageJ software (Version 1.49).

**Next-generation sequencing and data analysis.** Genomic DNA was isolated from cultured cells with the DNeasy Blood & Tissue Kit (Qiagen). The DNA region containing the target sequences were amplified by the proofreading HotStart^{®} ReadyMix from KAPA Biosystems (Wilmington, MA). PCR primers used for amplifying each target sequence were listed in Table 3. The purified PCR products were shipped to Genewiz Inc. (Morrisville, NC) to perform next generation sequencing using the Amplicon EZ service. Usually 50,000 reads/amplicon were obtained. Analysis of insertions and deletions (INDEL) was done with the online Cas-Analyzer software 28 and CRISPRESSO2 29, which gave very similar results.

**Intramuscular injection of RNP loaded EVs to mouse tibialis anterior (TA) muscle.** Five-month old female del52hDMD/mdx mice were used for injection. These mice carry copies of the human DMD gene with an exon 52 deletion in an mdx background (Veltrop, M. et al., PLoS One 13, e0193289 (2018)). EVs secreted by 5 million cells (low dosage) or 40 million cells (high dosage) in 48 hours were injected into each TA muscle using a Hamilton syringe. The mice were sacrificed 7 days after injection. TA muscles were removed for cryosections. The tissues were mounted in OCT embedding compound and frozen in liquid nitrogen. Six or seven blocks of three continuous 10-µm-thick sections, 200 µm away from each block, were collected for histological analysis. The rest of the tissues were used to purify genomic DNA for NGS analysis.

**Immunohistochemistry assay.** The sections were fixed in 4% paraformaldehyde for 10 minutes. After 3 times of PBS washing, the sections were incubated with 0.2% TritonX-100 PBS buffer for 10 minutes, and with Protein Block (Cat. X090930-2, Agilent) for 30 minutes. The sections were then incubated with anti- dystrophin primary antibody (Abcam, ab3068050, 1:10000) for 1 hr. Then Alexa Fluor^{®} 633 secondary antibody (A-21052, Thermo Fisher, 1:5000) was incubated with the tissues for 45 minutes after PBS washing for 3 times. Then the slides were washed 6 times with PBS and mounted in DAPI-containing mounting medium for 5 minutes. The slides were observed under a fluorescence microscope.

**Statistical analysis.** GraphPad Prism software (version 5.0) was used for statistical analyses. T-tests were used to compare the averages of two groups. Analysis of Variance (ANOVA) was performed followed by Tukey post hoc tests to analyze data from more than two groups. p<0.05 was regarded as statistically significant.

### Example 2. CD63, aptamer and ABP mediated RNP enrichment in extracellular vesicles (EVs)

The experiments described above were used to determine whether the specific interaction between com and Com could enrich RNPs into EVs. CD63 is a tetraspan transmembrane protein with the N- and C-termini in the cytoplasm. Com was fused to the N-terminus, the C-terminus, or both termini of CD63 (Fig. 1A), so that Com faces the cytoplasmic side of the plasma membrane. It was hypothesized that, during exosome generation, Cas9 or ABE RNPs would be enriched in exosomes via interactions between CD63-Com, com-sgRNA, and Cas9 (Fig. 1B). Alternatively, RNPs can also be enriched in microvesicles via the outward budding and fission of membrane vesicles from the cell surface (Fig. 1C). In these studies, it is understood that all vesicle structures produced, including exosomes and microvesicles, are called EVs, regardless of the mechanisms of vesicle generation.

Whether the fusion of Com affected CD63 expression was examined, and it was found that fusing Com at either end of CD63 did not impair CD63 expression. Indeed, fusing Com at the C-terminus greatly increased the expression of CD63 (Fig. 2A). Whether Com fusion to CD63 enabled packaging of SaCas9 RNPs into EVs was also tested. Since escape of EV from cells can be limited, VSV-G was expressed in the EV producing cells so that the EVs have VSV-G protein to facilitate their escape from the endosome system in recipient cells. Com-CD63, CD63-Com or Com-CD63-Com was co-expressed with VSV-G, SaCas9 and com-modified IL2RG-targeting sgRNA 12 in HEK293T cells. The EVs were collected from the supernatant of the transfected cells and concentrated by ultracentrifugation. The resuspended EVs were added to the medium of HBB-IL2RG GFP reporter cells described previously (Javidi-Parsijani 2017)) . These cells expressed no EGFP due to the disruption of the EGFP reading frame by the insertion of the 119 nt HBB sickle mutation and IL2RG target sequences right after the start codon of EGFP coding sequence. INDELs formed after gene editing may restore the EGFP reading frame, resulting in EGFP expression.

Flow cytometry analysis found that without aptamer-binding protein Com or aptamer com, few GFP-positive cells could be observed, perahps indicative of random packaging of RNPs into the EVs. When the sgRNA was modified with aptamer com, Com-CD63-Com generated the most GFP-positive reporter cells, followed by CD63-Com and Com-CD63 (Fig. 2B). CD63-Com had stronger expression levels than Com-CD63, and the EV delivered RNPs also generated more GFP-positive cells than Com-CD63 did. The expression of Com-CD63-Com was weaker than that of CD63-Com, but its EV-associated RNPs generated significantly more GFP-positive cells. The data suggested that Com at both termini had synergistic effects on recruiting RNPs. The data showed that both the ABP (Com) and the aptamer (com) were needed for generating EV associated RNPs with high gene editing activity.

Com-CD63-Com was used in further experiments since it generated EV-associated RNPs with the highest gene editing activities. Different ratios of Com-CD63-Com and Cas9 RNP expressing plasmid DNA were tested and the best activities were obtained when they were at a mass ratio of 1:4 (Fig. 3).

VSV-G was co-expressed in the EV production cells to help the EVs escape from the endosome system in recipient cells. To check whether VSV-G was necessary for functional delivery of the EV-associated RNPs, EV-associated RNPs were generated in the absence of VSV-G. These RNPs generated background levels of GFP-positive reporter cells (Fig. 2C). Whether fusing Com to the C-terminus of VSV-G could further increase gene editing activity was tested. This fusion greatly decreased gene editing activity of the EV-associated RNPs. Two possibilities might underlie this observation: 1) fusing Com to the C-terminus of VSV-G decreased the expression of VSV-G by over 50% (Fig. 4); and 2) doing so might interfere with VSV-G's fusogenic activity.

The data showed that RNPs can be enriched in and functionally delivered by EVs. Further, the aptamer com, ABP Com and, optionally, VSV-G protein, could be used for functional delivery of RNPs by EVs.

Whether SpCas9 RNPs could be packaged and delivered by EVs was ablso tested. Replacing the ST2 loop with com aptamer best preserves the functions of SpCas9 sgRNAs and enables efficient delivery of SpCas9 RNPs by lentivirus-like particles. Therefore, similarly modified sgRNA were used to package SpCas9 RNPs into EVs. Various Com- CD63 fusion proteins, VSV-G, SpCas9, and ST2-com modified IL2RG-targeting sgRNA, were co-expressed in HEK293T cells. The resultant EVs were applied to HBB-IL2RG GFP reporter cells. SpCas9 RNPs were efficiently packaged by Com-CD63-Com, and both the ABP and aptamer were needed for best gene editing activities (Fig. 2D). Thus, an ABP/aptamer interaction can be used to package SpCas9 RNPs into EVs.

Adenine base editor (ABE) RNPs targeting ABE site 5 (GRCh38.p13, chromosome 20, 32752960-32752979) 31 were packaged in EVs. qPCR showed that EV-delivered site 5 ABE RNPs generated 24.3 ± 0.7 (n=3) fold base edited products compared with EV-delivered non-targeting ABE RNPs. The data show that EVs can also enrich and deliver ABE RNPs.

Nuclear export of sgRNAs driven by U6 promoter can be inefficient. RNA polymerase II promoter-driven sgRNA, that was flanked by the Hammerhead (HH) ribozyme and hepatitis delta virus (HDV) ribozyme were used in some studies. This design has been shown to generate mature sgRNA after ribozyme cleavage (Yoshioka et al., Sci Rep 5, 18341 (2015). This design only slightly increased the gene editing activities of the EV-delivered RNPs (Fig. 5). This suggested that sgRNA nuclear export was not a limiting factor in these studies. It is possible that aptamer modified sgRNA provides an active enrichment mechanism to recruit sgRNAs into membrane vesicles.

### Example 3. Com/com interaction enriched RNPs into EVs

Whether Com/com interaction enriched RNPs into EVs was examined by Western blotting. EVs loaded with RNPs containing sgRNAs were prepared with and without com aptamer. SaCas9, SpCas9 and ABE content was detected in the respective EVs. 2-5-fold Cas9 or ABE protein was detected in RNPs with com-modified sgRNAs (com+) compared with RNPs with unmodified sgRNAs (com-), although the amount of Com-CD63-Com protein was comparable (Figs. 6A-6B). Consistent with these observations, the presence of Com and com packaged the most sgRNA in EVs (Fig. 6C). The data showed that Com/com interactions enriched RNPs into EVs although there was random packaging of RNPs. Using the Pierce^{™} BCA Protein Assay Kit, it was found that 1 million transfected cells secreted about 625 ng total EV proteins in 48 hours. Among which, 65 ng SaCas9, 80 ng SpCas9 and 35 ng ABE proteins were detected based on Western blotting of Cas9 proteins of known concentrations (Figs. 6A-6B).

### Example 4. EV delivered RNPs achieved efficient gene editing on multiple targets in different cells

Genome editing activities of the EV delivered RNPs, targeting various targets in different cells, were confirmed by NGS. As shown in Table 5 INDELs were observed in different lentiviral integrated targets in GFP-reporter cells, in HEK293T cells where the EVs were generated, and in MDA-MB-231 cells that are different from the EV source cells and are hard to transfect. A to G changes, on site 5, in different cells treated with EV- delivered ABE RNPs, were also detected (Table 5).

**Table 5. NGS analyses of INDEL rates and base editing rates of EV-delivered RNPs**

| | **SaCas9** | **SpCas9** | | | | **ABE** |
|---|---|---|---|---|---|---|
| | ***IL2RG*** | ***DMD*** | ***GAPDH*** | ***P53*** | ***Site 5*** | ***Site 5*** |
| **GFP-reporter cells** | 48.2% ^{a} | 51.4% ^{b} | ND | ND | ND | ND |
| **HEK293T** | ND | ND | 15.7% ^{c} | 16.1%^{d} | 35.5%^{d} | 39.7% ^{c} |
| **MDA-MB-231** | ND | ND | | 14.4% ^{d} | 33.8% ^{d} | 24.2% ^{c} |

For all assays, EVs were added to 2.5x10⁴ cells. ^{a}EVs produced by 0.6 million cells in 48 hours were added to *HBB-IL2RG* GFP-reporter cells. ^{b}EVs produced by 2.5 million cells were added to *DMD* GFP-reporter cells. ^{c}EVs produced by 5 million cells were added to the respective cells. ^{d}EVs produced by 20 million cells were added to the respective cells.

Based on estimation of production rates for EV-associated RNPs (Figs. 6A-B), the amounts of EV- delivered RNPs used in these experiments were between 0.13 to 6.4 µg for 105 cells. The highest dosage was lower than that used in a typical electroporation experiment (10 µg).

### Example 5. Characterization of EVs with RNP enrichment

Whether over-expressing Com-CD63-Com affected EV biogenesis, was examined, by isolating EVs from cells with and without Com-CD63-Com overexpression, and examining protein expression by Western blotting. It was found that the expression of exosome marker proteins CD9 and RAB5B was not decreased from EVs isolated from cells with Com-CD63-Com overexpression (Fig. 7A), suggesting that Com-CD63-Com overexpression did not decrease overall EV biogenesis. In addition, the overexpression of Cas9 RNPs did not impair EV biogenesis either. Com-CD63-Com overexpression greatly increased the detection of CD63-reactive antigens in EVs. The observed CD63 size was much larger than expected, and the bands appeared diffused. Both observations were the results of heterogeneous glycosylation of CD63. For the same reason, the size difference between endogenous CD63 and the overexpressed Com-CD63-Com were not obvious. Compared with cellular Com-CD63-Com (Fig. 2A), Com-CD63-Com in EVs apparently appeared larger and more heterogeneous in size. It seems that CD63 proteins in EVs had a high degree of glycosylation. As expected, VSV-G and Cas9 were only observed in EVs from cells overexpressing the respective protein. GRP94, a protein not involved in the endosome pathway, was not observed in the EV preparations, suggesting minimal cellular protein contamination.

Transmission electron microscopy was performed to examine the morphology of the EVs isolated from cells without CD63 overexpression, with CD63 or Com-CD63-Com overexpression (all cells overexpressed VSV-G and Cas9 RNPs). No difference was observed in morphology of the EVs isolated from these cells (Fig. 7B). We performed nanoparticle tracking analysis (Nanosight) to examine the particle number and size of the EVs isolated from the cells, and found that overexpressing Com-CD63-Com and Cas9 RNPs slightly decreased the total number of EV particles generated (Fig. 7C). In addition, overexpressing Com-CD63-Com and Cas9 RNPs changed the EV size distribution, with a decrease of EVs of 100 nm in diameter and an increase of EVs of 200 nm in diameter (Fig. 7D). The right shift of size distribution explained why CD9 and RAB5B expression in EVs was not decreased although the total EV number was decreased with Com-CD63-Com overexpression.

### Example 6. Short half-life of Cas9 RNPs delivered by EVs

In order to examine the fate of EV delivered RNPs in human cells, EV delivered, DMD exon 53-targeting SpCas9 RNPs were added to HEK293T cultures, and the cells were collected at different time after EV addition. Western blotting found that 6 hours after delivery, the SpCas9 protein level was the highest and thereafter SpCas9 protein level decreased quickly (Fig. 8A). Densitometry analysis revealed that 18 hours post-delivery, Cas9 level was only 10% of that of 6 hours post-delivery (Fig. 8B). The half-life of EV delivered SpCas9 protein in human cells was estimated to be 3 hours.

Similar experiments were performed using EV delivered, IL2RG-targeting SaCas9 RNPs, and similar, quick degradation of the RNPs (Fig. 5C) was observed. Thus Cas9 RNPs delivered by EVs had very short half- lives. This short half-life ensures the specificity and safety of EV delivered RNPs.

### Example 7. Single preparation of RNP-enriched EVs for multiplex gene editing

Since each CD63 molecule is fused to two Com molecules, and each EV may have more than one Com-CD63-Com molecule, it was reasoned that EVs could be ideal for delivering RNPs for simultaneously targeting multiple loci. Two SaCas9 RNPs, targeting DMD intron 50 (Sa-50) and intron 51 (Sa-51) respectively, to remove the 2361 bp between the two target sites (Fig. 9A), were tested. EVs loaded with RNPs targeting DMD intron 50, RNPs targeting DMD intron 51, and RNPs targeting both introns, were prepared. EVs loaded with RNPs targeting both introns were prepared in a single EV preparation simply by using half of each target plasmid DNA. The two individually packaged RNPs were used together to compare with the co-packaged RNPs for exon 51 removal. All RNP- loaded EVs were prepared in parallel and similarly concentrated. PCR was used to detect exon 51 removal: a 2645 bp would be amplified with primers DMD50-F and DMD51-R2 without exon 51 removal (Fig.9A), otherwise a 284 bp product would be amplified. A 284 bp amplicon was observed in cells treated with the co-packaged RNPs, but not in cells treated with the two individually packaged RNPs (Fig. 9B). DNA sequencing confirmed that the ~284bp amplicon was the result of deleting the sequences between the two sgRNAs. This experiment showed that one single preparation can produce EVs loaded with RNPs targeting more than one locus, and doing so is more efficient for multiplex gene targeting.

Packaging SaCas9 and SpCas9 RNPs in one single preparation was also tested. In this case, DMD intron 50-targeting SaCas9 RNPs and DMD exon 53-targeting SpCas9 RNPs were individually packaged or co-packaged into EVs. The two individually packaged RNPs were used together to compare with the co-packaged RNPs for removing the sequences between the two target sites 96 kb away (Fig.9A). PCR showed that the expected 263 bp DNA amplicon, indicating the deletion of the 96kb and confirmed by DNA sequencing, were only observed in cells treated with the co-packaged RNPs but not in cells treated with the two individually packaged RNPs (Fig. 9C). Thus RNPs of Cas9 from different species can also be co-packaged into EVs in single preparation for efficient multiplex gene editing.

### Example 8. In vivo activity of EV delivered RNPs

The in vivo activity of EV delivered DMD exon 53-targeting RNPs was examined. RNP- loaded EVs produced by 40 million cells in 48 hours were concentrated and injected into each TA muscle of del52hDMD/mdx mice. One week later, the mice were sacrificed and the TA muscles were collected to examine for target gene editing. Genomic DNA was isolated from the TA muscle and the target DNA region was amplified for NGS analysis. Up to 0.2% INDEL rates (316 of 157982 reads) were observed in the RNP injected muscle, and 0% INDEL rate was observed in the PBS injected muscle (0 of 51302 reads, p<0.0001 by Chi-square tests). The INDELs were all around the predicted cleavage site.

Immunostaining was performed to examine the expression of dystrophin in injected TA muscle. These mice have low background dystrophin expression in skeletal muscle due to spontaneous exon 53 skipping, which restores the dystrophin reading frame. In RNP injected TA muscle, areas with stronger dystrophin expression were observed, that were not observed in the PBS injected muscles.

The studies described herein showed that the specific interaction between an aptamer binding protein (fused to CD63) and an aptamer (inserted in sgRNA) could be used to actively enrich Cas9 and ABE RNPs in EVs. In this example, a com/Com interaction associates RNPs to the N- and C-termini of CD63, which is in the cytoplasm of the cells and the lumen of exosomes. Since CD63 is abundant in exosomes this method specifically enriched Cas9 and ABE RNPs into EVs. In fact, up to 10 times more gene editing activity was observed compared with EVs without RNP enriching mechanism (e.g., without aptamer com or aptamer-binding protein Com). Since the Cas9 and sgRNA expressing plasmid DNA were also present in conditions without RNP enriching mechanism, the low gene editing activity observed in these conditions ruled out a major contribution of EV transferred plasmid DNA.

It was interesting that adding aptamer-binding protein Com to both the N- and C-termini of CD63 showed a synergistic effect on gene editing activity. There could be several explanations to this observation: 1) Com may function as a homodimer and fusing Com to both the N- and C-termini of CD63 increases the chance of making a functional unit; or 2) a threshold of Cas9 molecules are needed to be functional and fusing Com to both the N- and C-termini of CD63 increases the chance of reaching that threshold.

Another interesting observation is that VSV-G could be important for genome editing activity for the EV delivered RNPs. The most likely explanation is that VSV-G helps the escape of the RNPs from the endosome system in recipient cells. Based on the studies described herein, it is likely that an intact and free C-terminus is important for VSV-G to induce endosome escape.

Advantages of an EV-mediated RNP delivery system are that RNPs targeting more than one loci and RNPs with Cas9 proteins from different species can be enriched in EVs in a single RNP preparation. As demonstrated herein, co-packaging of SaCas9 and SpCas9 RNPs is possible. It is expected that Cas9 proteins from other species may also be co-packaged as long as the com aptamer can be inserted into their sgRNA. In addition, the co-packaged RNPs are more active than the combination of the individually packaged RNPs for multiplex genome editing. These features make EVs an ideal delivery tool for multiplex genome editing, which is needed in many situations, including knockout of antigens to reduce the risk of immuno-rejection, eradicating HIV proviral DNA from a genome, and enhancing response in cancer therapy.

In addition to these advantages EVs may be more suitable for systemic delivery, for example, as compared to virus-like particles, which tend to be inactivated by complement system in circulation. Also, EVs have the ability to cross the blood brain barrier, thus expanding therapeutic potential. In summary, RNPs can be efficiently and functionally packaged into EVs. EV-delivered RNPs show high on-target gene editing activities.

### Sequences

| | | |
|---|---|---|
| SEQ ID NO:1 | MS2 coat protein (MCP) DNA Sequence | ATGGCTTCTAACTTTACTCAGTTCGTTCTCGTCGA CAATGGCGGAACTGGCGACGTGACTGTCGCCCCA AGCAACTTCGCTAACGGGATCGCTGAATGGATCA GCTCTAACTCGCGTTCACAGGCTTACAAAGTAACC TGTAGCGTTCGTCAGAGCTCTGCGCAGAATCGCA AATACACCATCAAAGTCGAGGTGCCTAAAGGCGC |
| | | CTGGCGTTCGTACTTAAATATGGAACTAACCATTC CAATTTTCGCCACGAATTCCGACTGCGAGCTTATT GTTAAGGCAATGCAAGGTCTCCTAAAAGATGGAA ACCCGATTCCCTCAGCAATCGCAGCAAACTCCGG CATCTAC |
| SEQ ID NO:2 | MS2 coat protein (MCP) Amino Acid Sequence | MASNFTQFVLVDNGGTGDVTVAPSNFANGIAEWISS NSRSQAYKVTCSVRQSSAQNRKYTIKVEVPKGAWR SYLNMELTIPIFATNSDCELIVKAMQGLLKDGNPIPS AIAANSGIY |
| SEQ ID NO:3 | PP7 coat protein (PCP) DNA Sequence | tccaaaacaatagtcctctccgtaggggaggcaacacggactttgaccgaaatcc agtcaaccgctgaccgacaaatctttgaagagaaagtagggcctcttgtgggccg actgcgcttgactgcaagcttgcgacaaaacggcgcaaagactgcctatagggtc aaccttaaactcgaccaagccgacgtggtcgatagcggtctccctaaggttcggta tacgcaggtctggagtcatgacgtaacaatcgtagcaaacagcacagaagcctcc cgaaaaagcctctacgatctgacgaaatccttggtggctacgtcacaggtggaag acctcgttgtcaaccttgtacctctgggtcga |
| SEQ ID NO:3 | PP7 coat protein (PCP) Amino Acid Sequence | SKTIVLSVGEATRTLTEIQSTADRQIFEEKVGPLVGR LRLTASLRQNGAKTAYRVNLKLDQADVVDSGLPKV RYTQVWSHDVTIVANSTEASRKSLYDLTKSLVATSQ VEDLVVNLVPLGR |
| SEQ ID NO:5 | lambda N RNA-binding domain (positions (1-22) DNA Sequence | ATGGATGCACAAACACGCCGCCGCGAACGTCGCG CAGAGAAACAGGCTCAATGGAAAGCAGCAAAT |
| SEQ ID NO:6 | lambda N RNA-binding domain (positions (1-22) Amino Acid Sequence | MDAQTRRRERRAEKQAQWKAAN |
| SEQ ID NO:7 | Com Protein DNA Sequence | atgaaatcaattcgctgtaaaaactgcaacaaactgttatttaaggcggattcctttga tcacattgaaatcaggtgtccgcgttgcaaacgtcacatcataatgctgaatgcctg cgagcatcccacggagaaacattgtgggaaaagagaaaaaatcacgcattctgac gaaaccgtgcgttattgagtat |
| SEQ ID NO:8 | Com Protein Amino Acid Sequence (GenBank AAF01130.1) | MKSIRCKNCNKLLFKADSFDHIEIRCPRCKRHIIMLN ACEHPTEKHCGKREKITHSDETVRY |
| SEQ ID NO:9 | MS2 aptamer sequence (RNA) | ACAUGAGGAUCACCCAUGU |
| SEQ ID NO:10 | MS2 aptamer sequence (DNA) | ACATGAGGATCACCCATGT |
| SEQ **ID** NO:11 | PP7 aptamer sequence (RNA) | GGAGCAGACGAUAUGGCGUCGCUCC |
| SEQ ID NO:12 | PP7 aptamer sequence (DNA) | GGAGCAGACGATATGGCGTCGCTCC |
| SEQ ID NO: 13 | Box-B; lambda N RNA-binding domain aptamer sequence (RNA) | GGGCCCUGAAGAAGGGCCC |
| SEQ ID NO: 14 | Box-B; lambda N RNA-binding domain aptamer sequence (DNA) | GGGCCCTGAAGAAGGGCCC |
| SEQ ID NO:15 | com aptamer RNA sequence | CUGAAUGCCUGCGAGCAUC |
| SEQ ID NO:16 | com aptamer DNA sequence | CTGAATGCCTGCGAGCAT |
| SEQ ID NO:17 | human beta hemoglobin (HBB) 3' UTR (DNA) | gctcgctttcttgctgtccaatttctattaaaggttcctttgttccctaagtccaactactaa actgggggatattatgaagggccttgagcatctggattctgcctaataaaaaacattta ttttcattgc |
| SEQ ID NO:18 | human beta hemoglobin (HBB) 3' UTR (RNA) | gcucgcuuucuugcuguccaauuucuauuaaagguuccuuuguucccuaa guccaacuacuaaacugggggauauuaugaagggccuugagcaucuggauu cugccuaauaaaaaacauuuauuuucauugc |
| SEQ ID NO: 19 | Nucleic acid encoding ABE-SpCas9(D10A) fusion protein | atgaaacggacagccgacggaagcgagttcgagtcaccaaagaagaagcggaaa gtctctgaagtcgagtttagccacgagtattggatgaggcacgcactgaccctggca aagcgagcatgggatgaaagagaagtccccgtgggcgccgtgctggtgcacaac aatagagtgatcggagagggatggaacaggccaatcggccgccacgaccctacc gcacacgcagagatcatggcactgaggcagggaggcctggtcatgcagaattacc gcctgatcgatgccaccctgtatgtgacactggagccatgcgtgatgtgcgcagga gcaatgatccacagcaggatcggaagagtggtgttcggagcacgggacgccaag accggcgcagcaggctccctgatggatgtgctgcaccaccccggcatgaaccacc gggtggagatcacagagggaatcctggcagacgagtgcgccgccctgctgagcg atttctttagaatgcggagacaggagatcaaggcccagaagaaggcacagagctcc accgactctggaggatctagcggaggatcctctggaagcgagacaccaggcacaa gcgagtccgccacaccagagagctccggcggctcctccggaggatcctctgaggt ggagttttcccacgagtactggatgagacatgccctgaccctggccaagagggcac gcgatgagagggaggtgcctgtgggagccgtgctggtgctgaacaatagagtgat cggcgagggctggaacagagccatcggcctgcacgacccaacagcccatgccga aattatggccctgagacagggcggcctggtcatgcagaactacagactgattgacg ccaccctgtacgtgacattcgagccttgcgtgatgtgcgccggcgccatgatccact ctaggatcggccgcgtggtgtttggcgtgaggaacgcaaaaaccggcgccgcag gctccctgatggacgtgctgcactaccccggcatgaatcaccgcgtcgaaattaccg agggaatcctggcagatgaatgtgccgccctgctgtgctatttctttcggatgcctag acaggtgttcaatgctcagaagaaggcccagagctccaccgactccggaggatcta gcggaggctcctctggctctgagacacctggcacaagcgagagcgcaacacctga aagcagcgggggcagcagcggggggtcagacaagaagtacagcatcggcctgg ccatcggcaccaactctgtgggctgggccgtgatcaccgacgagtacaaggtgcc cagcaagaaattcaaggtgctgggcaacaccgaccggcacagcatcaagaagaa cctgatcggagccctgctgttcgacagcggcgaaacagccgaggccacccggctg aagagaaccgccagaagaagatacaccagacggaagaaccggatctgctatctgc aagaGATCTTCAGCAACGAGATGGCCAAGGTGGACG ACAGCTTCTTCCACAGACTGGAAGAGTCCTTCCTG GTGGAAGAGGATAAGAAGCACGAGCGGCACCCCA TCTTCGGCAACATCGTGGACGAGGTGGCCTACCAC GAGAAGTACCCCACCATCTACCACCTGAGAAAGAA ACTGGTGGACAGCACCGACAAGGCCGACCTGCGG CTGATCTATCTGGCCCTGGCCCACATGATCAAGTTC CGGGGCCACTTCCTGATCGAGGGCGACCTGAACCC CGACAACAGCGACGTGGACAAGCTGTTCATCCAGC |
| | | TGGTGCAGACCTACAACCAGCTGTTCGAGGAAAAC CCCATCAACGCCAGCGGCGTGGACGCCAAGGCCAT CCTGTCTGCCAGACTGAGCAAGAGCAGACGGCTGG AAAATCTGATCGCCCAGCTGCCCGGCGAGAAGAA GAATGGCCTGTTCGGAAACCTGATTGCCCTGAGCC TGGGCCTGACCCCCAACTTCAAGAGCAACTTCGAC CTGGCCGAGGATGCCAAACTGCAGCTGAGCAAGG ACACCTACGACGACGACCTGGACAACCTGCTGGCC CAGATCGGCGACCAGTACGCCGACCTGTTTCTGGC CGCCAAGAACCTGTCCGACGCCATCCTGCTGAGCG ACATCCTGAGAGTGAACACCGAGATCACCAAGGCC CCCCTGAGCGCCTCTATGATCAAGAGATACGACGA GCACCACCAGGACCTGACCCTGCTGAAAGCTCTCG TGCGGCAGCAGCTGCCTGAGAAGTACAAAGAGATT TTCTTCGACCAGAGCAAGAACGGCTACGCCGGCTA CATTGACGGCGGAGCCAGCCAGGAAGAGTTCTACA AGTTCATCAAGCCCATCCTGGAAAAGATGGACGGC ACCGAGGAACTGCTCGTGAAGCTGAACAGAGAGG ACCTGCTGCGGAAGCAGCGGACCTTCGACAACGGC AGCATCCCCCACCAGATCCACCTGGGAGAGCTGCA CGCCATTCTGCGGCGGCAGGAAGATTTTTACCCAT TCCTGAAGGACAACCGGGAAAAGATCGAGAAGAT CCTGACCTTCCGCATCCCCTACTACGTGGGCCCTCT GGCCAGGGGAAACAGCAGATTCGCCTGGATGACC AGAAAGAGCGAGGAAACCATCACCCCCTGGAACT TCGAGGAAGTGGTGGACAAGGGCGCTTCCGCCCAG AGCTTCATCGAGCGGATGACCAACTTCGATAAGAA CCTGCCCAACGAGAAGGTGCTGCCCAAGCACAGCC TGCTGTACGAGTACTTCACCGTGTATAACGAGCTG ACCAAAGTGAAATACGTGACCGAGGGAATGAGAA AGCCCGCCTTCCTGAGCGGCGAGCAGAAAAAGGC CATCGTGGACCTGCTGTTCAAGACCAACCGGAAAG TGACCGTGAAGCAGCTGAAAGAGGACTACTTCAAG AAAATCGAGTGCTTCGACTCCGTGGAAATCTCCGG CGTGGAAGATCGGTTCAACGCCTCCCTGGGCACAT ACCACGATCTGCTGAAAATTATCAAGGACAAGGAC TTCCTGGACAATGAGGAAAACGAGGACATTCTGGA AGATATCGTGCTGACCCTGACACTGTTTGAGGACA GAGAGATGATCGAGGAACGGCTGAAAACCTATGC CCACCTGTTCGACGACAAAGTGATGAAGCAGCTGA AGCGGCGGAGATACACCGGCTGGGGCAGGCTGAG CCGGAAGCTGATCAACGGCATCCGGGACAAGCAG TCCGGCAAGACAATCCTGGATTTCCTGAAGTCCGA CGGCTTCGCCAACAGAAACTTCATGCAGCTGATCC ACGACGACAGCCTGACCTTTAAAGAGGACATCCAG AAAGCCCAGGTGTCCGGCCAGGGCGATAGCCTGCA CGAGCACATTGCCAATCTGGCCGGCAGCCCCGCCA |
| | | TTAAGAAGGGCATCCTGCAGACAGTGAAGGTGGTG GACGAGCTCGTGAAAGTGATGGGCCGGCACAAGC CCGAGAACATCGTGATCGAAATGGCCAGAGAGAA CCAGACCACCCAGAAGGGACAGAAGAACAGCCGC GAGAGAATGAAGCGGATCGAAGAGGGCATCAAAG AGCTGGGCAGCCAGATCCTGAAAGAACACCCCGTG GAAAACACCCAGCTGCAGAACGAGAAGCTGTACC TGTACTACCTGCAGAATGGGCGGGATATGTACGTG GACCAGGAACTGGACATCAACCGGCTGTCCGACTA CGATGTGGACCATATCGTGCCTCAGAGCTTTCTGA AGGACGACTCCATCGACAACAAGGTGCTGACCAG AAGCGACAAGAACCGGGGCAAGAGCGACAACGTG CCCTCCGAAGAGGTCGTGAAGAAGATGAAGAACT ACTGGCGGCAGCTGCTGAACGCCAAGCTGATTACC CAGAGAAAGTTCGACAATCTGACCAAGGCCGAGA GAGGCGGCCTGAGCGAACTGGATAAGGCCGGCTTC ATCAAGAGACAGCTGGTGGAAACCCGGCAGATCA CAAAGCACGTGGCACAGATCCTGGACTCCCGGATG AACACTAAGTACGACGAGAATGACAAGCTGATCC GGGAAGTGAAAGTGATCACCCTGAAGTCCAAGCTG GTGTCCGATTTCCGGAAGGATTTCCAGTTTTACAAA GTGCGCGAGATCAACAACTACCACCACGCCCACGA CGCCTACCTGAACGCCGTCGTGGGAACCGCCCTGA TCAAAAAGTACCCTAAGCTGGAAAGCGAGTTCGTG TACGGCGACTACAAGGTGTACGACGTGCGGAAGAT GATCGCCAAGAGCGAGCAGGAAATCGGCAAGGCT ACCGCCAAGTACTTCTTCTACAGCAACATCATGAA CTTTTTCAAGACCGAGATTACCCTGGCCAACGGCG AGATCCGGAAGCGGCCTCTGATCGAGACAAACGG CGAAACCGGGGAGATCGTGTGGGATAAGGGCCGG GATTTTGCCACCGTGCGGAAAGTGCTGAGCATGCC CCAAGTGAATATCGTGAAAAAGACCGAGGTGCAG ACAGGCGGCTTCAGCAAAGAGTCTATCCTGCCCAA GAGGAACAGCGATAAGCTGATCGCCAGAAAGAAG GACTGGGACCCTAAGAAGTACGGCGGCTTCGACAG CCCCACCGTGGCCTATTCTGTGCTGGTGGTGGCCA AAGTGGAAAAGGGCAAGTCCAAGAAACTGAAGAG TGTGAAAGAGCTGCTGGGGATCACCATCATGGAAA GAAGCAGCTTCGAGAAGAATCCCATCGACTTTCTG GAAGCCAAGGGCTACAAAGAAGTGAAAAAGGACC TGATCATCAAGCTGCCTAAGTACTCCCTGTTCGAG CTGGAAAACGGCCGGAAGAGAATGCTGGCCTCTGC CGGCGAACTGCAGAAGGGAAACGAACTGGCCCTG CCCTCCAAATATGTGAACTTCCTGTACCTGGCCAG CCACTATGAGAAGCTGAAGGGCTCCCCCGAGGATA ATGAGCAGAAACAGCTGTTTGTGGAACAGCACAAG CACTACCTGGACGAGATCATCGAGCAGATCAGCGA |
| | | GTTCTCCAAGAGAGTGATCCTGGCCGACGCTAATC TGGACAAAGTGCTGTCCGCCTACAACAAGCACCGG GATAAGCCCATCAGAGAGCAGGCCGAGAATATCA TCCACCTGTTTACCCTGACCAATCTGGGAGCCCCTG CCGCCTTCAAGTACTTTGACACCACCATCGACCGG AAGAGGTACACCAGCACCAAAGAGGTGCTGGACG CCACCCTGATCCACCAGAGCATCACCGGCCTGTAC GAGACACGGATCGACCTGTCTCAGCTGGGAGGCGA CAAAAGGCCGGCGGCCACGAAAAAGGCCGGccaggc aaaaaagaaaaagggatcctaa |
| SEQ ID NO: 20 | ABEMAX fusion protein comprising deaminase and spCas9 (D10A) | MKRTADGSEFESPKKKRKVSEVEFSHEYWMRHALTL AKRAWDEREVPVGAVLVHNNRVIGEGWNRPIGRHD PTAHAEIMALRQGGLVMQNYRLIDATLYVTLEPCVM CAGAMIHSRIGRVVFGARDAKTGAAGSLMDVLHHP GMNHRVEITEGILADECAALLSDFFRMRRQEIKAQKK AQSSTDSGGSSGGSSGSETPGTSESATPESSGGSSGGS SEVEFSHEYWMRHALTLAKRARDEREVPVGAVLVL NNRVIGEGWNRAIGLHDPTAHAEIMALRQGGLVMQ NYRLIDATLYVTFEPCVMCAGAMIHSRIGRVVFGVR NAKTGAAGSLMDVLHYPGMNHRVEITEGILADECAA LLCYFFRMPRQVFNAQKKAQSSTDSGGSSGGSSGSET PGTSESATPESSGGSSGGSDKKYSIGLAIGTNSVGWA VITDEYKVPSKKFKVLGNTDRHSIKKNLIGALLFDSG ETAEATRLKRTARRRYTRRKNRICYLQEIFSNEMAKV DDSFFHRLEESFLVEEDKKHERHPIFGNIVDEVAYHE KYPTIYHLRKKLVDSTDKADLRLIYLALAHMIKFRGH FLIEGDLNPDNSDVDKLFIQLVQTYNQLFEENPINASG VDAKAILSARLSKSRRLENLIAQLPGEKKNGLFGNLI ALSLGLTPNFKSNFDLAEDAKLQLSKDTYDDDLDNL LAQIGDQYADLFLAAKNLSDAILLSDILRVNTEITKAP LSASMIKRYDEHHQDLTLLKALVRQQLPEKYKEIFFD QSKNGYAGYIDGGASQEEFYKFIKPILEKMDGTEELL VKLNREDLLRKQRTFDNGSIPHQIHLGELHAILRRQE DFYPFLKDNREKIEKILTFRIPYYVGPLARGNSRFAW MTRKSEETITPWNFEEVVDKGASAQSFIERMTNFDKN LPNEKVLPKHSLLYEYFTVYNELTKVKYVTEGMRKP AFLSGEQKKAIVDLLFKTNRKVTVKQLKEDYFKKIEC FDSVEISGVEDRFNASLGTYHDLLKIIKDKDFLDNEEN EDILEDIVLTLTLFEDREMIEERLKTYAHLFDDKVMK QLKRRRYTGWGRLSRKLINGIRDKQSGKTILDFLKSD GFANRNFMQLIHDDSLTFKEDIQKAQVSGQGDSLHE HIANLAGSPAIKKGILQTVKVVDELVKVMGRHKPENI VIEMARENQTTQKGQKNSRERMKRIEEGIKELGSQIL KEHPVENTQLQNEKLYLYYLQNGRDMYVDQELDIN RLSDYDVDHIVPQSFLKDDSIDNKVLTRSDKNRGKSD NVPSEEVVKKMKNYWRQLLNAKLITQRKFDNLTKA ERGGLSELDKAGFIKRQLVETRQITKHVAQILDSRMN |
| | | TKYDENDKLIREVKVITLKSKLVSDFRKDFQFYKVRE INNYHHAHDAYLNAVVGTALIKKYPKLESEFVYGDY KVYDVRKMIAKSEQEIGKATAKYFFYSNIMNFFKTEI TLANGEIRKRPLIETNGETGEIVWDKGRDFATVRKVL SMPQVNIVKKTEVQTGGFSKESILPKRNSDKLIARKK DWDPKKYGGFDSPTVAYSVLVVAKVEKGKSKKLKS VKELLGITIMERSSFEKNPIDFLEAKGYKEVKKDLIIKL PKYSLFELENGRKRMLASAGELQKGNELALPSKYVN FLYLASHYEKLKGSPEDNEQKQLFVEQHKHYLDEIIE QISEFSKRVILADANLDKVLSAYNKHRDKPIREQAENI IHLFTLTNLGAPAAFKYFDTTIDRKRYTSTKEVLDATL IHQSITGLYETRIDLSQLGGDKRPAATKKAGQAKKKK GS* |
| SEQ ID NO: 21 | Nucleic acid sequence encoding spCas9 (D10A) | ATGGACTATAAGGACCACGACGGAGACTACAAGG ATCATGATATTGATTACAAAGACGATGACGATAAG ATGGCCCCAAAGAAGAAGCGGAAGGTCGGTATCC ACGGAGTCCCAGCAGCCGACAAGAAGTACAGCAT CGGCCTGGACATCGGCACCAACTCTGTGGGCTGGG CCGTGATCACCGACGAGTACAAGGTGCCCAGCAAG AAATTCAAGGTGCTGGGCAACACCGACCGGCACA GCATCAAGAAGAACCTGATCGGAGCCCTGCTGTTC GACAGCGGCGAAACAGCCGAGGCCACCCGGCTGA AGAGAACCGCCAGAAGAAGATACACCAGACGGAA GAACCGGATCTGCTATCTGCAAGAGATCTTCAGCA ACGAGATGGCCAAGGTGGACGACAGCTTCTTCCAC AGACTGGAAGAGTCCTTCCTGGTGGAAGAGGATAA GAAGCACGAGCGGCACCCCATCTTCGGCAACATCG TGGACGAGGTGGCCTACCACGAGAAGTACCCCACC ATCTACCACCTGAGAAAGAAACTGGTGGACAGCAC CGACAAGGCCGACCTGCGGCTGATCTATCTGGCCC TGGCCCACATGATCAAGTTCCGGGGCCACTTCCTG ATCGAGGGCGACCTGAACCCCGACAACAGCGACG TGGACAAGCTGTTCATCCAGCTGGTGCAGACCTAC AACCAGCTGTTCGAGGAAAACCCCATCAACGCCAG CGGCGTGGACGCCAAGGCCATCCTGTCTGCCAGAC TGAGCAAGAGCAGACGGCTGGAAAATCTGATCGC CCAGCTGCCCGGCGAGAAGAAGAATGGCCTGTTCG GAAACCTGATTGCCCTGAGCCTGGGCCTGACCCCC AACTTCAAGAGCAACTTCGACCTGGCCGAGGATGC CAAACTGCAGCTGAGCAAGGACACCTACGACGAC GACCTGGACAACCTGCTGGCCCAGATCGGCGACCA GTACGCCGACCTGTTTCTGGCCGCCAAGAACCTGT CCGACGCCATCCTGCTGAGCGACATCCTGAGAGTG AACACCGAGATCACCAAGGCCCCCCTGAGCGCCTC TATGATCAAGAGATACGACGAGCACCACCAGGAC CTGACCCTGCTGAAAGCTCTCGTGCGGCAGCAGCT GCCTGAGAAGTACAAAGAGATTTTCTTCGACCAGA GCAAGAACGGCTACGCCGGCTACATTGACGGCGG AGCCAGCCAGGAAGAGTTCTACAAGTTCATCAAGC |
| | | CCATCCTGGAAAAGATGGACGGCACCGAGGAACT GCTCGTGAAGCTGAACAGAGAGGACCTGCTGCGG AAGCAGCGGACCTTCGACAACGGCAGCATCCCCCA CCAGATCCACCTGGGAGAGCTGCACGCCATTCTGC GGCGGCAGGAAGATTTTTACCCATTCCTGAAGGAC AACCGGGAAAAGATCGAGAAGATCCTGACCTTCCG CATCCCCTACTACGTGGGCCCTCTGGCCAGGGGAA ACAGCAGATTCGCCTGGATGACCAGAAAGAGCGA GGAAACCATCACCCCCTGGAACTTCGAGGAAGTGG TGGACAAGGGCGCTTCCGCCCAGAGCTTCATCGAG CGGATGACCAACTTCGATAAGAACCTGCCCAACGA GAAGGTGCTGCCCAAGCACAGCCTGCTGTACGAGT ACTTCACCGTGTATAACGAGCTGACCAAAGTGAAA TACGTGACCGAGGGAATGAGAAAGCCCGCCTTCCT GAGCGGCGAGCAGAAAAAGGCCATCGTGGACCTG CTGTTCAAGACCAACCGGAAAGTGACCGTGAAGCA GCTGAAAGAGGACTACTTCAAGAAAATCGAGTGCT TCGACTCCGTGGAAATCTCCGGCGTGGAAGATCGG TTCAACGCCTCCCTGGGCACATACCACGATCTGCT GAAAATTATCAAGGACAAGGACTTCCTGGACAATG AGGAAAACGAGGACATTCTGGAAGATATCGTGCTG ACCCTGACACTGTTTGAGGACAGAGAGATGATCGA GGAACGGCTGAAAACCTATGCCCACCTGTTCGACG ACAAAGTGATGAAGCAGCTGAAGCGGCGGAGATA CACCGGCTGGGGCAGGCTGAGCCGGAAGCTGATC AACGGCATCCGGGACAAGCAGTCCGGCAAGACAA TCCTGGATTTCCTGAAGTCCGACGGCTTCGCCAAC AGAAACTTCATGCAGCTGATCCACGACGACAGCCT GACCTTTAAAGAGGACATCCAGAAAGCCCAGGTGT CCGGCCAGGGCGATAGCCTGCACGAGCACATTGCC AATCTGGCCGGCAGCCCCGCCATTAAGAAGGGCAT CCTGCAGACAGTGAAGGTGGTGGACGAGCTCGTGA AAGTGATGGGCCGGCACAAGCCCGAGAACATCGT GATCGAAATGGCCAGAGAGAACCAGACCACCCAG AAGGGACAGAAGAACAGCCGCGAGAGAATGAAGC GGATCGAAGAGGGCATCAAAGAGCTGGGCAGCCA GATCCTGAAAGAACACCCCGTGGAAAACACCCAG CTGCAGAACGAGAAGCTGTACCTGTACTACCTGCA GAATGGGCGGGATATGTACGTGGACCAGGAACTG GACATCAACCGGCTGTCCGACTACGATGTGGACCA TATCGTGCCTCAGAGCTTTCTGAAGGACGACTCCA TCGACAACAAGGTGCTGACCAGAAGCGACAAGAA CCGGGGCAAGAGCGACAACGTGCCCTCCGAAGAG GTCGTGAAGAAGATGAAGAACTACTGGCGGCAGCT GCTGAACGCCAAGCTGATTACCCAGAGAAAGTTCG ACAATCTGACCAAGGCCGAGAGAGGCGGCCTGAG CGAACTGGATAAGGCCGGCTTCATCAAGAGACAGC TGGTGGAAACCCGGCAGATCACAAAGCACGTGGC ACAGATCCTGGACTCCCGGATGAACACTAAGTACG ACGAGAATGACAAGCTGATCCGGGAAGTGAAAGT GATCACCCTGAAGTCCAAGCTGGTGTCCGATTTCC |
| | | GGAAGGATTTCCAGTTTTACAAAGTGCGCGAGATC AACAACTACCACCACGCCCACGACGCCTACCTGAA CGCCGTCGTGGGAACCGCCCTGATCAAAAAGTACC CTAAGCTGGAAAGCGAGTTCGTGTACGGCGACTAC AAGGTGTACGACGTGCGGAAGATGATCGCCAAGA GCGAGCAGGAAATCGGCAAGGCTACCGCCAAGTA CTTCTTCTACAGCAACATCATGAACTTTTTCAAGAC CGAGATTACCCTGGCCAACGGCGAGATCCGGAAGC GGCCTCTGATCGAGACAAACGGCGAAACCGGGGA GATCGTGTGGGATAAGGGCCGGGATTTTGCCACCG TGCGGAAAGTGCTGAGCATGCCCCAAGTGAATATC GTGAAAAAGACCGAGGTGCAGACAGGCGGCTTCA GCAAAGAGTCTATCCTGCCCAAGAGGAACAGCGAT AAGCTGATCGCCAGAAAGAAGGACTGGGACCCTA AGAAGTACGGCGGCTTCGACAGCCCCACCGTGGCC TATTCTGTGCTGGTGGTGGCCAAAGTGGAAAAGGG CAAGTCCAAGAAACTGAAGAGTGTGAAAGAGCTG CTGGGGATCACCATCATGGAAAGAAGCAGCTTCGA GAAGAATCCCATCGACTTTCTGGAAGCCAAGGGCT ACAAAGAAGTGAAAAAGGACCTGATCATCAAGCT GCCTAAGTACTCCCTGTTCGAGCTGGAAAACGGCC GGAAGAGAATGCTGGCCTCTGCCGGCGAACTGCAG AAGGGAAACGAACTGGCCCTGCCCTCCAAATATGT GAACTTCCTGTACCTGGCCAGCCACTATGAGAAGC TGAAGGGCTCCCCCGAGGATAATGAGCAGAAACA GCTGTTTGTGGAACAGCACAAGCACTACCTGGACG AGATCATCGAGCAGATCAGCGAGTTCTCCAAGAGA GTGATCCTGGCCGACGCTAATCTGGACAAAGTGCT GTCCGCCTACAACAAGCACCGGGATAAGCCCATCA GAGAGCAGGCCGAGAATATCATCCACCTGTTTACC CTGACCAATCTGGGAGCCCCTGCCGCCTTCAAGTA CTTTGACACCACCATCGACCGGAAGAGGTACACCA GCACCAAAGAGGTGCTGGACGCCACCCTGATCCAC CAGAGCATCACCGGCCTGTACGAGACACGGATCGA CCTGTCTCAGCTGGGAGGCGACAAAAGGCCGGCG GCCACGAAAAAGGCCGGccaggcaaaaaagaaaaagggatccta a |
| SEQ ID NO: 22 | spCas9 (D10A) protein sequence | DKKYSIGLAIGTNSVGWAVITDEYKVPSKKFKVLGNT DRHSIKKNLIGALLFDSGETAEATRLKRTARRRYTRR KNRICYLQEIFSNEMAKVDDSFFHRLEESFLVEEDKK HERHPIFGNIVDEVAYHEKYPTIYHLRKKLVDSTDKA DLRLIYLALAHMIKFRGHFLIEGDLNPDNSDVDKLFIQ LVQTYNQLFEENPINASGVDAKAILSARLSKSRRLEN LIAQLPGEKKNGLFGNLIALSLGLTPNFKSNFDLAED AKLQLSKDTYDDDLDNLLAQIGDQYADLFLAAKNLS DAILLSDILRVNTEITKAPLSASMIKRYDEHHQDLTLL KALVRQQLPEKYKEIFFDQSKNGYAGYIDGGASQEEF YKFIKPILEKMDGTEELLVKLNREDLLRKQRTFDNGSI PHQIHLGELHAILRRQEDFYPFLKDNREKIEKILTFRIP YYVGPLARGNSRFAWMTRKSEETITPWNFEEVVDKG |
| | | ASAQSFIERMTNFDKNLPNEKVLPKHSLLYEYFTVYN ELTKVKYVTEGMRKPAFLSGEQKKAIVDLLFKTNRK VTVKQLKEDYFKKIECFDSVEISGVEDRFNASLGTYH DLLKIIKDKDFLDNEENEDILEDIVLTLTLFEDREMIEE RLKTYAHLFDDKVMKQLKRRRYTGWGRLSRKLINGI RDKQSGKTILDFLKSDGFANRNFMQLIHDDSLTFKED IQKAQVSGQGDSLHEHIANLAGSPAIKKGILQTVKVV DELVKVMGRHKPENIVIEMARENQTTQKGQKNSRER MKRIEEGIKELGSQILKEHPVENTQLQNEKLYLYYLQ NGRDMYVDQELDINRLSDYDVDHIVPQSFLKDDSID NKVLTRSDKNRGKSDNVPSEEVVKKMKNYWRQLLN AKLITQRKFDNLTKAERGGLSELDKAGFIKRQLVETR QITKHVAQILDSRMNTKYDENDKLIREVKVITLKSKL VSDFRKDFQFYKVREINNYHHAHDAYLNAVVGTALI KKYPKLESEFVYGDYKVYDVRKMIAKSEQEIGKATA KYFFYSNIMNFFKTEITLANGEIRKRPLIETNGETGEIV WDKGRDFATVRKVLSMPQVNIVKKTEVQTGGFSKES ILPKRNSDKLIARKKDWDPKKYGGFDSPTVAYSVLV VAKVEKGKSKKLKSVKELLGITIMERSSFEKNPIDFLE AKGYKEVKKDLIIKLPKYSLFELENGRKRMLASAGEL QKGNELALPSKYVNFLYLASHYEKLKGSPEDNEQKQ LFVEQHKHYLDEIIEQISEFSKRVILADANLDKVLSAY NKHRDKPIREQAENIIHLFTLTNLGAPAAFKYFDTTID RKRYTSTKEVLDATLIHQSITGLYETRIDLSQLGGD |
| SEQ ID NO: 23 | SpCas9 nucleic acid sequence | atggactataaggaccacgacggagactacaaggatcatgatattgattacaaagac gatgacgataagatggccccaaagaagaagcggaaggtcggtatccacggagtcc cagcagccgacaagaagtacagcatcggcctggacatcggcaccaactctgtggg ctgggccgtgatcaccgacgagtacaaggtgcccagcaagaaattcaaggtgctg ggcaacaccgaccggcacagcatcaagaagaacctgatcggagccctgctgttcg acagcggcgaaacagccgaggccacccggctgaagagaaccgccagaagaag atacaccagacggaagaaccggatctgctatctgcaagagatcttcagcaacgaga tggccaaggtggacgacagcttcttccacagactggaagagtccttcctggtggaa gaggataagaagcacgagcggcaccccatcttcggcaacatcgtggacgaggtg gcctaccacgagaagtaccccaccatctaccacctgagaaagaaactggtggaca gcaccgacaaggccgacctgcggctgatctatctggccctggcccacatgatcaag ttccggggccacttcctgatcgagggcgacctgaaccccgacaacagcgacgtgg acaagctgttcatccagctggtgcagacctacaaccagctgttcgaggaaaacccc atcaacgccagcggcgtggacgccaaggccatcctgtctgccagactgagcaaga gcagacggctggaaaatctgatcgcccagctgcccggcgagaagaagaatggcct gttcggaaacctgattgccctgagcctgggcctgacccccaacttcaagagcaactt cgacctggccgaggatgccaaactgcagctgagcaaggacacctacgacgacga cctggacaacctgctggcccagatcggcgaccagtacgccgacctgtttctggccg ccaagaacctgtccgacgccatcctgctgagcgacatcctgagagtgaacaccga gatcaccaaggcccccctgagcgcctctatgatcaagagatacgacgagcaccac caggacctgaccctgctgaaagctctcgtgcggcagcagctgcctgagaagtacaa agagattttcttcgaccagagcaagaacggctacgccggctacattgacggcggag ccagccaggaagagttctacaagttcatcaagcccatcctggaaaagatggacggc accgaggaactgctcgtgaagctgaacagagaggacctgctgcggaagcagcgg accttcgacaacggcagcatcccccaccagatccacctgggagagctgcacgcca ttctgcggcggcaggaagatttttacccattcctgaaggacaaccgggaaaagatcg |
| | | agaagatcctgaccttccgcatcccctactacgtgggccctctggccaggggaaac agcagattcgcctggatgaccagaaagagcgaggaaaccatcaccccctggaact tcgaggaagtggtggacaagggcgcttccgcccagagcttcatcgagcggatgac caacttcgataagaacctgcccaacgagaaggtgctgcccaagcacagcctgctgt acgagtacttcaccgtgtataacgagctgaccaaagtgaaatacgtgaccgaggga atgagaaagcccgccttcctgagcggcgagcagaaaaaggccatcgtggacctgc tgttcaagaccaaccggaaagtgaccgtgaagcagctgaaagaggactacttcaag aaaatcgagtgcttcgactccgtggaaatctccggcgtggaagatcggttcaacgcc tccctgggcacataccacgatctgctgaaaattatcaaggacaaggacttcctggac aatgaggaaaacgaggacattctggaagatatcgtgctgaccctgacactgtttgag gacagagagatgatcgaggaacggctgaaaacctatgcccacctgttcgacgaca aagtgatgaagcagctgaagcggcggagatacaccggctggggcaggctgagcc ggaagctgatcaacggcatccgggacaagcagtccggcaagacaatcctggatttc ctgaagtccgacggcttcgccaacagaaacttcatgcagctgatccacgacgacag cctgacctttaaagaggacatccagaaagcccaggtgtccggccagggcgatagc ctgcacgagcacattgccaatctggccggcagccccgccattaagaagggcatcct gcagacagtgaaggtggtggacgagctcgtgaaagtgatgggccggcacaagcc cgagaacatcgtgatcgaaatggccagagagaaccagaccacccagaagggaca gaagaacagccgcgagagaatgaagcggatcgaagagggcatcaaagagctgg gcagccagatcctgaaagaacaccccgtggaaaacacccagctgcagaacgaga agctgtacctgtactacctgcagaatgggcgggatatgtacgtggaccaggaactg gacatcaaccggctgtccgactacgatgtggaccatatcgtgcctcagagctttctga aggacgactccatcgacaacaaggtgctgaccagaagcgacaagaaccggggca agagcgacaacgtgccctccgaagaggtcgtgaagaagatgaagaactactggcg gcagctgctgaacgccaagctgattacccagagaaagttcgacaatctgaccaagg ccgagagaggcggcctgagcgaactggataaggccggcttcatcaagagacagc tggtggaaacccggcagatcacaaagcacgtggcacagatcctggactcccggat gaacactaagtacgacgagaatgacaagctgatccgggaagtgaaagtgatcacc ctgaagtccaagctggtgtccgatttccggaaggatttccagttttacaaagtgcgcg agatcaacaactaccaccacgcccacgacgcctacctgaacgccgtcgtgggaac cgccctgatcaaaaagtaccctaagctggaaagcgagttcgtgtacggcgactaca aggtgtacgacgtgcggaagatgatcgccaagagcgagcaggaaatcggcaagg ctaccgccaagtacttcttctacagcaacatcatgaactttttcaagaccgagattacc ctggccaacggcgagatccggaagcggcctctgatcgagacaaacggcgaaacc ggggagatcgtgtgggataagggccgggattttgccaccgtgcggaaagtgctga gcatgccccaagtgaatatcgtgaaaaagaccgaggtgcagacaggcggcttcag caaagagtctatcctgcccaagaggaacagcgataagctgatcgccagaaagaag gactgggaccctaagaagtacggcggcttcgacagccccaccgtggcctattctgt gctggtggtggccaaagtggaaaagggcaagtccaagaaactgaagagtgtgaaa gagctgctggggatcaccatcatggaaagaagcagcttcgagaagaatcccatcga ctttctggaagccaagggctacaaagaagtgaaaaaggacctgatcatcaagctgc ctaagtactccctgttcgagctggaaaacggccggaagagaatgctggcctctgcc ggcgaactgcagaagggaaacgaactggccctgccctccaaatatgtgaacttcct gtacctggccagccactatgagaagctgaagggctcccccgaggataatgagcag aaacagctgtttgtggaacagcacaagcactacctggacgagatcatcgagcagat cagcgagttctccaagagagtgatcctggccgacgctaatctggacaaagtgctgtc cgcctacaacaagcaccgggataagcccatcagagagcaggccgagaatatcatc cacctgtttaccctgaccaatctgggagcccctgccgccttcaagtactttgacacca ccatcgaccggaagaggtacaccagcaccaaagaggtgctggacgccaccctgat ccaccagagcatcaccggcctgtacgagacacggatcgacctgtctcagctggga |
| | | ggcgacaaaaggccggcggccacgaaaaaggccggccaggcaaaaaagaaaa agtaa |
| SEQ ID NO: 24 | SpCas9 | MDYKDHDGDYKDHDIDYKDDDDKMAPKKKRKVGI HGVPAADKKYSIGLDIGTNSVGWAVITDEYKVPSKK FKVLGNTDRHSIKKNLIGALLFDSGETAEATRLKRTA RRRYTRRKNRICYLQEIFSNEMAKVDDSFFHRLEESF LVEEDKKHERHPIFGNIVDEVAYHEKYPTIYHLRKKL VDSTDKADLRLIYLALAHMIKFRGHFLIEGDLNPDNS DVDKLFIQLVQTYNQLFEENPINASGVDAKAILSARL SKSRRLENLIAQLPGEKKNGLFGNLIALSLGLTPNFKS NFDLAEDAKLQLSKDTYDDDLDNLLAQIGDQYADLF LAAKNLSDAILLSDILRVNTEITKAPLSASMIKRYDEH HQDLTLLKALVRQQLPEKYKEIFFDQSKNGYAGYID GGASQEEFYKFIKPILEKMDGTEELLVKLNREDLLRK QRTFDNGSIPHQIHLGELHAILRRQEDFYPFLKDNREK IEKILTFRIPYYVGPLARGNSRFAWMTRKSEETITPWN FEEVVDKGASAQSFIERMTNFDKNLPNEKVLPKHSLL YEYFTVYNELTKVKYVTEGMRKPAFLSGEQKKAIVD LLFKTNRKVTVKQLKEDYFKKIECFDSVEISGVEDRF NASLGTYHDLLKIIKDKDFLDNEENEDILEDIVLTLTL FEDREMIEERLKTYAHLFDDKVMKQLKRRRYTGWG RLSRKLINGIRDKQSGKTILDFLKSDGFANRNFMQLIH DDSLTFKEDIQKAQVSGQGDSLHEHIANLAGSPAIKK GILQTVKVVDELVKVMGRHKPENIVIEMARENQTTQ KGQKNSRERMKRIEEGIKELGSQILKEHPVENTQLQN EKLYLYYLQNGRDMYVDQELDINRLSDYDVDHIVPQ SFLKDDSIDNKVLTRSDKNRGKSDNVPSEEVVKKMK NYWRQLLNAKLITQRKFDNLTKAERGGLSELDKAGF IKRQLVETRQITKHVAQILDSRMNTKYDENDKLIREV KVITLKSKLVSDFRKDFQFYKVREINNYHHAHDAYL NAVVGTALIKKYPKLESEFVYGDYKVYDVRKMIAKS EQEIGKATAKYFFYSNIMNFFKTEITLANGEIRKRPLIE TNGETGEIVWDKGRDFATVRKVLSMPQVNIVKKTEV QTGGFSKESILPKRNSDKLIARKKDWDPKKYGGFDSP TVAYSVLVVAKVEKGKSKKLKSVKELLGITIMERSSF EKNPIDFLEAKGYKEVKKDLIIKLPKYSLFELENGRKR MLASAGELQKGNELALPSKYVNFLYLASHYEKLKGS PEDNEQKQLFVEQHKHYLDEIIEQISEFSKRVILADAN LDKVLSAYNKHRDKPIREQAENIIHLFTLTNLGAPAA FKYFDTTIDRKRYTSTKEVLDATLIHQSITGLYETRID LSQLGGDKRPAATKKAGQAKKKK* |
| SEQ ID NO: 25 | SaCas9 nucleic acid sequence | ATGGCCCCAAAGAAGAAGCGGAAGGTCGGTATCC ACGGAGTCCCAGCAGCCAAGCGGAACTACATCCTG GGCCTGGACATCGGCATCACCAGCGTGGGCTACGG CATCATCGACTACGAGACACGGGACGTGATCGATG CCGGCGTGCGGCTGTTCAAAGAGGCCAACGTGGAA AACAACGAGGGCAGGCGGAGCAAGAGAGGCGCCA GAAGGCTGAAGCGGCGGAGGCGGCATAGAATCCA GAGAGTGAAGAAGCTGCTGTTCGACTACAACCTGC |
| | | TGACCGACCACAGCGAGCTGAGCGGCATCAACCCC TACGAGGCCAGAGTGAAGGGCCTGAGCCAGAAGC TGAGCGAGGAAGAGTTCTCTGCCGCCCTGCTGCAC CTGGCCAAGAGAAGAGGCGTGCACAACGTGAACG AGGTGGAAGAGGACACCGGCAACGAGCTGTCCAC CAAAGAGCAGATCAGCCGGAACAGCAAGGCCCTG GAAGAGAAATACGTGGCCGAACTGCAGCTGGAAC GGCTGAAGAAAGACGGCGAAGTGCGGGGCAGCAT CAACAGATTCAAGACCAGCGACTACGTGAAAGAA GCCAAACAGCTGCTGAAGGTGCAGAAGGCCTACC ACCAGCTGGACCAGAGCTTCATCGACACCTACATC GACCTGCTGGAAACCCGGCGGACCTACTATGAGGG ACCTGGCGAGGGCAGCCCCTTCGGCTGGAAGGACA TCAAAGAATGGTACGAGATGCTGATGGGCCACTGC ACCTACTTCCCCGAGGAACTGCGGAGCGTGAAGTA CGCCTACAACGCCGACCTGTACAACGCCCTGAACG ACCTGAACAATCTCGTGATCACCAGGGACGAGAAC GAGAAGCTGGAATATTACGAGAAGTTCCAGATCAT CGAGAACGTGTTCAAGCAGAAGAAGAAGCCCACC CTGAAGCAGATCGCCAAAGAAATCCTCGTGAACGA AGAGGATATTAAGGGCTACAGAGTGACCAGCACC GGCAAGCCCGAGTTCACCAACCTGAAGGTGTACCA CGACATCAAGGACATTACCGCCCGGAAAGAGATTA TTGAGAACGCCGAGCTGCTGGATCAGATTGCCAAG ATCCTGACCATCTACCAGAGCAGCGAGGACATCCA GGAAGAACTGACCAATCTGAACTCCGAGCTGACCC AGGAAGAGATCGAGCAGATCTCTAATCTGAAGGGC TATACCGGCACCCACAACCTGAGCCTGAAGGCCAT CAACCTGATCCTGGACGAGCTGTGGCACACCAACG ACAACCAGATCGCTATCTTCAACCGGCTGAAGCTG GTGCCCAAGAAGGTGGACCTGTCCCAGCAGAAAG AGATCCCCACCACCCTGGTGGACGACTTCATCCTG AGCCCCGTCGTGAAGAGAAGCTTCATCCAGAGCAT CAAAGTGATCAACGCCATCATCAAGAAGTACGGCC TGCCCAACGACATCATTATCGAGCTGGCCCGCGAG AAGAACTCCAAGGACGCCCAGAAAATGATCAACG AGATGCAGAAGCGGAACCGGCAGACCAACGAGCG GATCGAGGAAATCATCCGGACCACCGGCAAAGAG AACGCCAAGTACCTGATCGAGAAGATCAAGCTGCA CGACATGCAGGAAGGCAAGTGCCTGTACAGCCTGG AAGCCATCCCTCTGGAAGATCTGCTGAACAACCCC TTCAACTATGAGGTGGACCACATCATCCCCAGAAG CGTGTCCTTCGACAACAGCTTCAACAACAAGGTGC TCGTGAAGCAGGAAGAAAACAGCAAGAAGGGCAA CCGGACCCCATTCCAGTACCTGAGCAGCAGCGACA GCAAGATCAGCTACGAAACCTTCAAGAAGCACATC CTGAATCTGGCCAAGGGCAAGGGCAGAATCAGCA AGACCAAGAAAGAGTATCTGCTGGAAGAACGGGA CATCAACAGGTTCTCCGTGCAGAAAGACTTCATCA ACCGGAACCTGGTGGATACCAGATACGCCACCAGA GGCCTGATGAACCTGCTGCGGAGCTACTTCAGAGT |
| | | GAACAACCTGGACGTGAAAGTGAAGTCCATCAATG GCGGCTTCACCAGCTTTCTGCGGCGGAAGTGGAAG TTTAAGAAAGAGCGGAACAAGGGGTACAAGCACC ACGCCGAGGACGCCCTGATCATTGCCAACGCCGAT TTCATCTTCAAAGAGTGGAAGAAACTGGACAAGGC CAAAAAAGTGATGGAAAACCAGATGTTCGAGGAA AAGCAGGCCGAGAGCATGCCCGAGATCGAAACCG AGCAGGAGTACAAAGAGATCTTCATCACCCCCCAC CAGATCAAGCACATTAAGGACTTCAAGGACTACAA GTACAGCCACCGGGTGGACAAGAAGCCTAATAGA GAGCTGATTAACGACACCCTGTACTCCACCCGGAA GGACGACAAGGGCAACACCCTGATCGTGAACAAT CTGAACGGCCTGTACGACAAGGACAATGACAAGCT GAAAAAGCTGATCAACAAGAGCCCCGAAAAGCTG CTGATGTACCACCACGACCCCCAGACCTACCAGAA ACTGAAGCTGATTATGGAACAGTACGGCGACGAGA AGAATCCCCTGTACAAGTACTACGAGGAAACCGGG AACTACCTGACCAAGTACTCCAAAAAGGACAACG GCCCCGTGATCAAGAAGATTAAGTATTACGGCAAC AAACTGAACGCCCATCTGGACATCACCGACGACTA CCCCAACAGCAGAAACAAGGTCGTGAAGCTGTCCC TGAAGCCCTACAGATTCGACGTGTACCTGGACAAT GGCGTGTACAAGTTCGTGACCGTGAAGAATCTGGA TGTGATCAAAAAAGAAAACTACTACGAAGTGAATA GCAAGTGCTATGAGGAAGCTAAGAAGCTGAAGAA GATCAGCAACCAGGCCGAGTTTATCGCCTCCTTCT ACAACAACGATCTGATCAAGATCAACGGCGAGCTG TATAGAGTGATCGGCGTGAACAACGACCTGCTGAA CCGGATCGAAGTGAACATGATCGACATCACCTACC GCGAGTACCTGGAAAACATGAACGACAAGAGGCC CCCCAGGATCATTAAGACAATCGCCTCCAAGACCC AGAGCATTAAGAAGTACAGCACAGACATTCTGGGC AACCTGTATGAAGTGAAATCTAAGAAGCACCCTCA GATCATCAAAAAGGGCAAAAGGCCGGCGGCCACG AAAAAGGCCGGCCAGGCAAAAAAGAAAAAGGGAT CCTACCCATACGATGTTCCAGATTACGCTTACCCAT ACGATGTTCCAGATTACGCTTACCCATACGATGTTC CAGATTACGCTTAA |
| SEQ ID NO: 26 | SaCas9 | MAPKKKRKVGIHGVPAAKRNYILGLDIGITSVGYGII DYETRDVIDAGVRLFKEANVENNEGRRSKRGARRLK RRRRHRIQRVKKLLFDYNLLTDHSELSGINPYEARVK GLSQKLSEEEFSAALLHLAKRRGVHNVNEVEEDTGN ELSTKEQISRNSKALEEKYVAELQLERLKKDGEVRGS INRFKTSDYVKEAKQLLKVQKAYHQLDQSFIDTYIDL LETRRTYYEGPGEGSPFGWKDIKEWYEMLMGHCTY FPEELRSVKYAYNADLYNALNDLNNLVITRDENEKL EYYEKFQIIENVFKQKKKPTLKQIAKEILVNEEDIKGY RVTSTGKPEFTNLKVYHDIKDITARKEIIENAELLDQI AKILTIYQSSEDIQEELTNLNSELTQEEIEQISNLKGYT GTHNLSLKAINLILDELWHTNDNQIAIFNRLKLVPKK |
| | | VDLSQQKEIPTTLVDDFILSPVVKRSFIQSIKVINAIIKK YGLPNDIIIELAREKNSKDAQKMINEMQKRNRQTNER IEEIIRTTGKENAKYLIEKIKLHDMQEGKCLYSLEAIPL EDLLNNPFNYEVDHIIPRSVSFDNSFNNKVLVKQEEN SKKGNRTPFQYLSSSDSKISYETFKKHILNLAKGKGRI SKTKKEYLLEERDINRFSVQKDFINRNLVDTRYATRG LMNLLRSYFRVNNLDVKVKSINGGFTSFLRRKWKFK KERNKGYKHHAEDALIIANADFIFKEWKKLDKAKKV MENQMFEEKQAESMPEIETEQEYKEIFITPHQIKHIKD FKDYKYSHRVDKKPNRELINDTLYSTRKDDKGNTLI VNNLNGLYDKDNDKLKKLINKSPEKLLMYHHDPQT YQKLKLIMEQYGDEKNPLYKYYEETGNYLTKYSKK DNGPVIKKIKYYGNKLNAHLDITDDYPNSRNKVVKL SLKPYRFDVYLDNGVYKFVTVKNLDVIKKENYYEVN SKCYEEAKKLKKISNQAEFIASFYNNDLIKINGELYRV IGVNNDLLNRIEVNMIDITYREYLENMNDKRPPRIIKT IASKTQSIKKYSTDILGNLYEVKSKKHPQIIKKGKRPA ATKKAGQAKKKKGSYPYDVPDYAYPYDVPDYAYPY DVPDYA* |
| SEQ ID NO: 27 | spCAs9-ST2-COM sgRNA sequence for IL2RG | GCGCTTGCTCTTCATTCCCTGTTTGAGAGCTAGAAA TAGCAAGTTCAAATAAGGCTAGTCCGTTATCAACT TGGCTGAATGCCTGCGAGCATCCCACCCAAGTGGC ACCGAGTCGGTGC |
| SEQ ID NO: 28 | spCAs9-ST2-COM sgRNA sequence for HBB (sickle cell mutant) | GTAACGGCAGACTTCTCCTCGTTTGAGAGCTAGAA ATAGCAAGTTCAAATAAGGCTAGTCCGTTATCAAC TTGGCTGAATGCCTGCGAGCATCCCACCCAAGTGG CACCGAGTCGGTGC |
| SEQ ID NO: 29 | spCAs9-ST2-COM sgRNA sequence for DMD Exon 53 | ACTGTTGCCTCCGGTTCTGAGTTTGAGAGCTAGAA ATAGCAAGTTCAAATAAGGCTAGTCCGTTATCAAC TTGGCTGAATGCCTGCGAGCATCCCACCCAAGTGG CACCGAGTCGGTGC |
| SEQ ID NO: 30 | spCAs9-ST2-COM sgRNA sequence for CLCN5 | GAGGACAAGTCGTACAATGGGTTTGAGAGCTAGAA ATAGCAAGTTCAAATAAGGCTAGTCCGTTATCAAC TTGGCTGAATGCCTGCGAGCATCCCACCCAAGTGG CACCGAGTCGGTGC |
| SEQ ID NO: 31 | spCAs9-ST2-COM sgRNA sequence for TP53 | CCATTGTTCAATATCGTCCGGTTTGAGAGCTAGAA ATAGCAAGTTCAAATAAGGCTAGTCCGTTATCAAC TTGGCTGAATGCCTGCGAGCATCCCACCCAAGTGG CACCGAGTCGGTGC |
| SEQ ID NO: 32 | spCAs9-ST2-COM sgRNA sequence for GAPDH | AGCCCCAGCAAGAGCACAAGGTTTGAGAGCTAGA AATAGCAAGTTCAAATAAGGCTAGTCCGTTATCAA CTTGGCTGAATGCCTGCGAGCATCCCACCCAAGTG GCACCGAGTCGGTGC |
| SEQ ID NO: 33 | ABE site 5 St2-com sgRNA | GATGAGATAATGATGAGTCAGTTTGAGAGCTAGAA ATAGCAAGTTCAAATAAGGCTAGTCCGTTATCAAC TTGGCTGAATGCCTGCGAGCATCCCACCCAAGTGG CACCGAGTCGGTGC |
| SEQ ID NO: 34 | SaCas9 Tetra-com sgRNA sequence for DMD intron 50 | TATGTGGCTTTACCAAGGTCCgtttaagtactctgCTGAATG CCTGCGAGCATCCCACcagaatctacttaaacaaggcaaaatgccgt gtttatctcgtcaacttgttggcgagat |
| SEQ ID NO: 35 | SaCas9 Tetra-com sgRNA sequence for IL2RG | ACACAGACAGACTACACCCAgtttaagtactctgCTGAATG CCTGCGAGCATCCCACcagaatctacttaaacaaggcaaaatgccgt gtttatctcgtcaacttgttggcgagat |
| SEQ ID NO: 36 | CMV enhancer and promoter | cgttacataacttacggtaaatggcccgcctggctgaccgcccaacgacccccgcc cattgacgtcaataatgacgtatgttcccatagtaacgccaatagggactttccattga cgtcaatgggtggagtatttacggtaaactgcccacttggcagtacatcaagtgtatc atatgccaagtacgccccctattgacgtcaatgacggtaaatggcccgcctggcatt atgcccagtacatgaccttatgggactttcctacttggcagtacatctacgtattagtca tcgctattaccatggtgatgcggttttggcagtacatcaatgggcgtggatagcggttt gactcacggggatttccaagtctccaccccattgacgtcaatgggagtttgttttggca ccaaaatcaacgggactttccaaaatgtcgtaacaactccgccccattgacgcaaat gggcggtaggcgtgtacggtgggaggtctatataagcagagct |
| SEQ ID NO: 37 | 5'UTR | ggtttagtgaaccgtcagatccgctag |
| SEQ ID NO: 38 | linker | GGCGGTCACAACTCCGGAGGTGGAGGTGGACAGA GCCCAGGCCCGgcagcc |
| SEQ ID NO: 39 | Amino acid sequence encoded by SEQ ID NO: 38 | GGHNSGGGGGQSPGPAA |
| SEQ ID NO: 40 | linker | tccggtggaggtggatccatggcttctaactttactcagttcgttctcgtcgacaatgg cggaactggcGACGTG |
| SEQ ID NO: 41 | Amino acid sequence encoded by SEQ ID NO: 40 | SGGGGSMASNFTQFVLVDNGGTGDV |
| SEQ ID NO: 42 | Nucleic acid encoding CD63 | atggcggtggaaggaggaatgaaatgtgtgaagttcttgctctacgtcctcctgctgg ccttttgcgcctgtgcagtgggactgattgccgtgggtgtcggggcacagcttgtcct gagtcagaccataatccagggggctacccctggctctctgttgccagtggtcatcatc gcagtgggtgtcttcctcttcctggtggcttttgtgggctgctgcggggcctgcaagg agaactattgtcttatgatcacgtttgccatctttctgtctcttatcatgttggtggaggtg gccgcagccattgctggctatgtgtttagagataaggtgatgtcagagtttaataacaa cttccggcagcagatggagaattacccgaaaaataaccacactgcttcgatcctgga caggatgcaggcagattttaagtgctgtggggctgctaactacacagattgggagaa aatcccttccatgtcgaagaaccgagtccccgactcctgctgcattaatgttactgtgg |
| | | gctgtgggattaatttcaacgagaaggcgatccataaggagggctgtgtggagaag attgggggctggctgaggaaaaatgtgctggtggtagctgcagcagcccttggaatt gcttttgtcgaggttttgggaattgtctttgcctgctgcctcgtgaagagtatcagaagt ggctacgaggtgatgtag |
| SEQ ID NO: 43 | CD63 protein sequence | MAVEGGMKCVKFLLYVLLLAFCACAVGLIAVGVGA QLVLSQTIIQGATPGSLLPVVIIAVGVFLFLVAFVGCC GACKENYCLMITFAIFLSLIMLVEVAAAIAGYVFRDK VMSEFNNNFRQQMENYPKNNHTASILDRMQADFKC CGAANYTDWEKIPSMSKNRVPDSCCINVTVGCGINF NEKAIHKEGCVEKIGGWLRKNVLVVAAAALGIAFVE VLGIVFACCLVKSIRSGYEVM |
| SEQ ID NO: 44 | Nucleic acid encoding VSV-G | atgaagtgccttttgtacttagcctttttattcattggggtgaattgcaagttcaccatagtt tttccacacaaccaaaaaggaaactggaaaaatgttccttctaattaccattattgccc gtcaagctcagatttaaattggcataatgacttaataggcacagccatacaagtcaaa atgcccaagagtcacaaggctattcaagcagacggttggatgtgtcatgcttccaaat gggtcactacttgtgatttccgctggtatggaccgaagtatataacacagtccatccg atccttcactccatctgtagaacaatgcaaggaaagcattgaacaaacgaaacaagg aacttggctgaatccaggcttccctcctcaaagttgtggatatgcaactgtgacggat gccgaagcagtgattgtccaggtgactcctcaccatgtgctggttgatgaatacaca ggagaatgggttgattcacagttcatcaacggaaaatgcagcaattacatatgcccc actgtccataactctacaacctggcattctgactataaggtcaaagggctatgtgattct aacctcatttccatggacatcaccttcttctcagaggacggagagctatcatccctgg gaaaggagggcacagggttcagaagtaactactttgcttatgaaactggaggcaag gcctgcaaaatgcaatactgcaagcattggggagtcagactcccatcaggtgtctgg ttcgagatggctgataaggatctctttgctgcagccagattccctgaatgcccagaag ggtcaagtatctctgctccatctcagacctcagtggatgtaagtctaattcaggacgtt gagaggatcttggattattccctctgccaagaaacctggagcaaaatcagagcgggt cttccaatctctccagtggatctcagctatcttgctcctaaaaacccaggaaccggtcc tgctttcaccataatcaatggtaccctaaaatactttgagaccagatacatcagagtcg atattgctgctccaatcctctcaagaatggtcggaatgatcagtggaactaccacaga aagggaactgtgggatgactgggcaccatatgaagacgtggaaattggacccaatg gagttctgaggaccagttcaggatataagtttcctttatacatgattggacatggtatgtt ggactccgatcttcatcttagctcaaaggctcaggtgttcgaacatcctcacattcaag acgctgcttcgcaacttcctgatgatgagagtttattttttggtgatactgggctatccaa aaatccaatcgagcttgtagaaggttggttcagtagttggaaaagctctattgcctcttt tttctttatcatagggttaatcattggactattcttggttctccgagttggtatccatctttgc attaaattaaagcacaccaagaaaagacagatttatacagacatagagatgaaccga cttggaaagtaa |
| SEQ ID NO: 45 | VSV-G Protein | MKCLLYLAFLFIGVNCKFTIVFPHNQKGNWKNVPSN YHYCPSSSDLNWHNDLIGTAIQVKMPKSHKAIQADG WMCHASKWVTTCDFRWYGPKYITQSIRSFTPSVEQC KESIEQTKQGTWLNPGFPPQSCGYATVTDAEAVIVQV TPHHVLVDEYTGEWVDSQFINGKCSNYICPTVHNSTT WHSDYKVKGLCDSNLISMDITFFSEDGELSSLGKEGT GFRSNYFAYETGGKACKMQYCKHWGVRLPSGVWFE MADKDLFAAARFPECPEGSSISAPSQTSVDVSLIQDVE |
| | | RILDYSLCQETWSKIRAGLPISPVDLSYLAPKNPGTGP AFTIINGTLKYFETRYIRVDIAAPILSRMVGMISGTTTE RELWDDWAPYEDVEIGPNGVLRTSSGYKFPLYMIGH GMLDSDLHLSSKAQVFEHPHIQDAASQLPDDESLFFG DTGLSKNPIELVEGWFSSWKSSIASFFFIIGLIIGLFLVL RVGIHLCIKLKHTKKRQIYTDIEMNRLGK* |

## Claims

1. A plasmid system comprising:
(a) a first mammalian expression plasmid comprising a eukaryotic promoter operably linked to a nucleic acid sequence, wherein the nucleic acid sequence comprises:
(i) a nucleic acid sequence encoding a CRISPR-associated endonuclease and
a guide RNA (gRNA) coding sequence, wherein the gRNA coding sequence comprises at least one Com aptamer coding sequence; or
(ii) a nucleic acid sequence encoding a heterologous polypeptide and
at least one Com aptamer coding sequence; and
(b) a second mammalian expression plasmid comprising a eukaryotic promoter operably linked to a nucleic acid sequence encoding a fusion protein comprising CD63 and at least one Com aptamer binding protein, wherein the Com aptamer binding protein (ABP) binds to the at least one Com aptamer coding sequence of the first mammalian expression plasmid.

2. The plasmid system of claim 1 part (i), further comprising an envelope plasmid comprising a nucleic acid sequence encoding vesicular stomatis virus G (VSV G) protein; and/or wherein the CRISPR-associated endonuclease is a Cas9 protein, a Cpf1 protein or a derivative of either; and/or wherein the CRISPR-associated endonuclease is a catalytically impaired CRISPR-associated endonuclease; and optionally wherein the catalytically impaired CRISPR-associated endonuclease coding sequence encodes a Cas9 D10A protein, and/or wherein the nucleic acid sequence of the first mammalian expression plasmid encodes a nucleic acid sequence encoding an adenosine base pair editor (ABE), wherein the ABE is a fusion protein comprising an adenosine deaminase and the catalytically impaired CRISPR-associated endonuclease and optionally wherein the adenine base editor is ABE 7.10 or ABE8; and/or wherein the sgRNA coding sequence comprises at least one Com aptamer coding sequence inserted into the tetraloop or the ST2 loop of the sgRNA coding sequence.

3. The plasmid system of any one of claims 1 or 2, wherein the fusion protein comprises a first Com ABP fused to the N-terminus of CD63 and a second Com ABP fused to the C-terminus of CD63.

4. An extracellular vesicle comprising:
(a)
(i) a ribonucleotide protein (RNP) complex comprising a CRISPR-associated endonuclease and a gRNA comprising at least one Com aptamer coding sequence; or
(ii) a mRNA encoding a heterologous polypeptide and at least one Com aptamer coding sequence; and
and
(b) a fusion protein comprising CD63 and at least one Com aptamer binding protein (ABP), wherein the Com ABP binds to the at least one Com aptamer coding sequence.

5. The extracellular vesicle of claim 4, further comprising a VSV-G protein; and/or wherein the CRISPR-associated endonuclease is a Cas9 protein, a Cpf1 protein or a derivative of either; or wherein the CRISPR-associated endonuclease is a catalytically impaired CRISPR-associated endonuclease, optionally wherein the catalytically impaired CRISPR-associated endonuclease coding sequence encodes a Cas9 D10A protein, and optionally wherein the RNP comprises an adenine base pair editor (ABE), wherein the ABE is a fusion protein comprising an adenosine deaminase and the catalytically impaired CRISPR-associated endonuclease; optionally wherein the adenine base editor is ABE 7.10 or ABE8; and/or wherein the sgRNA coding sequence comprises at least one Com aptamer coding sequence inserted into the tetraloop or the ST2 loop of the sgRNA coding sequence.

6. The extracellular vesicle of any one of claims 4 or 5 , wherein the fusion protein comprises a first Com ABP fused to the N-terminus of CD63 and a second Com ABP fused to the C-terminus of CD63; and/or wherein the extracellular vesicle is an exosome or a microvesicle.

7. A method of producing an extracellular vesicle, the method comprising:
(a) transfecting a plurality of eukaryotic cells with the first mammalian expression plasmid and the second mammalian expression plasmid of the system of any one of claims 1 to 3; and
b) culturing the transfected eukaryotic cells for sufficient time for extracellular vesicles to be produced.

8. The method of claim 7, further comprising transfecting the plurality of eukaryotic cells with the envelope plasmid of the system of claim 2; and optionally the extracellular vesicle comprises:
(a) a RNP comprising (i) a CRISPR-associated endonuclease and (ii) a gRNA comprising at least one Com aptamer coding sequence;
(b) a fusion protein comprising CD63 and at least one Com aptamer binding protein, wherein the Com aptamer binding protein (ABP) binds to the Com aptamer coding sequence; and
(c) a VSV-G protein; or, wherein the extracellular vesicle comprises:
(a)
(i) a RNP comprising: a CRISPR-associated endonuclease; and a gRNA comprising at least one Com aptamer coding sequence, or
(ii) an mRNA encoding the heterologous polypeptide and the at least one Com aptamer sequence; and
(b) a fusion protein comprising CD63 and at least one Com aptamer binding protein, wherein the Com aptamer binding protein (ABP) binds to the at least one Com aptamer coding sequence.

9. The method of claims 7 or 8, wherein the plurality of eukaryotic cells are mammalian cells; and/or further comprising isolating the extracellular vesicles from the cultured transfected eukaryotic cells.

10. An extracellular vesicle made by the method of any one of claims 7 to 9.

11. An *ex vivo* method of modifying a genomic target sequence in a cell, the method comprising transducing a plurality of eukaryotic cells with a plurality of extracellular vesicles, wherein the plurality of extracellular vesicles comprise an extracellular vesicle according to any one of claims 4 to 6, wherein the RNP binds to the genomic target sequence in genomic DNA of the cell, thereby modifying the genomic target sequence.

12. The method of claim 11, wherein the plurality of eukaryotic cells are mammalian cells.

13. A plurality of extracellular vesicles for use in a method of modifying a genomic target sequence in a cell, the method comprising transducing a plurality of eukaryotic cells with a plurality of extracellular vesicles, wherein the plurality of extracellular vesicles comprise an extracellular vesicle according to any one of claims 4 to 6, wherein the RNP binds to the genomic target sequence in genomic DNA of the cell, thereby modifying the genomic target sequence.

14. The plurality of extracellular vesicles for the use of claim 13, wherein the subject is a human subject; and/or wherein the subject is injected with the plurality of extracellular vesicles.

15. Modified cells for use in a method for treating a disease in a subject, the method comprising:
a) providing cells obtained from the subject;
b) modifying the cells of the subject using the method of claim 11; and
c) administering the modified cells to the subject.

16. The modified cells for use according to claim 15 wherein the disease is cancer; or wherein the disease is sickle cell anemia; and/or wherein the cells are T cells.

## Patentansprüche

1. Plasmidsystem, umfassend:
(a) ein erstes Säugetierexpressionsplasmid, umfassend einen eukaryotischen Promotor, der funktionell mit einer Nukleinsäuresequenz verbunden ist, wobei die Nukleinsäuresequenz umfasst:
(i) eine Nukleinsäuresequenz, die für eine CRISPR-assoziierte Endonuklease kodiert, und eine Führungs-RNA-Kodiersequenz (gRNA-Kodiersequenz), wobei die gRNA-Kodiersequenz mindestens eine Com-Aptamer-Kodiersequenz umfasst; oder
(ii) eine Nukleinsäuresequenz, die für ein heterologes Polypeptid kodiert, und mindestens eine Com-Aptamer-Kodiersequenz; und
(b) ein zweites Säugetierexpressionsplasmid, umfassend einen eukaryotischen Promotor, der funktionell mit einer Nukleinsäuresequenz verbunden ist, die für ein Fusionsprotein kodiert, das CD63 und mindestens ein Com-Aptamer-Bindungsprotein umfasst, wobei das Com-Aptamer-Bindungsprotein (ABP) an die mindestens eine Com-Aptamer-Kodiersequenz des ersten Säugetierexpressionsplasmids bindet.

2. Plasmidsystem nach Anspruch 1 Teil (i), ferner umfassend ein Hüllplasmid, umfassend eine Nukleinsäuresequenz, die für vesikuläres Stomatitisvirus-G-Protein (VSV-G-Protein) kodiert; und/oder wobei die CRISPR-assoziierte Endonuklease ein Cas9-Protein, ein Cpf1-Protein oder ein Derivat von einem der beiden ist; und/oder wobei die CRISPR-assoziierte Endonuklease eine katalytisch beeinträchtigte CRISPR-assoziierte Endonuklease ist; und gegebenenfalls wobei die Kodiersequenz der katalytisch beeinträchtigten CRISPR-assoziierten Endonuklease für ein Cas9-D10A-Protein kodiert und/oder wobei die Nukleinsäuresequenz des ersten Säugetierexpressionsplasmids für eine Nukleinsäuresequenz kodiert, die für einen Adenosin-Basenpaareditor (ABE) kodiert, wobei der ABE ein Fusionsprotein ist, das eine Adenosindeaminase und die katalytisch beeinträchtigte CRISPR-assoziierte Endonuklease umfasst und gegebenenfalls wobei der Adenin-Baseneditor ABE 7.10 oder ABE8 ist; und/oder wobei die sgRNA-Kodiersequenz mindestens eine Com-Aptamer-Kodiersequenz umfasst, die in den Tetraloop oder den ST2-Loop der sgRNA-Kodiersequenz eingefügt ist.

3. Plasmidsystem nach einem der Ansprüche 1 oder 2, wobei das Fusionsprotein ein erstes Com-ABP, das an den N-Terminus von CD63 fusioniert ist, und ein zweites Com-ABP, das an den C-Terminus von CD63 fusioniert ist, umfasst.

4. Extrazelluläres Vesikel, umfassend:
(a)
(i) einen Ribonukleotidproteinkomplex (RNP-Komplex), umfassend eine CRISPR-assoziierte Endonuklease und eine gRNA, umfassend mindestens eine Com-Aptamer-Kodiersequenz; oder
(ii) eine mRNA, die für ein heterologes Polypeptid und mindestens eine Com-Aptamer-Kodiersequenz kodiert; und
und
(b) ein Fusionsprotein, umfassend CD63 und mindestens ein Com-Aptamer-Bindungsprotein (ABP), wobei das Com-ABP an die mindestens eine Com-Aptamer-Kodiersequenz bindet.

5. Extrazelluläres Vesikel nach Anspruch 4, ferner umfassend ein VSV-G-Protein; und/oder wobei die CRISPR-assoziierte Endonuklease ein Cas9-Protein, ein Cpf1-Protein oder ein Derivat von einem der beiden ist; oder wobei die CRISPR-assoziierte Endonuklease eine katalytisch beeinträchtigte CRISPR-assoziierte Endonuklease ist, gegebenenfalls wobei die katalytisch beeinträchtigte CRISPR-assoziierte Endonukleasekodiersequenz für ein Cas9-D10A-Protein kodiert und gegebenenfalls wobei das RNP einen Adenin-Basenpaareditor (ABE) umfasst, wobei der ABE ein Fusionsprotein ist, das eine Adenosindeaminase und die katalytisch beeinträchtigte CRISPR-assoziierte Endonuklease umfasst; gegebenenfalls wobei der Adenin-Baseneditor ABE 7.10 oder ABE8 ist; und/oder wobei die sgRNA-Kodiersequenz mindestens eine Com-Aptamer-Kodiersequenz umfasst, die in den Tetraloop oder den ST2-Loop der sgRNA-Kodiersequenz eingefügt ist.

6. Extrazelluläres Vesikel nach einem der Ansprüche 4 oder 5, wobei das Fusionsprotein ein erstes Com-ABP, das an den N-Terminus von CD63 fusioniert ist, und ein zweites Com-ABP, das an den C-Terminus von CD63 fusioniert ist, umfasst; und/oder wobei das extrazelluläre Vesikel ein Exosom oder ein Mikrovesikel ist.

7. Verfahren zur Herstellung eines extrazellulären Vesikels, wobei das Verfahren umfasst:
(a) Transfizieren einer Vielzahl eukaryotischer Zellen mit dem ersten Säugetierexpressionsplasmid und dem zweiten Säugetierexpressionsplasmid des Systems nach einem der Ansprüche 1 bis 3; und
b) Kultivieren der transfizierten eukaryotischen Zellen für eine ausreichende Zeit, damit extrazelluläre Vesikel produziert werden.

8. Verfahren nach Anspruch 7, ferner umfassend das Transfizieren der Vielzahl von eukaryotischen Zellen mit dem Hüllplasmid des Systems nach Anspruch 2; und gegebenenfalls umfasst das extrazelluläre Vesikel:
(a) ein RNP, umfassend (i) eine CRISPR-assoziierte Endonuklease und (ii) eine gRNA, die mindestens eine Com-Aptamer-Kodiersequenz umfasst;
(b) ein Fusionsprotein, umfassend CD63 und mindestens ein Com-Aptamer-Bindungsprotein, wobei das Com-Aptamer-Bindungsprotein (ABP) an die Com-Aptamer-Kodiersequenz bindet; und
(c) ein VSV-G-Protein oder wobei das extrazelluläre Vesikel umfasst:
(a)
(i) ein RNP, umfassend: eine CRISPR-assoziierte Endonuklease; und eine gRNA, umfassend mindestens eine Com-Aptamer-Kodiersequenz, oder
(ii) eine mRNA, die für das heterologe Polypeptid und die mindestens eine Com-Aptamer-Sequenz kodiert; und
(b) ein Fusionsprotein, umfassend CD63 und mindestens ein Com-Aptamer-Bindungsprotein, wobei das Com-Aptamer-Bindungsprotein (ABP) an die mindestens eine Com-Aptamer-Kodiersequenz bindet.

9. Verfahren nach Anspruch 7 oder 8, wobei die Vielzahl von eukaryotischen Zellen Säugetierzellen sind; und/oder ferner umfassend das Isolieren der extrazellulären Vesikel aus den kultivierten transfizierten eukaryotischen Zellen.

10. Extrazelluläres Vesikel, hergestellt durch das Verfahren nach einem der Ansprüche 7 bis 9.

11. Ex-vivo-Verfahren zum Modifizieren einer genomischen Zielsequenz in einer Zelle, wobei das Verfahren das Transduzieren einer Vielzahl von eukaryotischen Zellen mit einer Vielzahl von extrazellulären Vesikeln umfasst, wobei die Vielzahl von extrazellulären Vesikeln ein extrazelluläres Vesikel nach einem der Ansprüche 4 bis 6 umfasst, wobei das RNP an die genomische Zielsequenz in genomischer DNA der Zelle bindet, wodurch die genomische Zielsequenz modifiziert wird.

12. Verfahren nach Anspruch 11, wobei die Vielzahl von eukaryotischen Zellen Säugetierzellen sind.

13. Vielzahl von extrazellulären Vesikeln zur Verwendung in einem Verfahren zum Modifizieren einer genomischen Zielsequenz in einer Zelle, wobei das Verfahren das Transduzieren einer Vielzahl von eukaryotischen Zellen mit einer Vielzahl von extrazellulären Vesikeln umfasst, wobei die Vielzahl von extrazellulären Vesikeln ein extrazelluläres Vesikel nach einem der Ansprüche 4 bis 6 umfasst, wobei das RNP an die genomische Zielsequenz in genomischer DNA der Zelle bindet, wodurch die genomische Zielsequenz modifiziert wird.

14. Vielzahl von extrazellulären Vesikeln für die Verwendung nach Anspruch 13, wobei das Subjekt ein Mensch ist; und/oder wobei dem Subjekt die Vielzahl von extrazellulären Vesikeln injiziert wird.

15. Modifizierte Zellen zur Verwendung in einem Verfahren zum Behandeln einer Erkrankung bei einem Subjekt, wobei das Verfahren umfasst:
a) Bereitstellen von Zellen, die von dem Subjekt erhalten wurden;
b) Modifizieren der Zellen des Subjekts unter Verwendung des Verfahrens nach Anspruch 11; und
c) Verabreichen der modifizierten Zellen an das Subjekt.

16. Modifizierte Zellen zur Verwendung nach Anspruch 15, wobei die Erkrankung Krebs ist; oder wobei die Erkrankung Sichelzellenanämie ist; und/oder wobei die Zellen T-Zellen sind.

## Revendications

1. Système plasmidique comprenant :
(a) un premier plasmide d'expression mammalienne comprenant un promoteur eucaryote lié fonctionnellement à une séquence d'acides nucléiques, dans lequel la séquence d'acides nucléiques comprend :
(i) une séquence d'acides nucléiques codant pour une endonucléase associée à CRISPR et
une séquence codante d'ARN guide (ARNg), dans lequel la séquence codante d'ARNg comprend au moins une séquence codante d'aptamère Com ; ou
(ii) une séquence d'acides nucléiques codant pour un polypeptide hétérologue et au moins une séquence codante d'aptamère Com ; et
(b) un second plasmide d'expression mammalienne comprenant un promoteur eucaryote lié fonctionnellement à une séquence d'acides nucléiques codant pour une protéine de fusion comprenant CD63 et au moins une protéine de liaison d'aptamère Com, dans lequel la protéine de liaison d'aptamère (ABP) Com se lie à l'au moins une séquence codante d'aptamère Com du premier plasmide d'expression mammalienne.

2. Système plasmidique selon la revendication 1 partie (i), comprenant en outre un plasmide d'enveloppe comprenant une séquence d'acides nucléiques codant pour une protéine G du virus de la stomatite vésiculaire (VSV G) ; et/ou dans lequel l'endonucléase associée à CRISPR est une protéine Cas9, une protéine Cpf1 ou un dérivé de l'une ou l'autre ; et/ou dans lequel l'endonucléase associée à CRISPR est une endonucléase associée à CRISPR catalytiquement altérée ; et facultativement dans lequel la séquence codante d'endonucléase associée à CRISPR catalytiquement altérée code pour une protéine Cas9 D10A, et/ou dans lequel la séquence d'acides nucléiques du premier plasmide d'expression mammalienne code pour une séquence d'acides nucléiques codant pour un éditeur de paire de bases adénosine (ABE), dans lequel l'ABE est une protéine hybride comprenant une adénosine désaminase et l'endonucléase associée à CRISPR catalytiquement altérée et facultativement dans lequel l'éditeur de bases adénine est ABE 7.10 ou ABE8 ; et/ou dans lequel la séquence codante d'ARNsg comprend au moins une séquence codante d'aptamère Com insérée dans la boucle tétranucléotide ou la boucle ST2 de la séquence codante d'ARNsg.

3. Système plasmidique selon l'une quelconque des revendications 1 ou 2, dans lequel la protéine de fusion comprend une première ABP Com fusionnée à la terminaison N de CD63 et une seconde ABP Com fusionnée à la terminaison C de CD63.

4. Vésicule extracellulaire comprenant :
(a)
(i) un complexe ribonucléotide protéine (RNP) comprenant une endonucléase associée à CRISPR et un ARNg comprenant au moins une séquence codante d'aptamère Com ; ou
(ii) un ARNm codant pour un polypeptide hétérologue et au moins une séquence codante d'aptamère Com ; et
et
(b) une protéine de fusion comprenant CD63 et au moins une protéine de liaison d'aptamère (ABP) Com, dans laquelle l'ABP Com se lie à l'au moins une séquence codante d'aptamère Com.

5. Vésicule extracellulaire selon la revendication 4, comprenant en outre une protéine VSV-G ; et/ou dans laquelle l'endonucléase associée à CRISPR est une protéine Cas9, une protéine Cpf1 ou un dérivé de l'une ou l'autre ; ou dans laquelle l'endonucléase associée à CRISPR est une endonucléase associée à CRISPR catalytiquement altérée, facultativement dans laquelle la séquence codante d'endonucléase associée à CRISPR catalytiquement altérée code pour une protéine Cas9 D10A, et facultativement dans laquelle le RNP comprend un éditeur de paire de bases adénine (ABE), dans laquelle l'ABE est une protéine de fusion comprenant une adénosine désaminase et l'endonucléase associée à CRISPR catalytiquement altérée ; facultativement dans laquelle l'éditeur de bases adénine est ABE 7.10 ou ABE8 ; et/ou dans laquelle la séquence codante d'ARNsg comprend au moins une séquence codante d'aptamère Com insérée dans la boucle tétranucléotide ou la boucle ST2 de la séquence codante d'ARNsg.

6. Vésicule extracellulaire selon l'une quelconque des revendications 4 ou 5, dans laquelle la protéine de fusion comprend une première ABP Com fusionnée à la terminaison N de CD63 et une seconde ABP Com fusionnée à la terminaison C de CD63 ; et/ou dans laquelle la vésicule extracellulaire est un exosome ou une microvésicule.

7. Procédé de production d'une vésicule extracellulaire, le procédé comprenant :
(a) la transfection d'une pluralité de cellules eucaryotes avec le premier plasmide d'expression mammalienne et le second plasmide d'expression mammalienne du système selon l'une quelconque des revendications 1 à 3 ; et
b) la culture des cellules eucaryotes transfectées pendant un temps suffisant pour que des vésicules extracellulaires soient produites.

8. Procédé selon la revendication 7, comprenant en outre la transfection de la pluralité de cellules eucaryotes avec le plasmide d'enveloppe du système selon la revendication 2 ; et facultativement la vésicule extracellulaire comprend :
(a) un RNP comprenant (i) une endonucléase associée à CRISPR et (ii) un ARNg comprenant au moins une séquence codante d'aptamère Com ;
(b) une protéine de fusion comprenant CD63 et au moins une protéine de liaison d'aptamère Com, dans lequel la protéine de liaison d'aptamère (ABP) Com se lie à la séquence codante d'aptamère Com ; et
(c) une protéine VSV-G ; ou, dans lequel la vésicule extracellulaire comprend :
(a)
(i) un RNP comprenant : une endonucléase associée à CRISPR ; et un ARNg comprenant au moins une séquence codante d'aptamère Com, ou
(ii) un ARNm codant pour le polypeptide hétérologue et l'au moins une séquence d'aptamère Com ; et
(b) une protéine de fusion comprenant CD63 et au moins une protéine de liaison d'aptamère Com, dans lequel la protéine de liaison d'aptamère (ABP) Com se lie à l'au moins une séquence codante d'aptamère Com.

9. Procédé selon les revendications 7 ou 8, dans lequel la pluralité de cellules eucaryotes sont des cellules mammaliennes ; et/ou comprenant en outre l'isolement des vésicules extracellulaires par rapport aux cellules eucaryotes transfectées cultivées.

10. Vésicule extracellulaire fabriquée par le procédé selon l'une quelconque des revendications 7 à 9.

11. Procédé *ex vivo* de modification d'une séquence cible génomique dans une cellule, le procédé comprenant la transduction d'une pluralité de cellules eucaryotes avec une pluralité de vésicules extracellulaires, dans lequel la pluralité de vésicules extracellulaires comprennent une vésicule extracellulaire selon l'une quelconque des revendications 4 à 6, dans lequel le RNP se lie à la séquence cible génomique dans de l'ADN génomique de la cellule, en modifiant de ce fait la séquence cible génomique.

12. Procédé selon la revendication 11, dans lequel la pluralité de cellules eucaryotes sont des cellules mammaliennes.

13. Pluralité de vésicules extracellulaires pour utilisation dans un procédé de modification d'une séquence cible génomique dans une cellule, le procédé comprenant la transduction d'une pluralité de cellules eucaryotes avec une pluralité de vésicules extracellulaires, dans laquelle la pluralité de vésicules extracellulaires comprennent une vésicule extracellulaire selon l'une quelconque des revendications 4 à 6, dans laquelle le RNP se lie à la séquence cible génomique dans de l'ADN génomique de la cellule, en modifiant de ce fait la séquence cible génomique.

14. Pluralité de vésicules extracellulaires pour l'utilisation selon la revendication 13, dans laquelle le sujet est un sujet humain ; et/ou dans laquelle le sujet est injecté avec la pluralité de vésicules extracellulaires.

15. Cellules modifiées pour utilisation dans un procédé permettant de traiter une maladie chez un sujet, le procédé comprenant :
a) la fourniture de cellules obtenues auprès du sujet ;
b) la modification des cellules du sujet à l'aide du procédé selon la revendication 11 ; et
c) l'administration des cellules modifiées au sujet.

16. Cellules modifiées pour utilisation selon la revendication 15 dans lesquelles la maladie est un cancer ; ou dans lesquelles la maladie est anémie drépanocytaire ; et/ou dans lesquelles les cellules sont des cellules T.
